# EUROPEAN PATENT APPLICATION

(11) **EP 4 691 560 A2**
(43) Date of publication of application: **11.02.2026**
(21) Application number: 25211485.5
(22) Date of filing: 13.06.2019
(51) Int. Cl.: A61P 25/18

(54) **VMAT2 INHIBITOR COMPOUNDS, COMPOSITIONS, AND METHODS RELATING THERETO**

(30) Priority: 14.06.2018 US 201862684935 P
(62) Divisional of application: 19739758.1
(71) Applicant: NEUROCRINE BIOSCIENCES, INC., San Diego, CA 92130 (US)
(72) Inventor: O'BRIEN, Christopher F., San Diego, 92130 (US); HARRIOTT, Nicole, San Diego, 92130 (US)
(74) Representative: Mewburn Ellis LLP

(57) **Abstract**

Methods are provided herein for treating psychiatric disorders relating to 22q11.2 deletion syndrome and for selecting subjects for treatment with a VMAT2 inhibitor. The methods provided herein comprise administering a VMAT2 inhibitor to a subject in need thereof.

## Description

This disclosure relates generally to VMAT2 inhibitor compounds, compositions and methods related thereto.

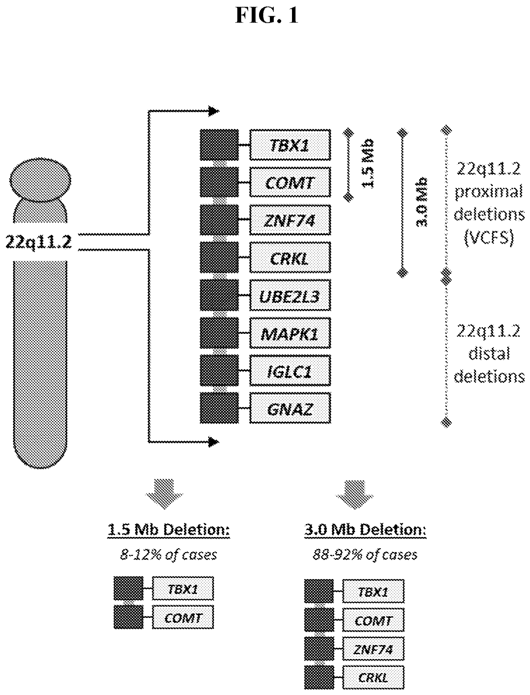

22q11.2 Deletion Syndrome (22q11.2 DS) is also known as Velocardiofacial syndrome ("VCFS"), DiGeorge syndrome, CATCH 22 and less often referred to as DiGeorge sequence, Microdeletion 22q11.2, Monosomy 22q11, Conotruncal anomaly face syndrome, Sedlačková syndrome, Shprintzen syndrome, Takao syndrome, or Cayler cardiofacial syndrome. It is an autosomal dominant genetic condition that arises from the deletion of genes on chromosome 22 at band q11.2. Approximately 90% of individuals with VCFS have a 3 Mb deletion with the deletion of two genes, COMT and TBX1, being specifically associated with VCFS (see, Fig. 1). Only ~10% of individuals have a smaller 1.5 Mb deletion, which also typically includes the deletion of TBX1 and COMT. However, not all genes related to VCFS have been identified.

COMT is a key enzyme for regulating catechol compounds, including dopamine, epinephrine and norepinephrine. Individuals with VCFS have approximately 50% less COMT mRNA, COMT protein expression, and enzyme activity compared to normal subjects. The characteristic behavioral manifestations of VCFS may be related to dopamine dysregulation resulting from COMT haploinsufficiency. However, that can be compounded by the presence of a low-activity COMT allele, leading to further dysregulation in patients with VCFS. COMT contains a common functional polymorphism, Val158Met (rs4680), which leads to alterations in enzyme activity. Individuals with VCFS who have a single copy of the Met allele have markedly low COMT activity. Compared with VCFS adults carrying the COMT Val allele, those carrying the Met allele tend to have increased risk for psychotic disorders, other neuropsychiatric syndromes, and have more severe cognitive deficits.

Despite the phenotypic variation, there are a few hallmark features found in a majority of patients with VCFS including physical and palatal-related features as well as cognitive and neuropsychiatric features. Psychiatric phenotypes of the syndrome have been well documented, and affected individuals have substantially greater rates of various psychiatric disorders found in the general population. Early studies consistently showed a greater than average prevalence of schizophrenia spectrum disorders. Although schizophrenia spectrum disorder is most frequently associated with VCFS, ~60% of patients meet diagnostic criteria for other psychiatric disorders including psychotic disorders, ADHD, mood disorders, anxiety, and autism spectrum disorders.

Treatment guidelines for schizophrenia in VCFS are currently the same as those for idiopathic schizophrenia and psychiatric medications are commonly prescribed to individuals with VCFS. However, patients with VCFS have an increased likelihood of neurologic side effects with antipsychotic therapy. In addition, dopamine dysregulation related to haploinsufficiency of the COMT gene might reduce clinical response to psychotropic medications. Patients also may be more vulnerable to adverse events, such as drug-induced cardiac arrhythmias, caused by their high rates of cardiovascular anomalies.

Despite the advances that have been made in this field, a need remains in the art for improved methods for treating psychiatric disorders and for selecting subjects for treatment with a VMAT2 inhibitor. The present disclosure fulfills these and other needs, as evident in reference to the following disclosure.

### BRIEF SUMMARY

Provided is a method for selecting a subject for treatment with a VMAT2 inhibitor, comprising:
determining whether the subject has 22q11.2 deletion syndrome; and
administering a therapeutically effective amount of a VMAT2 inhibitor to the subject if the subject has been determined to have 22q11.2 deletion syndrome.

Also provided is a method for selecting a subject for treatment with a VMAT2 inhibitor, comprising:
determining whether the subject has COMT haploinsufficiency; and
administering a therapeutically effective amount of a VMAT2 inhibitor to the subject if the subject has been determined to have COMT haploinsufficiency.

Also provided is a method of treating a psychiatric disorder in a subject in need thereof, comprising:
administering a therapeutically effective amount of a VMAT2 inhibitor to the subject,
wherein the subject is predisposed to developing said psychiatric disorder due to the subject having COMT haploinsufficiency.

Also provided is a method of treating a psychiatric disorder in a subject in need thereof, comprising:
administering a therapeutically effective amount of a VMAT2 inhibitor to the subject,
wherein the subject is predisposed to developing said psychiatric disorder due to the subject having 22q11.2 deletion syndrome.

Also provided herein is a method of treating a psychiatric disorder in a subject in need thereof, comprising:
determining whether the subject has COMT haploinsufficiency; and
administering a therapeutically effective amount of a VMAT2 inhibitor to the subject if the subject has been determined to have COMT haploinsufficiency.

Also provided herein is a method of treating a psychiatric disorder in a subject in need thereof, comprising:
determining whether the subject has 22q11.2 deletion syndrome; and
administering a therapeutically effective amount of a VMAT2 inhibitor to the subject if the subject has been determined to have 22q11.2 deletion syndrome.

### BRIEF DESCRIPTION OF DRAWINGS

Figure 1 illustrates the genetic basis of VCFS and particularly those deletions which are common in VCFS.
Figure 2 shows a process flow diagram for the production of high drug loading valbenazine capsules (80 mg).

### DETAILED DESCRIPTION

Terms not specifically defined herein should be given the meanings that would be given to them by one of skill in the art in light of the disclosure and the context. As used in the specification, however, unless specified to the contrary, the terms have the meaning indicated.

In the following description, certain specific details are set forth in order to provide a thorough understanding of various embodiments. However, one skilled in the art will understand that the present compounds may be made and used without these details. In other instances, well-known structures have not been shown or described in detail to avoid unnecessarily obscuring descriptions of the embodiments. Unless the context requires otherwise, throughout the specification and claims which follow, the word "comprise" and variations thereof, such as, "comprises" and "comprising," are to be construed in an open, inclusive sense, that is, as "including, but not limited to." In addition, the term "comprising" (and related terms such as "comprise" or "comprises" or "having" or "including") is not intended to exclude that in other certain embodiments, for example, an embodiment of any composition of matter, composition, method, or process, or the like, described herein, may "consist of" or "consist essentially of" the described features. Headings provided herein are for convenience only and do not interpret the scope or meaning of the claimed embodiments.

Reference throughout this specification to "one embodiment" or "an embodiment" means that a particular feature, structure or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

Also, as used in this specification and the appended claims, the singular forms "a," "an," and "the" include plural referents unless the content clearly dictates otherwise. Thus, for example, reference to "a non-human animal" may refer to one or more non-human animals, or a plurality of such animals, and reference to "a cell" or "the cell" includes reference to one or more cells and equivalents thereof (e.g., plurality of cells) known to those skilled in the art, and so forth. When steps of a method are described or claimed, and the steps are described as occurring in a particular order, the description of a first step occurring (or being performed) "prior to" (*i.e.,* before) a second step has the same meaning if rewritten to state that the second step occurs (or is performed) "subsequent" to the first step.

As used herein, the term "about" when referring to a number or a numerical range means that the number or numerical range referred to is an approximation within experimental variability (or within statistical experimental error), and thus the number or numerical range may vary between 1% and 15% of the stated number or numerical range. It should also be noted that the term "or" is generally employed in its sense including "and/or" unless the content clearly dictates otherwise. The term, "at least one," for example, when referring to at least one compound or to at least one composition, has the same meaning and understanding as the term, "one or more."

As used herein, "VMAT2" refers to human vesicular monoamine transporter isoform 2, an integral membrane protein that acts to transport monoamines, particularly neurotransmitters such as dopamine, norepinephrine, serotonin, and histamine, from cellular cytosol into synaptic vesicles.

As used herein, "VMAT2 inhibitor", "inhibit VMAT2", or "inhibition of VMAT2" refers to the ability of a compound disclosed herein to alter the function of VMAT2. A VMAT2 inhibitor may block or reduce the activity of VMAT2 by forming a reversible or irreversible covalent bond between the inhibitor and VMAT2 or through formation of a noncovalently bound complex. Such inhibition may be manifest only in particular cell types or may be contingent on a particular biological event. The term "VMAT2 inhibitor", "inhibit VMAT2", or "inhibition of VMAT2" also refers to altering the function of VMAT2 by decreasing the probability that a complex forms between a VMAT2 and a natural substrate. Example 2 herein provides methods for determining VMAT2 binding affinities (*Kᵢ*). In some embodiments, the VMAT2 inhibitor has a *Kᵢ* for VMAT2 of less than about 1000 nM. In some embodiments, the VMAT2 inhibitor has a *Kᵢ* for VMAT2 of less than about 100 nM. In some embodiments, the VMAT2 inhibitor has a *Kᵢ* for VMAT2 of less than about 20 nM. In some embodiments, the VMAT2 inhibitor has a *Kᵢ* for VMAT2 of less than about 10 nM. In some embodiments, the VMAT2 inhibitor has a *Kᵢ* for VMAT2 of from about 1 nM to about 1000 nM, about 1 nM to about 100 nM, or about 1 nM to about 10 nM.

In some embodiments, the VMAT2 inhibitor is a compound that inhibits VMAT2 activity preferentially over other monoamine receptors. In some embodiments, the VMAT2 inhibitor is a compound that inhibits VMAT2 activity preferentially over VMAT1. In some embodiments, the VMAT2 inhibitor is a compound that is about 10-fold more selective for VMAT2 over VMAT1. In some embodiments, the VMAT2 inhibitor is a compound that is about 3-fold, about 5-fold, about 10-fold, about 15-fold, or about 20-fold more selective for VMAT2 over VMAT1 as calculated by measuring binding affinities (*K*ᵢ) (see, e.g., Example 2).

The subject in need of the compositions and methods described herein includes a subject who has been diagnosed by a person skilled in the medical and psychiatric arts with a neurological and/or psychiatric diseases and disorder. A subject (or patient) to be treated may be a mammal, including a human or non-human primate. The mammal may be a domesticated animal such as a cat or a dog.

As understood by a person skilled in the medical art, the terms, "treat" and "treatment," refer to medical management of a disease, disorder, or condition of a subject *(i.e.,* patient) (*see, e.g.,* Stedman's Medical Dictionary). The terms "treatment" and "treating" embraces both preventative, i.e. prophylactic, or therapeutic, i.e. curative and/or palliative, treatment. Thus the terms "treatment" and "treating" comprise therapeutic treatment of patients having already developed the condition, in particular in manifest form. Therapeutic treatment may be symptomatic treatment in order to relieve the symptoms of the specific indication or causal treatment in order to reverse or partially reverse the conditions of the indication or to stop or slow down progression of the disease. Thus the compositions and methods described herein may be used, for instance, as therapeutic treatment over a period of time as well as for chronic therapy. In addition, the terms "treatment" and "treating" comprise prophylactic treatment, *i.e.,* a treatment of patients at risk to develop a condition mentioned hereinbefore, thus reducing the risk.

Therapeutic and/or prophylactic benefit includes, for example, an improved clinical outcome, both therapeutic treatment and prophylactic or preventative measures, wherein the object is to prevent or slow or retard (lessen) an undesired physiological change or disorder, or to prevent or slow or retard (lessen) the expansion or severity of such disorder. Prophylactic administration of a composition herein may commence upon first treatment with dopamine receptor blocking drugs such as neuroleptics. As discussed herein, beneficial or desired clinical results from treating a subject include, but are not limited to, abatement, lessening, or alleviation of symptoms that result from or are associated the disease, condition, or disorder to be treated; decreased occurrence of symptoms; improved quality of life; longer disease-free status *(i.e.,* decreasing the likelihood or the propensity that a subject will present symptoms on the basis of which a diagnosis of a disease is made); diminishment of extent of disease; stabilized (i.e., not worsening) state of disease; delay or slowing of disease progression; amelioration or palliation of the disease state; and remission (whether partial or total), whether detectable or undetectable; and/or overall survival. "Treatment" can also mean prolonging survival when compared to expected survival if a subject were not receiving treatment. Subjects in need of treatment include those who already have the condition or disorder as well as subjects prone to have or at risk of developing the disease, condition, or disorder, and those in which the disease, condition, or disorder is to be prevented *(i.e.,* decreasing the likelihood of occurrence of the disease, disorder, or condition). A therapeutically effective amount of any one of the VMAT2 inhibitors described herein in the amount of the VMAT2 inhibitor that provides a statistically or clinically significant therapeutic and/or prophylactic benefit to the treated subject.

As used herein, "schizophrenia spectrum disorder" refers to a neuropsychiatric disorder chosen from schizophrenia, schizoaffective disorder, delusional disorder, and catatonia.

Schizophrenia is a psychological disorder characterized by major disturbances in thought, perception, emotion, and behavior. In order to be diagnosed with schizophrenia, according to the DSM-5, a person must exhibit both a psychotic episode and two additional symptoms for most of one month, and their symptoms must have a significant impact on social or occupational functioning for at least six months. The "two additional symptoms" can be delusions, hallucinations, disorganized speech, or a negative symptom or severely disorganized or catatonic behavior. If delusions or hallucinations or severe, only one symptom may be sufficient for diagnosis.

Schizoaffective disorder is characterized by abnormal thought processes and dysregulated emotions. A person with this disorder has features of both schizophrenia and a mood disorder (either bipolar disorder or depression) but does not strictly meet the diagnostic criteria for either. The bipolar subtype is distinguished by symptoms of mania, hypomania, or mixed episodes; the depressive subtype is distinguished by symptoms of depression only. Common symptoms of schizoaffective disorder include hallucinations, paranoid delusions, and disorganized speech and thinking. The DSM-5 distinguishes schizoaffective disorder from psychotic depression or psychotic bipolar disorder by additionally requiring that a psychotic condition must last for at least two continuous weeks *without* mood symptoms (although a person may be mildly depressed during this time). Two episodes of psychosis (an increase from one episode in the DSM-IV) must be experienced in order for the person to qualify for this diagnosis.

Delusional disorder is a psychiatric condition in which the person presents with delusions but no accompanying hallucinations, thought disorder, mood disorder, or significant flattening of affect. There are seven subtypes of delusional disorder: Erotomanic type (erotomania); Grandiose type; Jealous type; Persecutory type; Somatic type; Mixed type; and Unspecified type.

As used herein, "anxiety disorder" refers to a chronic condition characterized by an excessive and persistent sense of apprehension, and may include physical symptoms such as sweating, palpitations, and feelings of stress. Anxiety disorders include generalized anxiety disorder, panic disorder, specific phobias, agoraphobia, social anxiety disorder and separation anxiety disorder.

As used herein, "mood disorder" refers a category of illnesses that describe a serious change in mood. Illness under mood disorders include: major depressive disorder, bipolar disorder (mania - euphoric, hyperactive, over inflated ego, unrealistic optimism), persistent depressive disorder (long lasting low grade depression), cyclothymia (a mild form of bipolar disorder), and SAD (seasonal affective disorder).

As used herein, "monotherapy" refers to the administration of a single active or therapeutic compound to a subject in need thereof. In some embodiments, monotherapy will involve administration of a therapeutically effective amount of a compound described herein. Monotherapy may be contrasted with combination therapy, in which a combination of multiple active compounds is administered, such as with each component of the combination present in a therapeutically effective amount.

As used herein, "maintenance therapy" refers to treatment given to patients to enable them, e.g., to stay in remission, to maintain their health in a disease-free, or limited-disease, state. Maintenance medications are typically taken for a prolonged period of time.

As used herein, "adjunctive therapy" refers to a treatment that is used in conjunction with a primary treatment and its purpose is to assist the primary treatment. Adjunctive therapies are co-administered therapies. For example, if Obsessive-Compulsive Disorder is being treated, the primary therapy may be, e.g., an antidepressant, and the co-administration of a compound described herein would be considered an adjunctive therapy.

As used herein, "co-administer" and "co-administration" and variants thereof mean the administration of at least two drugs to a patient either subsequently, simultaneously, or consequently proximate in time to one another (e.g., within the same day, or week or period of 30 days, or sufficiently proximate that each of the at least two drugs can be simultaneously detected in the blood plasma). When co-administered, two or more active agents can be co-formulated as part of the same composition or administered as separate formulations. This also may be referred to herein as "concomitant" administration or variants thereof.

As used herein, "adjusting administration", "altering administration", "adjusting dosing", or "altering dosing" are all equivalent and mean tapering off, reducing or increasing the dose of the substance, ceasing to administer the substance to the patient, or substituting a different active agent for the substance.

As used herein, "administering to a patient" refers to the process of introducing a composition or dosage form into the patient via an art-recognized means of introduction.

As used herein the term "disorder" is intended to be generally synonymous, and is used interchangeably with, the terms "disease," "syndrome," and "condition" (as in medical condition), in that all reflect an abnormal condition of the human or animal body or of one of its parts that impairs normal functioning, is typically manifested by distinguishing signs and symptoms.

As used herein, a "dose" means the measured quantity of an active agent to be taken at one time by a patient. In certain embodiments, wherein the active agent (i.e., a VMAT2 inhibitor) is not the free base or the free acid, the quantity is the molar equivalent to the corresponding amount of the free base or the free acid. For example, often a drug is packaged in a pharmaceutically acceptable salt form, for example valbenazine ditosylate, and the dosage for strength refers to the mass of the molar equivalent of the corresponding free base, valbenazine. As an example, 73 mg of valbenazine tosylate is the molar equivalent of 40 mg of valbenazine free base. In some embodiments, the VMAT2 inhibitor is administered at a daily dose of about 5 mg to about 160 mg. In some embodiments, the VMAT2 inhibitor is administered at a daily dose of about 5 mg. In some embodiments, the VMAT2 inhibitor is administered at a daily dose of about 20 mg. In some embodiments, the VMAT2 inhibitor is administered at a daily dose of about 40 mg. In some embodiments, the VMAT2 inhibitor is administered at a daily dose of about 60 mg. In some embodiments, the VMAT2 inhibitor is administered at a daily dose of about 80 mg. In some embodiments, the VMAT2 inhibitor is administered at a daily dose of about 100 mg. In some embodiments, the VMAT2 inhibitor is administered at a daily dose of about 120 mg. In some embodiments, the VMAT2 inhibitor is administered at a daily dose of about 140 mg. In some embodiments, the VMAT2 inhibitor is administered at a daily dose of about 160 mg.

As used herein, "dosing regimen" means the dose of an active agent taken at a first time by a patient and the interval (time or symptomatic) at which any subsequent doses of the active agent are taken by the patient such as from about 20 to about 160 mg once daily, e.g., about 20, about 40, about 60, about 80, about 100, about 120, or about 160 mg once daily. The additional doses of the active agent can be different from the dose taken at the first time.

As used herein, "effective amount" and "therapeutically effective amount" of an agent, compound, drug, composition or combination is an amount which is nontoxic and effective for producing some desired therapeutic effect upon administration to a subject or patient (e.g., a human subject or patient). The precise therapeutically effective amount for a subject may depend upon, e.g., the subject's size and health, the nature and extent of the condition, the therapeutics or combination of therapeutics selected for administration, and other variables known to those of skill in the art. The effective amount for a given situation is determined by routine experimentation and is within the judgment of the clinician.

As used herein, "informing" means referring to or providing published material, for example, providing an active agent with published material to a user; or presenting information orally, for example, by presentation at a seminar, conference, or other educational presentation, by conversation between a pharmaceutical sales representative and a medical care worker, or by conversation between a medical care worker and a patient; or demonstrating the intended information to a user for the purpose of comprehension.

As used herein, "labeling" means all labels or other means of written, printed, graphic, electronic, verbal, or demonstrative communication that is upon a pharmaceutical product or a dosage form or accompanying such pharmaceutical product or dosage form.

As used herein, a "medical care worker" means a worker in the health care field who may need or utilize information regarding an active agent, including a dosage form thereof, including information on safety, efficacy, dosing, administration, or pharmacokinetics. Examples of medical care workers include physicians, pharmacists, physician's assistants, nurses, aides, caretakers (which can include family members or guardians), emergency medical workers, and veterinarians.

As used herein, "patient" or "individual" or "subject" means a mammal, including a human, for whom or which therapy is desired, and generally refers to the recipient of the therapy.

As used herein, "patient package insert" means information for patients on how to safely use a pharmaceutical product that is part of the FDA-approved labeling. It is an extension of the professional labeling for a pharmaceutical product that may be distributed to a patient when the product is dispensed which provides consumer-oriented information about the product in lay language, for example it may describe benefits, risks, how to recognize risks, dosage, or administration.

As used herein, "Medication Guide" means an FDA-approved patient labeling for a pharmaceutical product conforming to the specifications set forth in 21 CFR 208 and other applicable regulations which contains information for patients on how to safely use a pharmaceutical product. A medication guide is scientifically accurate and is based on, and does not conflict with, the approved professional labeling for the pharmaceutical product under 21 CFR 201.57, but the language need not be identical to the sections of approved labeling to which it corresponds. A medication guide is typically available for a pharmaceutical product with special risk management information.

As used herein, a "product" or "pharmaceutical product" means a dosage form of an active agent plus published material, and optionally packaging.

As used herein, "product insert" means the professional labeling (prescribing information) for a pharmaceutical product, a patient package insert for the pharmaceutical product, or a medication guide for the pharmaceutical product.

As used herein, "professional labeling" or "prescribing information" means the official description of a pharmaceutical product approved by a regulatory agency (e.g., FDA or EMEA) regulating marketing of the pharmaceutical product, which includes a summary of the essential scientific information needed for the safe and effective use of the drug, such as, for example indication and usage; dosage and administration; who should take it; adverse events (side effects); instructions for use in special populations (pregnant women, children, geriatric, etc.); safety information for the patient, and the like.

As used herein, "published material" means a medium providing information, including printed, audio, visual, or electronic medium, for example a flyer, an advertisement, a product insert, printed labeling, an internet web site, an internet web page, an internet pop-up window, a radio or television broadcast, a compact disk, a DVD, an audio recording, or other recording or electronic medium.

As used herein, "alkenyl" refers to a straight or branched hydrocarbon chain radical group consisting solely of carbon and hydrogen atoms, containing at least one double bond, having from two to twelve carbon atoms, preferably two to eight carbon atoms and which is attached to the rest of the molecule by a single bond. Representative straight chain and branched alkenyls include ethylenyl, propylenyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, and the like; while representative straight chain and branched alkynyls include acetylenyl, propynyl, 1-butynyl, 2-butynyl, 1-pentynyl, 2-pentynyl, 3-methyl-1 butynyl, and the like.

As used herein, "alkyl" refers to a straight or branched hydrocarbon chain radical consisting solely of carbon and hydrogen atoms, containing no unsaturation, having from one to twelve carbon atoms, one to eight carbon atoms, or one to six carbon atoms, or one to four carbon atoms, and which is attached to the rest of the molecule by a single bond, e.g., methyl, ethyl, n-propyl, 1-methylethyl (iso-propyl), n-butyl, n-pentyl, 1,1-dimethylethyl (t-butyl), 3-methylhexyl, 2-methylhexyl, and the like.

As used herein, "lower alkyl" refers to a straight or branched hydrocarbon chain radical consisting solely of carbon and hydrogen atoms, saturated or unsaturated, having from one to eight carbon atoms (C1-8), or one to six carbon atoms (C1-6) in a more specific embodiment, or one to four carbon atoms (C1-4) in a more specific embodiment, and which is attached to the rest of the molecule by a single bond. Fully saturated lower alkyls include, but are not limited to, methyl, ethyl, n-propyl, iso-propyl, n-butyl, t-butyl, *n*-pentyl, iso-pentyl, neo-pentyl, n-hexyl, 2-methylpentyl, 3-methylpentyl, 2,3-dimethylbutyl, 2,2-dimethylbutyl, and the like. Unsaturated lower alkyls include, but are not limited to, any of the above listed saturated lower alkyls containing at least one double bond between adjacent carbon atoms, such as ethenyl, prop-1-enyl, but-1-enyl, pent-1-enyl, penta-1,4-dienyl, and the like.

As used herein, "aryl" refers to a hydrocarbon ring system radical comprising hydrogen, 6 to 18 carbon atoms and at least one aromatic ring. The aryl radical may be a monocyclic, bicyclic, tricyclic or tetracyclic ring system, which may include fused or bridged ring systems. Aryl radicals include, but are not limited to, aryl radicals derived from aceanthrylene, acenaphthylene, acephenanthrylene, anthracene, azulene, benzene, chrysene, fluoranthene, fluorene, as-indacene, s-indacene, indane, indene, naphthalene, phenalene, phenanthrene, pleiadene, pyrene, and triphenylene. In one embodiment, aryl is phenyl or naphthyl, and in another embodiment is phenyl.

As used herein, "aralkyl" refers to a radical of the formula -R_{bc} where R_{b} is an alkylene chain as defined herein and R_{c} is one or more aryl radicals as defined herein, for example, benzyl, diphenylmethyl and the like.

As used herein, "carbocyclyl" refers to a stable 3- to 18-membered aromatic or non-aromatic ring radical which consists of 3 to 18 carbon atoms. Unless stated otherwise specifically in the specification, the carbocyclyl radical may be a monocyclic, bicyclic, tricyclic or tetracyclic ring system, which may include fused or bridged ring systems, and may be partially or fully saturated. Non-aromatic carbocyclyl radicals include cycloalkyl, while aromatic carbocyclyl radicals include aryl.

As used herein, "cycloalkyl" refers to a stable non-aromatic monocyclic or polycyclic hydrocarbon radical consisting solely of carbon and hydrogen atoms, which may include fused or bridged ring systems, having from three to fifteen carbon atoms, preferably having from three to ten carbon atoms, and which is saturated or unsaturated and attached to the rest of the molecule by a single bond. Monocyclic radicals include, for example, cyclopropyl, cyclobutyl, cyclopentyl, cyclohexyl, cycloheptly, and cyclooctyl. Polycyclic radicals include, for example, adamantyl, norbomyl, decalinyl, 7,7-dimethyl-bicyclo-[2.2.1]heptanyl, and the like.

As used herein, "lower cycloalkyl" refers to a non-aromatic monocyclic hydrocarbon radical consisting solely of carbon and hydrogen atoms having from three to eight carbon atoms, or three to six carbon atoms (C3-6) in a more specific embodiment, or three or four carbon atoms (C₃ or C₄) in a more specific embodiment, which is saturated or unsaturated, and attached to the rest of the molecule by a single bond. Such radicals include, but are not limited to, cyclopropyl, cyclobutyl, cyclopentyl and cyclohexyl.

As used herein, "cycloalkylalkyl" refers to a radical of the formula -R_{bc} where R_{b} is an alkylene chain as defined herein and R_{c} is one or more cycloalkyl radicals as defined herein.

As used herein, "lower cycloalkylalkyl" refers to a lower alkyl as defined above wherein a hydrogen atom is replaced with a lower cycloalkyl radical as defined above. Such radicals include, but are not limited to, -CH₂-cyclopropyl, -CH₂-cyclobutyl, -CH₂-cyclopentyl and the like.

As used herein, "lower alkoxy" refers to the radical -O(lower alkyl), wherein lower alkyl is as defined above.

As used herein, "cyano" refers to the -CN radical.

As used herein, "halo" refers to bromo, chloro, fluoro or iodo.

As used herein, "heteroaryl" means an aromatic heterocycle ring of 5- to 10-members and having at least one heteroatom selected from nitrogen, oxygen and sulfur, and wherein the nitrogen and sulfur heteroatoms may be optionally oxidized, and containing at least 1 carbon atom, including both mono- and bicyclic ring systems. Representative heteroaryls include (but are not limited to) furyl, benzofuranyl, thiophenyl, benzothiophenyl, pyrrolyl, indolyl, isoindolyl, azaindolyl, pyridyl, quinolinyl, isoquinolinyl, oxazolyl, isooxazolyl, oxadiazolyl, thiadiazolyl, benzoxazolyl, pyrazolyl, imidazolyl, benzimidazolyl, thiazolyl, benzothiazolyl, isothiazolyl, pyridazinyl, pyrimidinyl, pyrazinyl, triazinyl, cinnolinyl, phthalazinyl, and quinazolinyl.

As used herein, "heteroarylalkyl" refers to a radical of the formula -R_{b}Rᵢ where R_{b} is an alkylene chain as defined herein and Rᵢ is a heteroaryl radical as defined herein.

As used herein, "heterocyclyl" refers to a stable 3- to 18-membered aromatic or non-aromatic ring radical which consists of two to twelve carbon atoms and from one to six heteroatoms selected from the group consisting of nitrogen, oxygen and sulfur. Unless stated otherwise specifically in the specification, the heterocyclyl radical may be a monocyclic, bicyclic, tricyclic or tetracyclic ring system, which may include fused or bridged ring systems; and the nitrogen, carbon or sulfur atoms in the heterocyclyl radical may be optionally oxidized; the nitrogen atom may be optionally quaternized; and the heterocyclyl radical may be partially or fully saturated. Examples or aromatic hetercyclyl radicals are listed below in the definition of heteroaryls (i.e., heteroaryl being a subset of heterocyclyl). Examples of non-aromatic heterocyclyl radicals include, but are not limited to, dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, pyrazolopyrimidinyl, quinuclidinyl, thiazolidinyl, tetrahydrofuryl, trioxanyl, trithianyl, triazinanyl, tetrahydropyranyl, thiomorpholinyl, thiamorpholinyl, 1-oxo-thiomorpholinyl, and 1, 1-dioxo-thiomorpholinyl.

As used herein, "heterocyclylalkyl" refers to a radical of the formula -R_{b}Rₕ where R_{b} is an alkylene chain as defined herein and Rₕ is a heterocyclyl radical as defined herein, and if the heterocyclyl is a nitrogen-containing heterocyclyl, the heterocyclyl may be attached to the alkyl radical at the nitrogen atom.

With regard to stereoisomers, the compounds described herein may have multiple chiral (or asymmetric) centers which give rise to enantiomers, diastereomers, and other stereoisomeric forms that may be defined, in terms of absolute stereochemistry, as *(R)-* or (S)-. When the compounds described herein contain olefinic double bonds or other centers of geometric asymmetry, and unless specified otherwise, it is intended that the compounds include both E and Z geometric isomers (e.g., *cis* or *trans).* Likewise, unless otherwise indicated, all possible isomers, as well as their racemic and optically pure forms, and all tautomeric forms are also intended to be included. It is therefore contemplated that various stereoisomers and mixtures thereof include "enantiomers," which refers to two stereoisomers whose molecules are nonsuperimposeable mirror images of one another. Thus, the compounds may occur in any isomeric form, including racemates, racemic mixtures, and as individual enantiomers or diastereomers.

Accordingly, it is understood that the present invention embraces each diastereoisomer, each enantiomer and mixtures thereof of each compound and generic formula disclosed herein just as if they were each individually disclosed with the specific stereochemical designation for each chiral carbon. Separation of the individual isomers (such as, by chiral HPLC, recrystallization of diastereoisomeric mixtures and the like) or selective synthesis (such as, by enantiomeric selective syntheses and the like) of the individual isomers is accomplished by application of various methods which are well known to practitioners in the art.

Additionally, individual compounds and chemical genera of the present invention encompass all pharmaceutically acceptable salts, solvates, and hydrates, thereof.

It is understood that when the phrase "pharmaceutically acceptable salts, solvates, and hydrates" or the phrase "pharmaceutically acceptable salt, solvate, or hydrate" is used when referring to compounds described herein, it embraces pharmaceutically acceptable solvates and/or hydrates of the compounds, pharmaceutically acceptable salts of the compounds, as well as pharmaceutically acceptable solvates and/or hydrates of pharmaceutically acceptable salts of the compounds. It is also understood that when the phrase "pharmaceutically acceptable solvates and hydrates" or the phrase "pharmaceutically acceptable solvate or hydrate" is used when referring to salts described herein, it embraces pharmaceutically acceptable solvates and/or hydrates of such salts.

The compounds described herein may generally be utilized as the free acid or free base. Alternatively, the compounds may be used in the form of acid or base addition salts. Acid addition salts of a free amino group may be prepared by methods well known in the art, and may be formed from organic and inorganic acids. Suitable organic acids include maleic, fumaric, benzoic, ascorbic, succinic, methanesulfonic, p-toluenesulfonic, acetic, trifluoroacetic, oxalic, propionic, tartaric, salicylic, citric, gluconic, lactic, mandelic, cinnamic, aspartic, stearic, palmitic, glycolic, glutamic, and benzenesulfonic acids. Suitable inorganic acids include hydrochloric, hydrobromic, sulfuric, phosphoric, and nitric acids. Base addition salts included those salts that form with the carboxylate anion and include salts formed with organic and inorganic cations such as those chosen from the alkali and alkaline earth metals (for example, lithium, sodium, potassium, magnesium, barium and calcium), as well as the ammonium ion and substituted derivatives thereof (for example, dibenzylammonium, benzylammonium, 2-hydroxyethylammonium, and the like). Thus, a "pharmaceutically acceptable salt" is intended to encompass any and all acceptable salt forms, including mono- and di-salt forms.

The compounds described herein may exist in a continuum of solid states ranging from fully amorphous to fully crystalline. Furthermore, some of the compounds described herein may exist as polymorphs.

In addition, some of the compounds may also form solvates with water or other organic solvents. The term "solvate" is used herein to describe a molecular complex comprising a compound described herein and one or more pharmaceutically acceptable solvent molecules. When the solvent molecule is water, the molecular complex is referred to as a "hydrate". Such hydrates and solvates are similarly included within the scope of this disclosure.

As used herein, "isotopic variant" (or, alternatively, an "isotope of a compound" or "isotopes of a compound") means a compound that contains an unnatural proportion of an isotope at one or more of the atoms that constitute such a compound. In certain embodiments, an "isotopic variant" of a compound contains unnatural proportions of one or more isotopes, including, but not limited to, hydrogen (¹H), deuterium (²H), tritium (³H), carbon-11 (¹¹C), carbon-12 (¹²C), carbon-13 (¹³C), carbon-14 (¹⁴C), nitrogen-13 (¹³N), nitrogen-14 (¹⁴N), nitrogen-15 (¹⁵N), oxygen-14 (¹⁴O), oxygen-15 (¹⁵O), oxygen-16 (¹⁶O), oxygen-17 (¹⁷O), oxygen-18 (¹⁸O), fluorine-17 (¹⁷F), fluorine-18 (¹⁸F), phosphorus-31 (³¹P), phosphorus-32 (³²P), phosphorus-33 (³³P), sulfur-32 (³²S), sulfur-33 (³³S), sulfur-34 (³⁴S), sulfur-35 (³⁵S), sulfur-36 (³⁶S), chlorine-35 (³⁵Cl), chlorine-36 (³⁶Cl), chlorine-37 (³⁷Cl), bromine-79 (⁷⁹Br), bromine-81 (⁸¹Br), iodine-123 (¹²³I), iodine-125 (¹²⁵I), iodine-127 (¹²⁷I), iodine-129 (¹²⁹I), and iodine-131 (¹³¹I). In certain embodiments, an "isotopic variant" of a compound is in a stable form, that is, non-radioactive. In certain embodiments, an "isotopic variant" of a compound contains unnatural proportions of one or more isotopes, including, but not limited to, hydrogen (¹H), deuterium (²H), carbon-12 (¹²C), carbon-13 (¹³C), nitrogen-14 (¹⁴N), nitrogen-15 (¹⁵N), oxygen-16 (¹⁶O), oxygen-17 (¹⁷O), and oxygen-18 (¹⁸O). In certain embodiments, an "isotopic variant" of a compound is in an unstable form, that is, radioactive. In certain embodiments, an "isotopic variant" of a compound contains unnatural proportions of one or more isotopes, including, but not limited to, tritium (³H), carbon-11 (¹¹C), carbon-14 (¹⁴C), nitrogen-13 (¹³N), oxygen-14 (¹⁴O), and oxygen-15 (¹⁵O). It will be understood that, in a compound as provided herein, any hydrogen can be ²H, as example, or any carbon can be ¹³C, as example, or any nitrogen can be ¹⁵N, as example, and any oxygen can be ¹⁸O, as example, where feasible according to the judgment of one of skill in the art. In certain embodiments, an "isotopic variant" of a compound contains an unnatural proportion of deuterium.

With regard to the compounds provided herein, when a particular atomic position is designated as having deuterium or "D" or "d", it is understood that the abundance of deuterium at that position is substantially greater than the natural abundance of deuterium, which is about 0.015%. A position designated as having deuterium typically has a minimum isotopic enrichment factor of, in certain embodiments, at least 1000 (15% deuterium incorporation), at least 2000 (30% deuterium incorporation), at least 3000 (45% deuterium incorporation), at least 3500 (52.5% deuterium incorporation), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation) at each designated deuterium position. The isotopic enrichment of the compounds provided herein can be determined using conventional analytical methods known to one of ordinary skill in the art, including mass spectrometry, nuclear magnetic resonance spectroscopy, and crystallography.

The present disclosure includes all isotopes of atoms occurring in the present compounds, intermediates, salts and crystalline forms thereof. Isotopes include those atoms having the same atomic number but different mass numbers. One aspect of the present invention includes every combination of one or more atoms in the present compounds, intermediates, salts, and crystalline forms thereof that is replaced with an atom having the same atomic number but a different mass number. One such example is the replacement of an atom that is the most naturally abundant isotope, such as ¹H or ¹²C, found in one the present compounds, intermediates, salts, and crystalline forms thereof, with a different atom that is not the most naturally abundant isotope, such as ²H or ³H (replacing ¹H), or ¹¹C, ¹³C, or ¹⁴C (replacing ¹²C). Accordingly, one aspect of the present invention includes every combination of one or more hydrogen atoms in the present compounds, intermediates, salts, and crystalline forms thereof that is replaced with a deuterium. For example, in some embodiments, the VMAT2 inhibitors disclosed herein comprise 1, 2, 3, 4, 5, or 6 instances wherein a hydrogen atom (¹H) is replaced with a deuterium atom (²H).

Another aspect of the present invention includes compositions, such as, those prepared during synthesis, preformulation, and the like, and pharmaceutical compositions, such as, those prepared with the intent of using in a mammal for the treatment of one or more of the disorders described herein, comprising one or more of the present compounds, intermediates, salts, and crystalline forms thereof, wherein the naturally occurring distribution of the isotopes in the composition is perturbed. Another aspect of the present invention includes compositions and pharmaceutical compositions comprising compounds as described herein wherein the compound is enriched at one or more positions with an isotope other than the most naturally abundant isotope. Methods are readily available to measure such isotope perturbations or enrichments, such as, mass spectrometry, and for isotopes that are radio-isotopes additional methods are available, such as, radio-detectors used in connection with HPLC or GC.

Certain isotopically-labeled compounds of the present invention are useful in compound and/or substrate tissue distribution assays. In some embodiments the radionuclide ³H and/or ¹⁴C isotopes are useful in these studies. Further, substitution with heavier isotopes such as deuterium *(i.e.,* ²H) may afford certain therapeutic advantages resulting from greater metabolic stability (*e.g.,* increased *in vivo* half-life or reduced dosage requirements) and hence may be preferred in some circumstances. Isotopically labeled compounds of the present invention can generally be prepared by following procedures analogous to those disclosed in the Examples by substituting an isotopically labeled reagent for a non-isotopically labeled reagent. Other synthetic methods that are useful are discussed below. Moreover, it should be understood that all of the atoms represented in the compounds of the invention can be either the most commonly occurring isotope of such atoms or a scarcer radio-isotope or nonradioactive isotope.

Synthetic methods for incorporating radio-isotopes into organic compounds are applicable to compounds of the invention and are well known in the art. These synthetic methods, for example, incorporating activity levels of tritium into target molecules, are as follows:
A. Catalytic Reduction with Tritium Gas: This procedure normally yields high specific activity products and requires halogenated or unsaturated precursors.
B. Reduction with Sodium Borohydride [³H]: This procedure is rather inexpensive and requires precursors containing reducible functional groups such as aldehydes, ketones, lactones, esters and the like.
C. Reduction with Lithium Aluminum Hydride [³H]: This procedure offers products at almost theoretical specific activities. It also requires precursors containing reducible functional groups such as aldehydes, ketones, lactones, esters and the like.
D. Tritium Gas Exposure Labeling: This procedure involves exposing precursors containing exchangeable protons to tritium gas in the presence of a suitable catalyst.
E. N-Methylation using Methyl Iodide [³H]: This procedure is usually employed to prepare O-methyl or N-methyl (³H) products by treating appropriate precursors with high specific activity methyl iodide (³H). This method in general allows for higher specific activity, such as for example, about 70-90 Ci/mmol.
Synthetic methods for incorporating activity levels of ¹²⁵I into target molecules include:
A. Sandmeyer and like reactions: This procedure transforms an aryl amine or a heteroaryl amine into a diazonium salt, such as a diazonium tetrafluoroborate salt and subsequently to 125I labeled compound using Na125I. A representative procedure was reported by Zhu, G-D. and co-workers in J. Org. Chem., 2002, 67, 943-948.
B. Ortho 125Iodination of phenols: This procedure allows for the incorporation of 125I at the ortho position of a phenol as reported by Collier, T. L. and co-workers in J. Labelled Compd. Radiopharm., 1999, 42, S264-S266.
C. Aryl and heteroaryl bromide exchange with 125I: This method is generally a two step process. The first step is the conversion of the aryl or heteroaryl bromide to the corresponding tri-alkyltin intermediate using for example, a Pd catalyzed reaction [i.e. Pd(Ph3P)4] or through an aryl or heteroaryl lithium, in the presence of a tri-alkyltinhalide or hexaalkylditin [e.g., (CH₃)₃SnSn(CH₃)₃]. A representative procedure was reported by Le Bas, M.-D. and co-workers in J. Labelled Compd. Radiopharm. 2001, 44, S280-S282.
The compounds described herein may generally be utilized as the free acid or free base.

Alternatively, the compounds may be used in the form of acid or base addition salts. Acid addition salts of the free amino compounds may be prepared by methods well known in the art, and may be formed from organic and inorganic acids. Suitable organic acids include maleic, fumaric, benzoic, ascorbic, succinic, methanesulfonic, acetic, trifluoroacetic, oxalic, propionic, tartaric, salicylic, citric, gluconic, lactic, mandelic, cinnamic, aspartic, stearic, palmitic, glycolic, glutamic, and benzenesulfonic acids. Suitable inorganic acids include hydrochloric, hydrobromic, sulfuric, phosphoric, and nitric acids. Base addition salts included those salts that form with the carboxylate anion and include salts formed with organic and inorganic cations such as those chosen from the alkali and alkaline earth metals (for example, lithium, sodium, potassium, magnesium, barium and calcium), as well as the ammonium ion and substituted derivatives thereof (for example, dibenzylammonium, benzylammonium, 2-hydroxyethylammonium, and the like). Thus, a "pharmaceutically acceptable salt" is intended to encompass any and all acceptable salt forms.

The compounds described herein may exist in a continuum of solid states ranging from fully amorphous to fully crystalline. Furthermore, some of the crystalline forms of the compounds may exist as polymorphs.

In addition, some of the compounds may also form solvates with water or other organic solvents. The term "solvate" is used herein to describe a molecular complex comprising a compound described herein and one or more pharmaceutically acceptable solvent molecules. When the solvent molecule is water, the molecular complex is referred to as a "hydrate". Such hydrates and solvates are similarly included within the scope of this disclosure.

As one of skill in the art would appreciate, any of the compounds described herein may incorporate isotopes. Accordingly, also contemplated is use of isotopically-labeled compounds identical to those described herein, wherein one or more atoms are replaced by an atom having an atomic mass or mass number different from the atomic mass or mass number usually found in nature. Examples of isotopes that can be incorporated into these compounds include isotopes of hydrogen, carbon, nitrogen, oxygen, fluorine and chlorine, such as, but not limited to, ²H, ³H, ¹³C, ¹⁴C, ¹⁵N, ¹⁸O, ¹⁷O, ¹⁸F, and ³⁶Cl. Certain isotopically-labeled compounds, for example those into which radioactive isotopes such as ³H and ¹⁴C are incorporated, are also useful in drug or substrate tissue distribution assays. Tritiated hydrogen (³H) and carbon-14 (¹⁴C) isotopes are particularly preferred for their ease of preparation and detectability. Substitution with heavier isotopes such as deuterium (²H or D) can provide certain therapeutic advantages resulting from greater metabolic stability, for example increased in vivo half-life or reduced dose requirements and, therefore, may be preferred in some circumstances. Isotopically-labeled compounds can generally be prepared by performing procedures routinely practiced in the art.

Provided is a method for selecting a subject for treatment with a VMAT2 inhibitor, comprising:
determining whether the subject has COMT haploinsufficiency; and
administering a therapeutically effective amount of a VMAT2 inhibitor to the subject if the subject has been determined to have COMT haploinsufficiency.

Also provided is a method for selecting a subject for treatment with a VMAT2 inhibitor, comprising:
determining whether the subject has COMT haploinsufficiency; and
administering a therapeutically effective amount of a VMAT2 inhibitor to the subject if the subject has been determined to have COMT haploinsufficiency,
wherein the VMAT2 inhibitor is not tetrabenazine or reserpine.

Also provided is a method for selecting a subject for treatment with a VMAT2 inhibitor, comprising:
determining whether the subject has 22q11.2 deletion syndrome; and
administering a therapeutically effective amount of a VMAT2 inhibitor to the subject if the subject has been determined to have 22q11.2 deletion syndrome.

Also provided is a method for selecting a subject for treatment with a VMAT2 inhibitor, comprising:
determining whether the subject has 22q11.2 deletion syndrome; and
administering a therapeutically effective amount of a VMAT2 inhibitor to the subject if the subject has been determined to have 22q11.2 deletion syndrome,
wherein the VMAT2 inhibitor is not tetrabenazine or reserpine.

Also provided is a method of treating a psychiatric disorder in a subject in need thereof, comprising:
administering a therapeutically effective amount of a VMAT2 inhibitor to the subject,
wherein the subject is predisposed to developing said psychiatric disorder due to the subject having COMT haploinsufficiency.

Also provided is a method of treating a psychiatric disorder in a subject in need thereof, comprising:
administering a therapeutically effective amount of a VMAT2 inhibitor to the subject,
wherein the subject is predisposed to developing said psychiatric disorder due to the subject having COMT haploinsufficiency,wherein the VMAT2 inhibitor is not tetrabenazine or reserpine.

Also provided is a method of treating a psychiatric disorder in a subject in need thereof, comprising:
administering a therapeutically effective amount of a VMAT2 inhibitor to the subject,
wherein the subject is predisposed to developing said psychiatric disorder due to the subject having 22q11.2 deletion syndrome.

Also provided is a method of treating a psychiatric disorder in a subject in need thereof, comprising:
administering a therapeutically effective amount of a VMAT2 inhibitor to the subject,
wherein the subject is predisposed to developing said psychiatric disorder due to the subject having 22q11.2 deletion syndrome,
wherein the VMAT2 inhibitor is not tetrabenazine or reserpine.

Also provided herein is a method of treating a psychiatric disorder in a subject in need thereof, comprising:
determining whether the subject has COMT haploinsufficiency; and
administering a therapeutically effective amount of a VMAT2 inhibitor to the subject if the subject has been determined to have COMT haploinsufficiency.

Also provided herein is a method of treating a psychiatric disorder in a subject in need thereof, comprising:
determining whether the subject has COMT haploinsufficiency; and
administering a therapeutically effective amount of a VMAT2 inhibitor to the subject if the subject has been determined to have COMT haploinsufficiency,
wherein the VMAT2 inhibitor is not tetrabenazine or reserpine.

Also provided herein is a method of treating a psychiatric disorder in a subject in need thereof, comprising:
determining whether the subject has 22q11.2 deletion syndrome; and
administering a therapeutically effective amount of a VMAT2 inhibitor to the subject if the subject has been determined to have 22q11.2 deletion syndrome.

Also provided herein is a method of treating a psychiatric disorder in a subject in need thereof, comprising:
determining whether the subject has 22q11.2 deletion syndrome; and
administering a therapeutically effective amount of a VMAT2 inhibitor to the subject if the subject has been determined to have 22q11.2 deletion syndrome,
wherein the VMAT2 inhibitor is not tetrabenazine or reserpine.

In some embodiments, the subject has a COMT108met variant on the nondeleted allele.

In some embodiments, determining whether the subject has 22q11.2 deletion syndrome comprises performing a genetic test to detect the presence or absence of a deletion at 22q11.2 in a sample from the subject. In some embodiments, fluorescence in situ hybridization (FISH) is used to identify the deletion. In some embodiments, microarray analysis (e.g., chromosomal microarray analysis) is used to identify the deletion. In some embodiments, multiple ligation-dependent probe amplification is used to identify the deletion.

In some embodiments, the methods provided herein further comprise obtaining results of the genetic test prior to initial administration of the VMAT2 inhibitor.

In some embodiments of the methods provided herein, the subject had been previously identified as having COMT haploinsufficiency. In some embodiments of the methods provided herein, the subject had been previously identified as having 22q11.2 deletion syndrome.

In some embodiments, the psychiatric disorder is schizophrenia spectrum disorder, attention deficit hyperactivity disorder, anxiety disorder, obsessive-compulsive disorder, autism spectrum disorder, mood disorder, anorexia nervosa, narcolepsy, heroin addiction, or early onset alcoholism. In some embodiments, the psychiatric disorder is schizophrenia spectrum disorder. In some embodiments, the psychiatric disorder is mood disorder, such as bipolar disorder. In some embodiments, the psychiatric disorder is autism spectrum disorder.

In some embodiments, the patient has a congenital defect of the cardiovascular system. In some embodiments, the patient has hypocalcemia and/or hypoparathyroidism. In some embodiments, the patient has hypothyroidism or hyperthyroidism. In some embodiments, the patient is obese. In some embodiments, the patient has recurrent seizures.

Each and every method, composition, or use described herein optionally includes the limitation "wherein the VMAT2 inhibitor is not tetrabenazine or reserpine."

In some embodiments, the VMAT2 inhibitor is tetrabenazine or reserpine.

In some embodiments, the VMAT2 inhibitor is selected from compounds of Formula (I) and pharmaceutically acceptable salts, solvates, and hydrates and/or isotopic variants thereof: 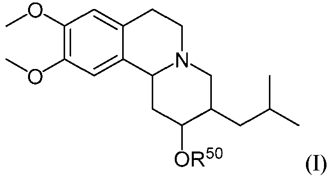 wherein:
R⁵⁰ is -C(=O)-O-(CR^{a}R^{b})ₘ or -C(=O)-(CR^{a}R^{b})ₙ-NH₂,
R^{a} and R^{b}, at each occurrence, are independently H, C₁₋₆alkyl, -NH-C(=NH)NH₂, -C(=O)OH, -C(=O)O-Me, -SH, -C(=O)NH₂, -NH₂, -SCH₃, phenyl, -OH, 4-hydroxy-phenyl, imidazolyl, or indolyl;
m is 1 to 6; and
n is 1 to 6.

In some embodiments, the VMAT2 inhibitor of Formula (I) is a compound of any one of the structures shown in Table 1, or a pharmaceutically acceptable salt, solvate, or hydrate and/or isotopic variant thereof.

**Table 1**

| Structure | Cmpd. No. |
|---|---|
| 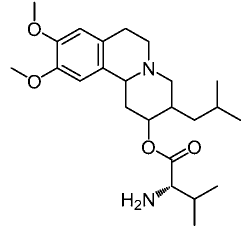 | 1-1 |
| 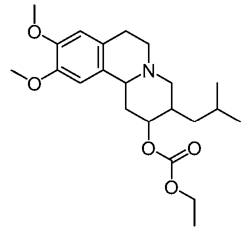 | 1-2 |
| 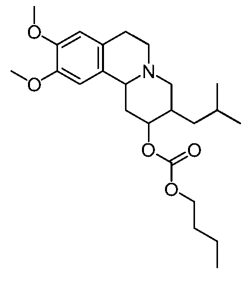 | 1-3 |
| 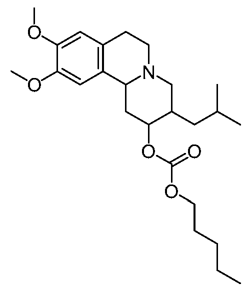 | 1-4 |
| 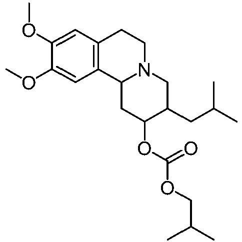 | 1-5 |
| 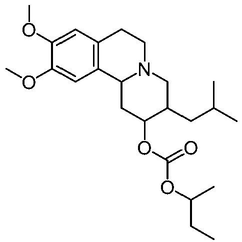 | 1-6 |
| 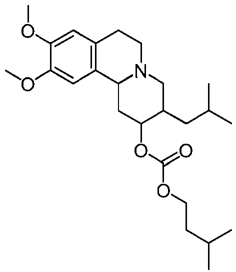 | 1-7 |
| 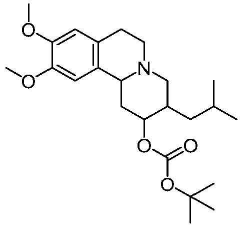 | 1-8 |
| 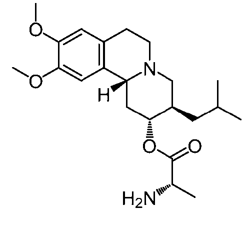 | 1-9 |
| 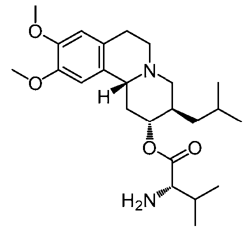 | 1-10 |
| 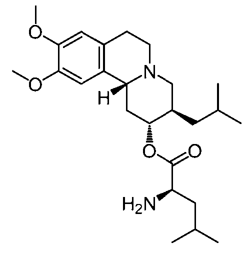 | 1-11 |
| 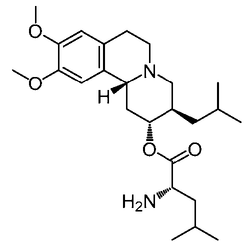 | 1-12 |
| 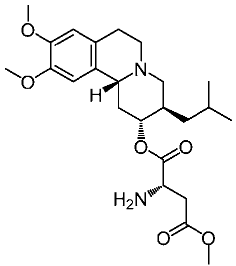 | 1-13 |
| 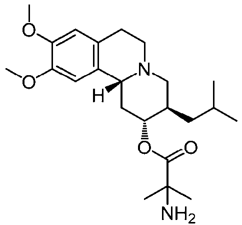 | 1-14 |
| 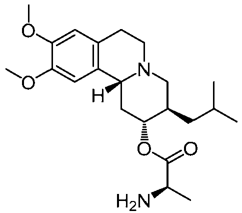 | 1-15 |
| 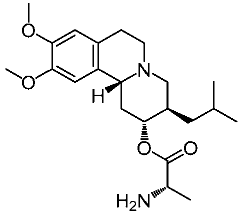 | 1-16 |
| 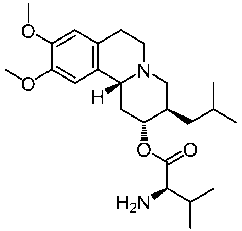 | 1-17 |
| 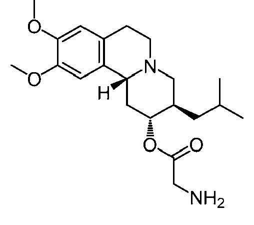 | 1-18 |
| 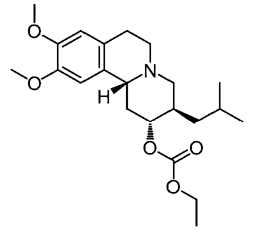 | 1-19 |
| 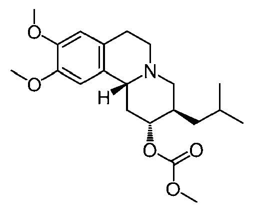 | 1-20 |
| 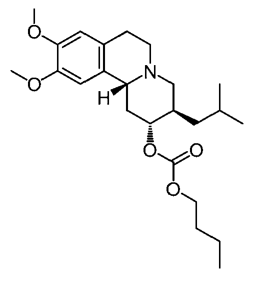 | 1-21 |
| 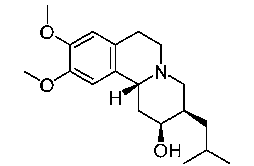 | 1-22 |
| 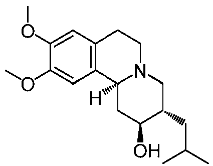 | 1-23 |
| 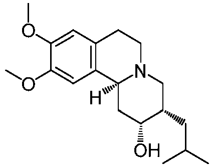 | 1-24 |

In some embodiments, the VMAT2 inhibitor of Formula (I) is:

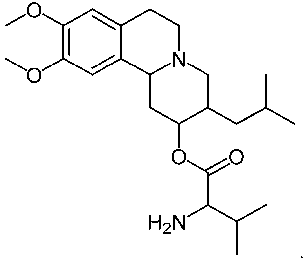

In some embodiments, the VMAT2 inhibitor of Formula (I) is:

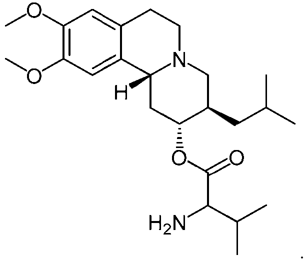

In some embodiments, the VMAT2 inhibitor of Formula (I) is:

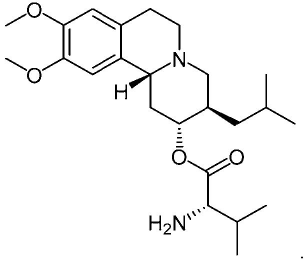

In some embodiments, a pharmaceutically acceptable salt of Formula (I) is a ditosylate salt. In some embodiments, the VMAT2 inhibitor is a ditosylate salt of the following structure:

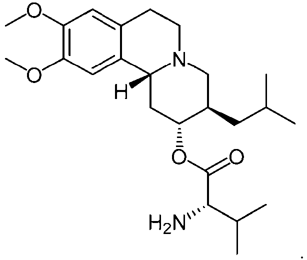

In some embodiments, the isotopic variant of Formula (I) is a compound of Formula (I), or a pharmaceutically acceptable salt, solvate or hydrate thereof, wherein 1, 2, 3, 4, 5, or 6 hydrogens are replaced by deuterium.

In some embodiments, the isotopic variant of Formula (I), is a compound of Formula (Ia), or a pharmaceutically acceptable salt, solvate, or hydrate thereof: 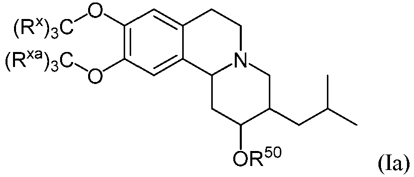 wherein:
each R^{x} is independently selected from hydrogen (¹H) and deuterium (²H);
each R^{xa} is independently selected from hydrogen (¹H) and deuterium (²H);
R⁵⁰ is -C(=O)-O-(CR^{a}R^{b})ₘ or -C(=O)-(CR^{a}R^{b})ₙ-NH₂,
R^{a} and R^{b}, at each occurrence, are independently H, C₁₋₆alkyl, -NH-C(=NH)NH₂, -C(=O)OH, -C(=O)O-Me, -SH, -C(=O)NH₂, -NH₂, -SCH₃, phenyl, -OH, 4-hydroxy-phenyl, imidazolyl, or indolyl;
m is 1 to 6; and
n is 1 to 6.

In some embodiments of the compound of Formula (Ia), or a pharmaceutically acceptable salt, solvate, or hydrate thereof, each R^{x} is deuterium.

In some embodiments of the compound of Formula (Ia), or a pharmaceutically acceptable salt, solvate, or hydrate thereof, each R^{xa} is deuterium.

In some embodiments of the compound of Formula (Ia), or a pharmaceutically acceptable salt, solvate, or hydrate thereof, each R^{x} is deuterium; and each R^{xa} is deuterium.

In some embodiments, the VMAT2 inhibitor is selected from compounds of Formula (II): 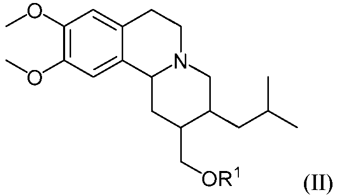 and pharmaceutically acceptable hydrates, solvates, and salts and/or isotopic variants thereof, wherein:
R¹ is
   a) hydrogen;
   b) -P(=O)(OR³)₂;
   c) -C(=O)alkyl, wherein alkyl is optionally substituted with R¹⁰ and/or R²⁰;
   d) -C(=O)heterocyclyl, wherein heterocyclyl is optionally substituted with R¹⁰ and/or R²⁰;
   e) -C(=O)carbocyclyl, wherein carbocyclyl is optionally substituted with R¹⁰ and/or R²⁰;
   f) -C(=O)N(R₃)alkyl, wherein alkyl is optionally substituted with R¹⁰ and/or R²⁰;
   g) -C(=O)N(R₃)carbocyclyl, wherein carbocyclyl is optionally substituted with R¹⁰ and/or R²⁰;
   h) -C(=O)Oalkyl, wherein alkyl is optionally substituted with R¹⁰ and/or R²⁰; or
   i) alkyl, wherein alkyl is optionally substituted with R¹⁰ and/or R²⁰;
and wherein,
each R³ is independently hydrogen or alkyl;
each R¹⁰ is independently halo, haloalkyl, cyano, nitro, trimethylsilanyl, -OR³⁰, -SR³⁰, -OC(=O)-R³⁰, -N(R³⁰)₂, -C(=O)R³⁰, -C(=O)OR³⁰, -C(=O)N(R³⁰)₂, -N(R³⁰)C(=O)OR³¹, -N(R³⁰)C(=O)R³¹, -N(R³⁰)C(=NR³¹)N(R³²)₂, -N(R³⁰)S(=O)ₜR³¹ (where t is 1 to 2), -S(=O)ₜOR³⁰ (where t is 1 to 2), -S(=O)ₚR³⁰ (where p is 0 to 2), -S(=O)ₜN(R³⁰)₂ (where t is 1 to 2), or -OP(=O)(OR³⁰)₂, or when a single atom bears two R¹⁰ groups such two R¹⁰ groups may be taken together to form oxo;
each R²⁰ is independently alkyl, alkenyl, aryl, aralkyl, cycloalkyl, cycloalkylalkyl, heterocyclyl, heterocyclylalkyl, heteroaryl, or heteroarylalkyl, or when a single atom bears two R²⁰ groups such two R²⁰ groups may be taken together to form cycloalkyl, wherein each of said alkyl, alkenyl, aryl, aralkyl, cycloalkyl, cycloalkylalkyl, heterocyclyl, heterocyclylalkyl, heteroaryl, and heteroarylalkyl groups is optionally substituted with R¹⁰ and/or R²²;
each R²² is independently alkyl, alkenyl, aryl, aralkyl, cycloalkyl, cycloalkylalkyl, heterocyclyl, heterocyclylalkyl, heteroaryl, or heteroarylalkyl, wherein each of said alkyl, alkenyl, aryl, aralkyl, cycloalkyl, cycloalkylalkyl, heterocyclyl, heterocyclylalkyl, heteroaryl, and heteroarylalkyl groups is optionally substituted with R¹⁰; and
each R³⁰, R³¹, and R³² is independently hydrogen or alkyl.

In some embodiments, the VMAT2 inhibitor of Formula (II) is:

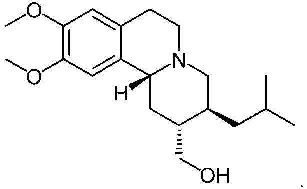

In some embodiments, the VMAT2 inhibitor of Formula (II) is a compound of any one of the structures shown in Table 2, or a pharmaceutically acceptable salt, solvate, or hydrate and/or isotopic variant thereof.

**Table 2**

| Structure | Cmpd. No. |
|---|---|
| 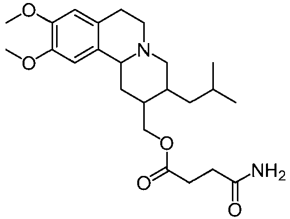 | 2-1 |
| 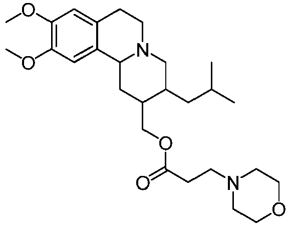 | 2-2 |
| 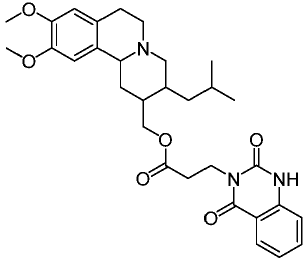 | 2-3 |
| 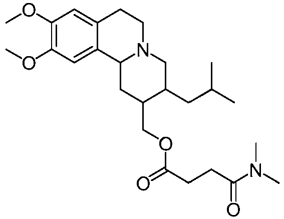 | 2-4 |
| 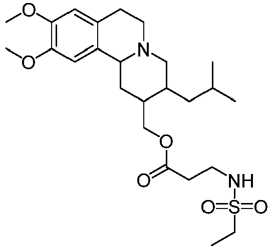 | 2-5 |
| 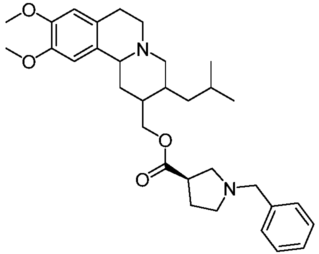 | 2-6 |
| 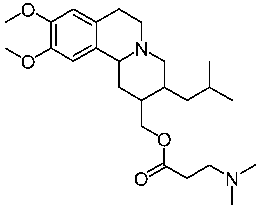 | 2-7 |
| 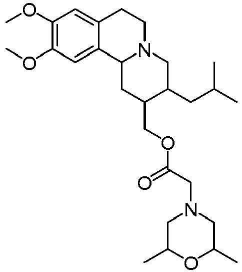 | 2-8 |
| 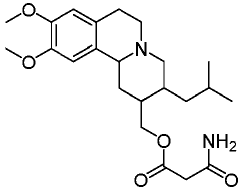 | 2-9 |
| 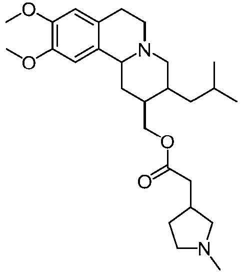 | 2-10 |
| 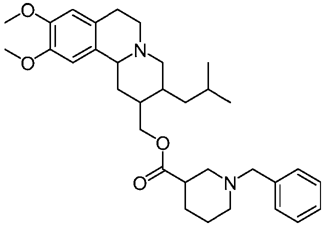 | 2-11 |
| 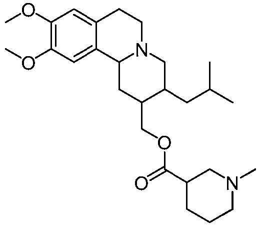 | 2-12 |
| 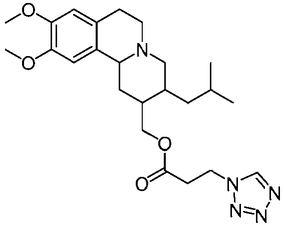 | 2-13 |
| 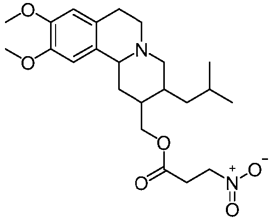 | 2-14 |
| 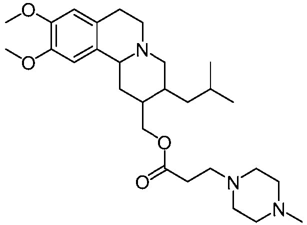 | 2-15 |
| 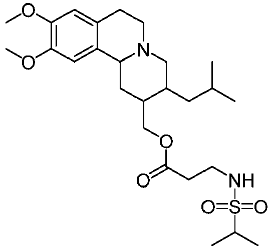 | 2-16 |
| 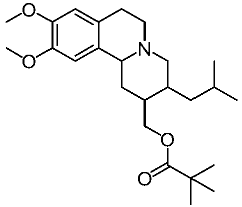 | 2-17 |
| 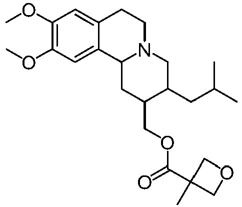 | 2-18 |
| 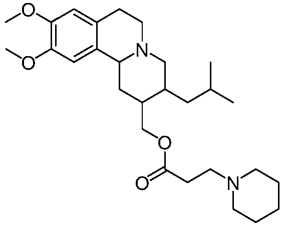 | 2-19 |
| 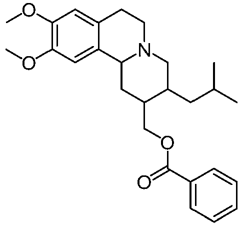 | 2-20 |
| 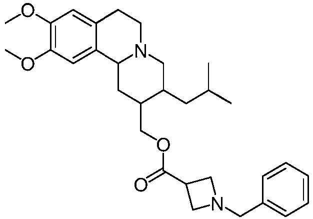 | 2-21 |
| 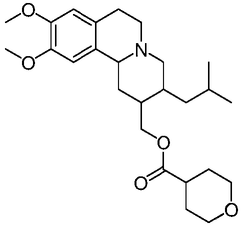 | 2-22 |
| 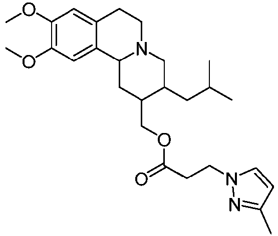 | 2-23 |
| 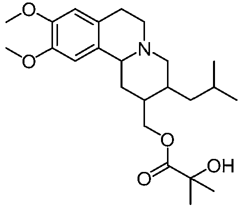 | 2-24 |
| 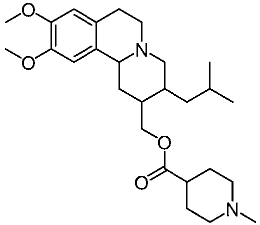 | 2-25 |
| 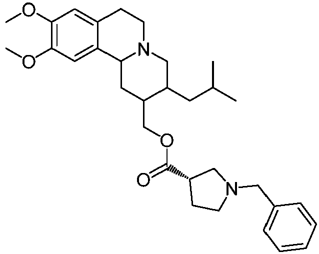 | 2-26 |
| 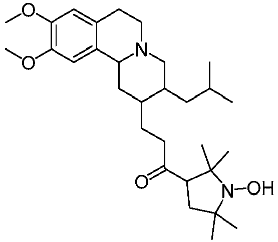 | 2-27 |
| 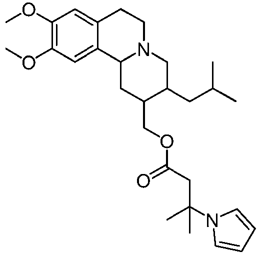 | 2-28 |
| 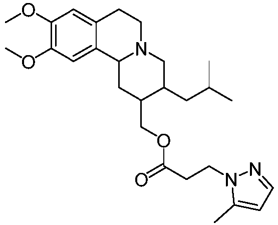 | 2-29 |
| 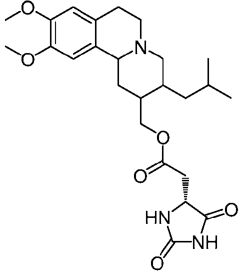 | 2-30 |
| 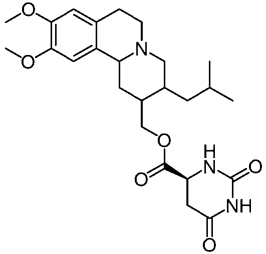 | 2-31 |
| 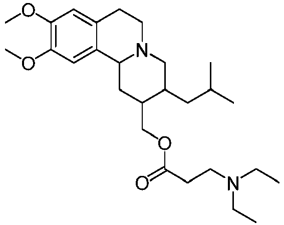 | 2-32 |
| 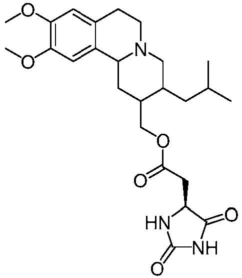 | 2-33 |
| 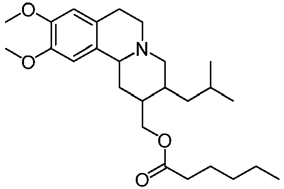 | 2-34 |
| 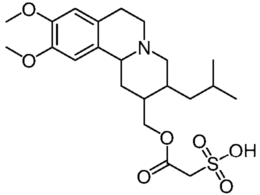 | 2-35 |
| 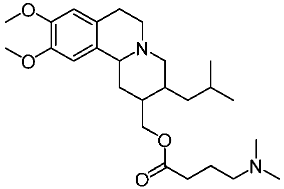 | 2-36 |
| 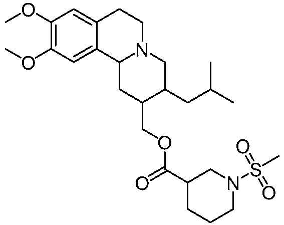 | 2-37 |
| 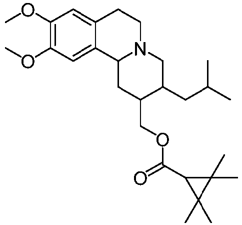 | 2-38 |
| 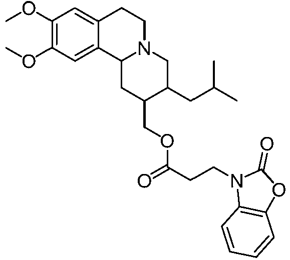 | 2-39 |
| 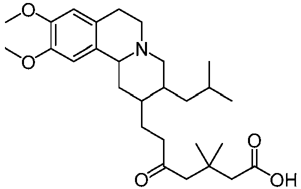 | 2-40 |
| 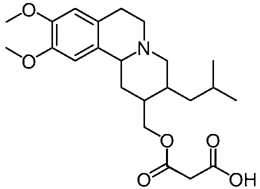 | 2-41 |
| 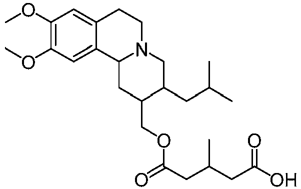 | 2-42 |
| 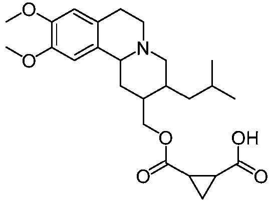 | 2-43 |
| 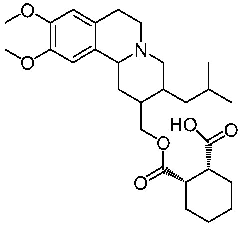 | 2-44 |
| 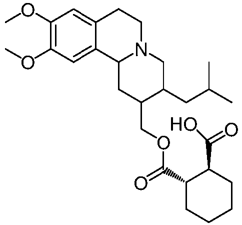 | 2-45 |
| 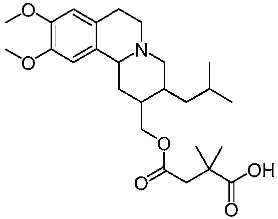 | 2-46 |
| 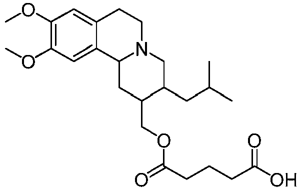 | 2-47 |
| 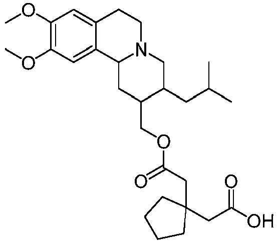 | 2-48 |
| 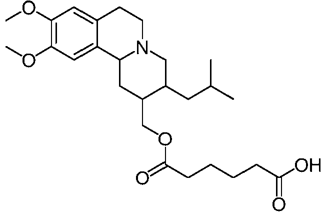 | 2-49 |
| 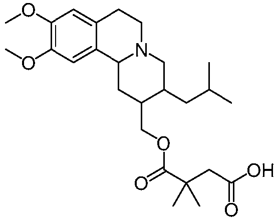 | 2-50 |
| 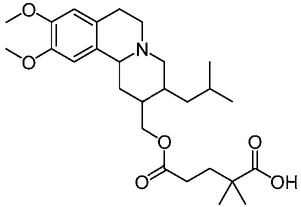 | 2-51 |
| 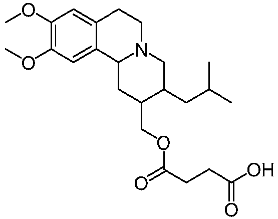 | 2-52 |
| 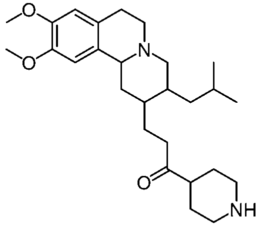 | 2-53 |
| 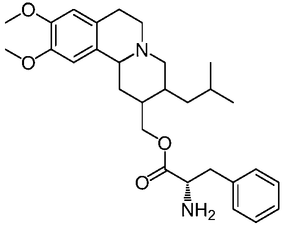 | 2-54 |
| 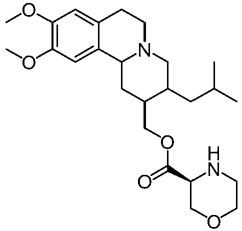 | 2-55 |
| 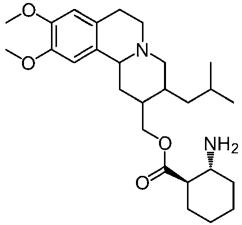 | 2-56 |
| 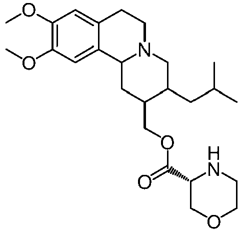 | 2-57 |
| 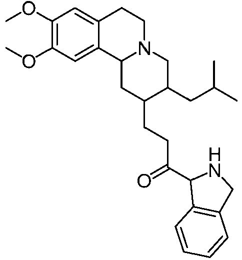 | 2-58 |
| 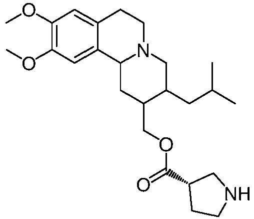 | 2-59 |
| 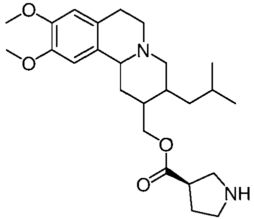 | 2-60 |
| 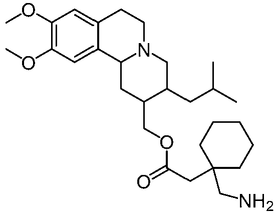 | 2-61 |
| 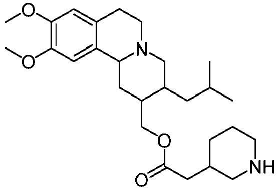 | 2-62 |
| 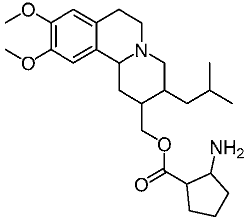 | 2-63 |
| 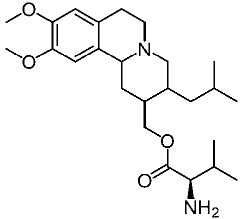 | 2-64 |
| 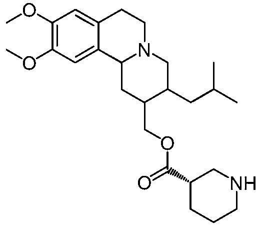 | 2-65 |
| 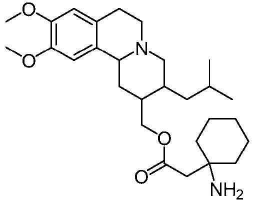 | 2-66 |
| 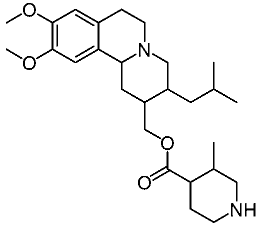 | 2-67 |
| 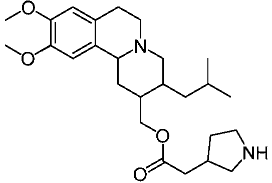 | 2-68 |
| 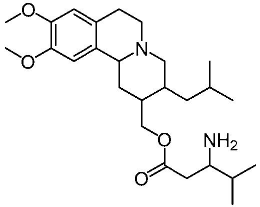 | 2-69 |
| 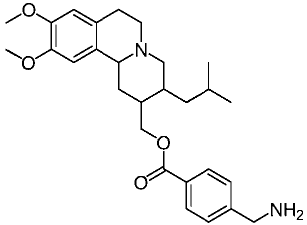 | 2-70 |
| 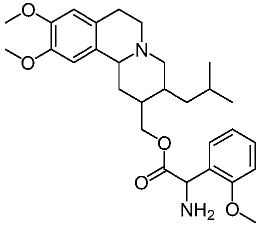 | 2-71 |
| 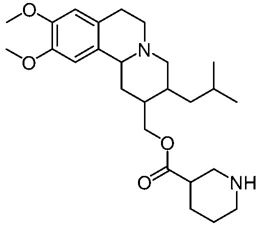 | 2-72 |
| 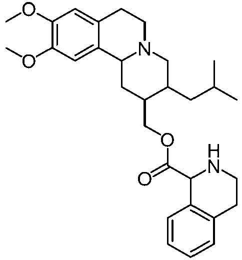 | 2-73 |
| 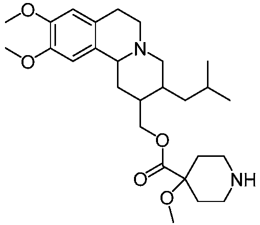 | 2-74 |
| 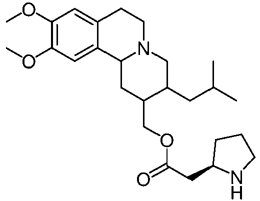 | 2-75 |
| 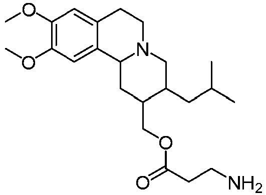 | 2-76 |
| 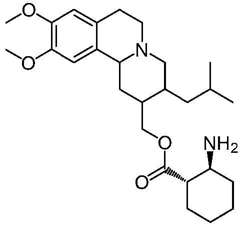 | 2-77 |
| 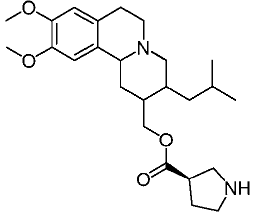 | 2-78 |
| 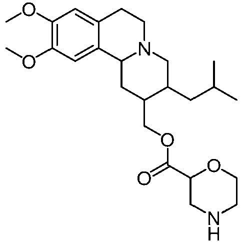 | 2-79 |
| 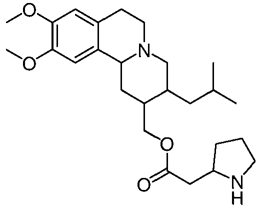 | 2-80 |
| 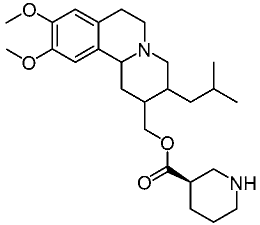 | 2-81 |
| 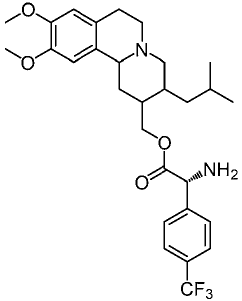 | 2-82 |
| 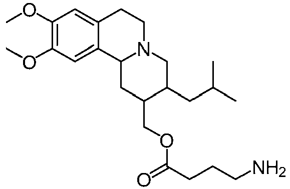 | 2-83 |
| 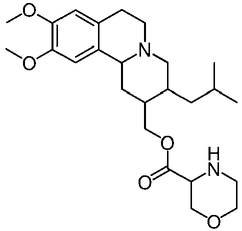 | 2-84 |
| 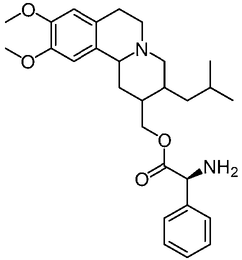 | 2-85 |
| 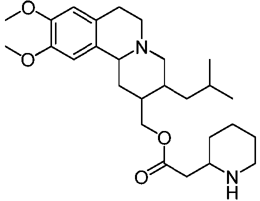 | 2-86 |
| 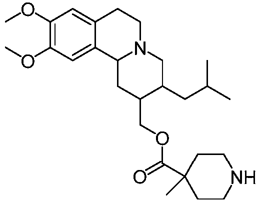 | 2-87 |
| 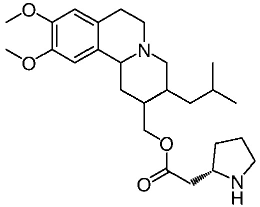 | 2-88 |
| 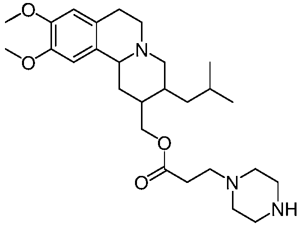 | 2-89 |
| 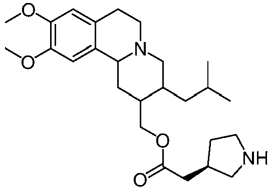 | 2-90 |
| 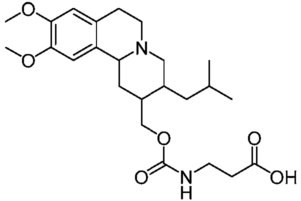 | 2-91 |
| 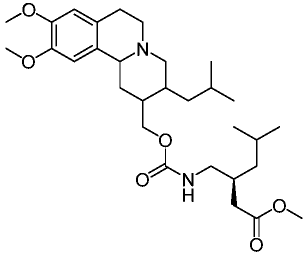 | 2-92 |
| 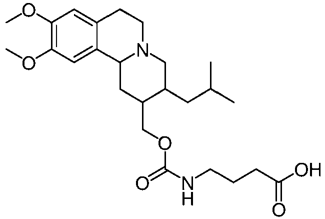 | 2-93 |
| 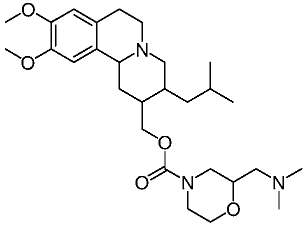 | 2-94 |
| 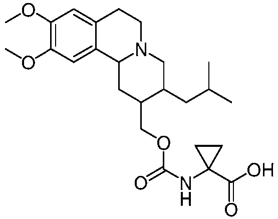 | 2-95 |
| 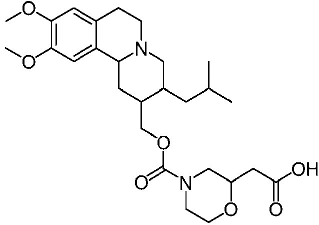 | 2-96 |
| 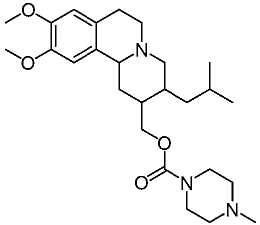 | 2-97 |
| 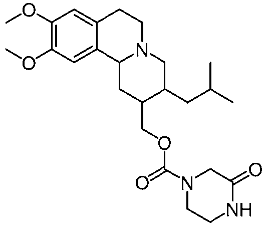 | 2-98 |
| 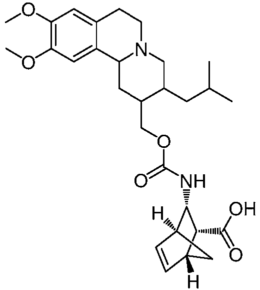 | 2-99 |
| 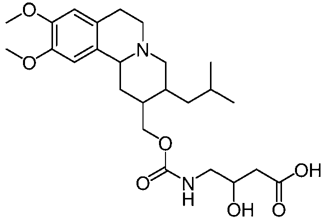 | 2-100 |
| 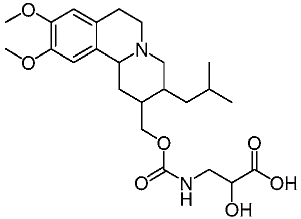 | 2-101 |
| 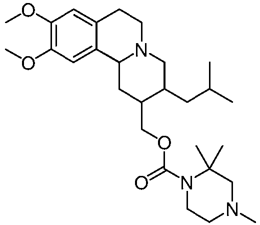 | 2-102 |
| 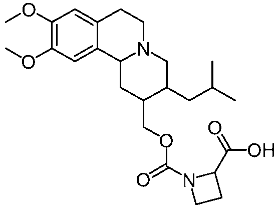 | 2-103 |
| 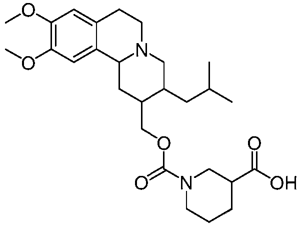 | 2-104 |
| 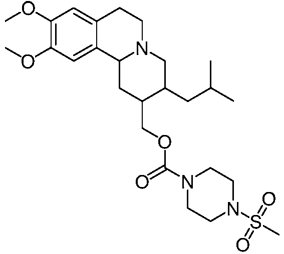 | 2-105 |
| 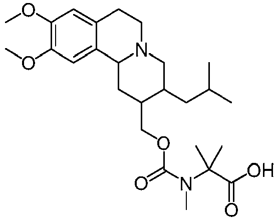 | 2-106 |
| 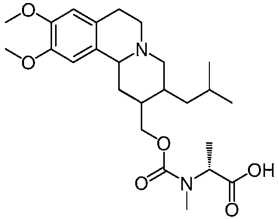 | 2-107 |
| 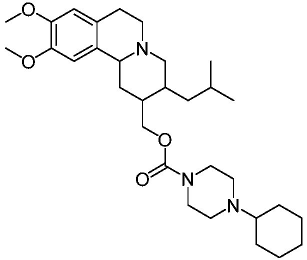 | 2-108 |
| 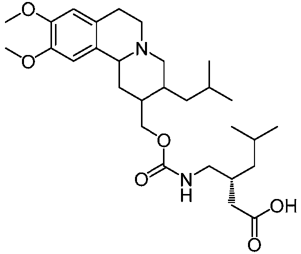 | 2-109 |
| 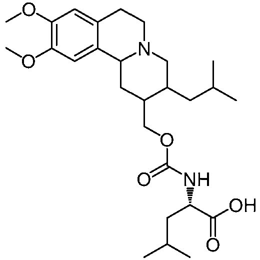 | 2-110 |
| 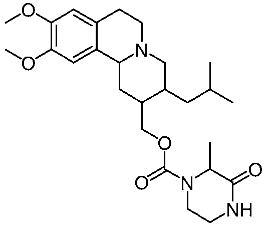 | 2-111 |
| 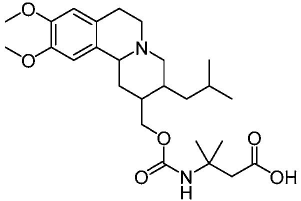 | 2-112 |
| 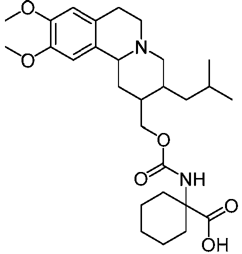 | 2-113 |
| 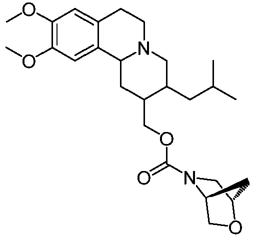 | 2-114 |
| 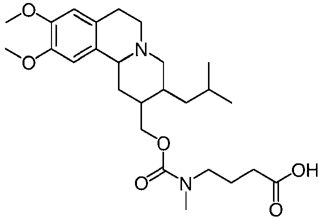 | 2-115 |
| 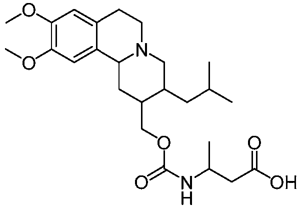 | 2-116 |
| 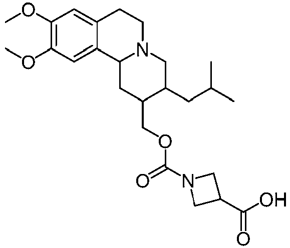 | 2-117 |
| 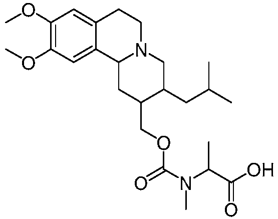 | 2-118 |
| 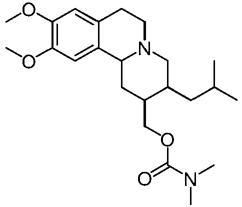 | 2-119 |
| 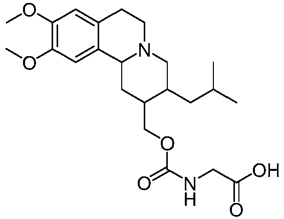 | 2-120 |
| 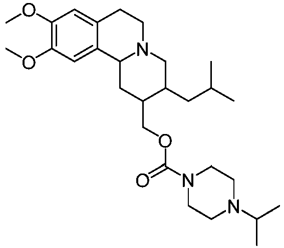 | 2-121 |
| 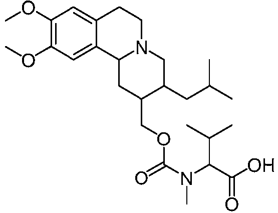 | 2-122 |
| 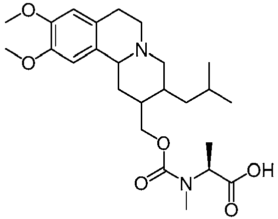 | 2-123 |
| 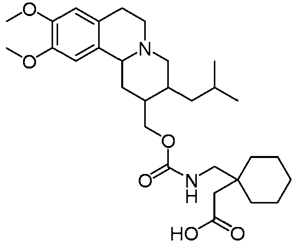 | 2-124 |
| 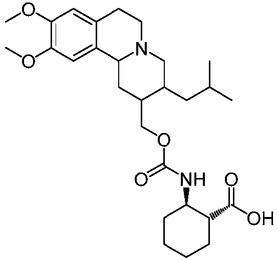 | 2-125 |
| 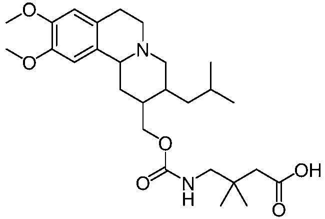 | 2-126 |
| 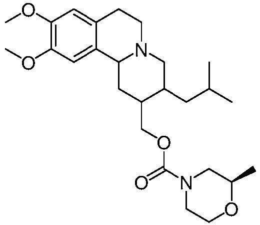 | 2-127 |
| 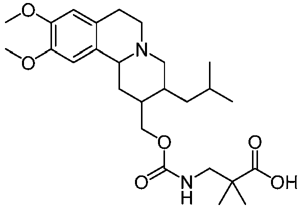 | 2-128 |
| 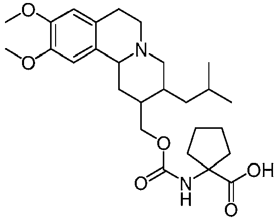 | 2-129 |
| 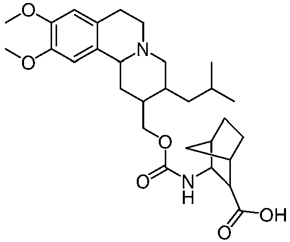 | 2-130 |
| 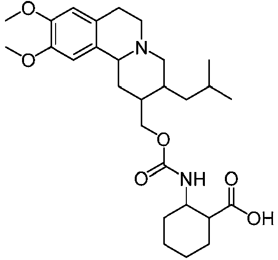 | 2-131 |
| 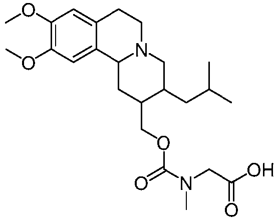 | 2-132 |
| 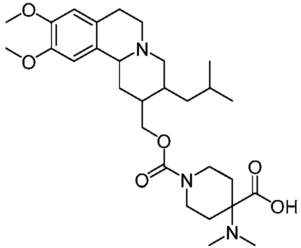 | 2-133 |
| 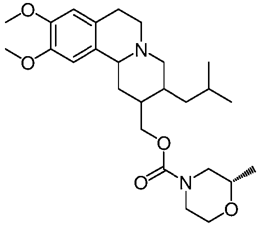 | 2-134 |
| 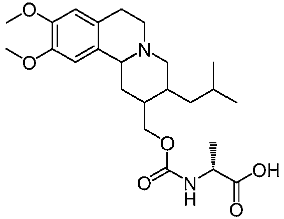 | 2-135 |
| 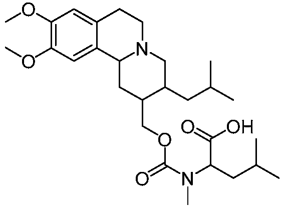 | 2-136 |
| 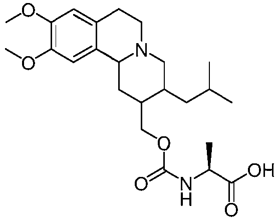 | 2-137 |
| 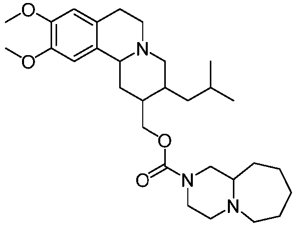 | 2-138 |
| 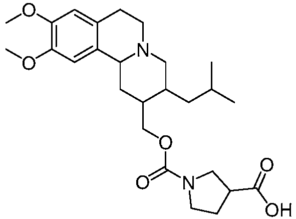 | 2-139 |
| 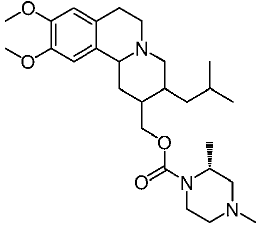 | 2-140 |
| 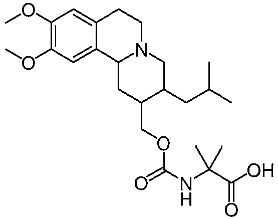 | 2-141 |
| 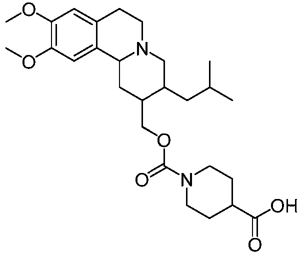 | 2-142 |
| 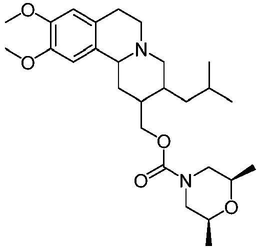 | 2-143 |
| 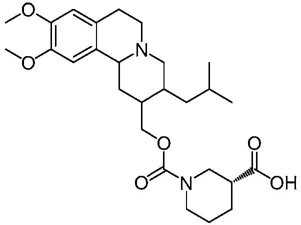 | 2-144 |
| 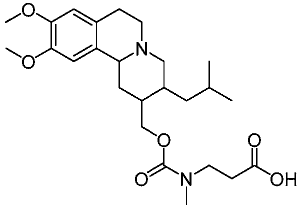 | 2-145 |
| 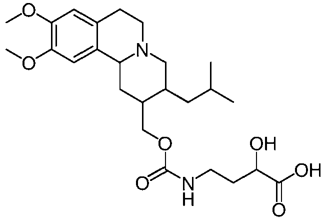 | 2-146 |
| 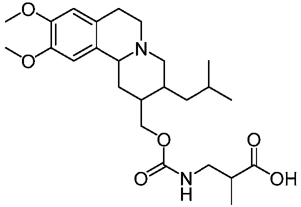 | 2-147 |
| 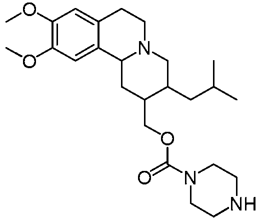 | 2-148 |
| 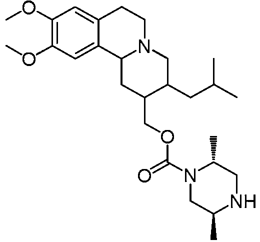 | 2-149 |
| 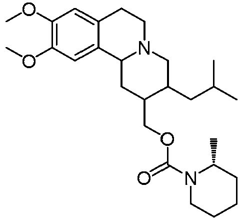 | 2-150 |
| 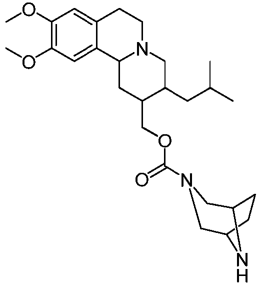 | 2-151 |
| 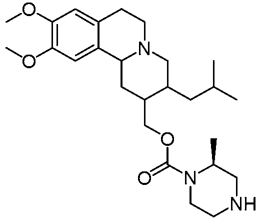 | 2-152 |
| 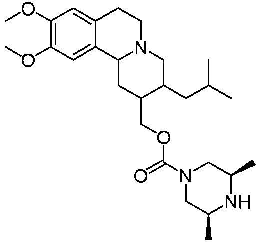 | 2-153 |
| 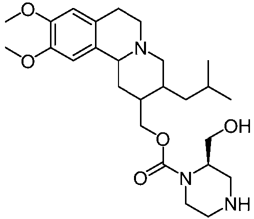 | 2-154 |
| 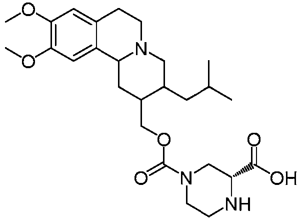 | 2-155 |
| 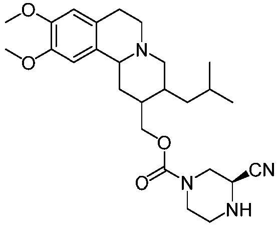 | 2-156 |
| 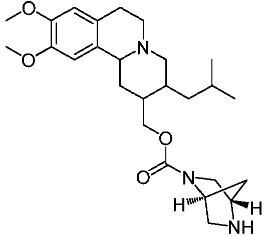 | 2-157 |
| 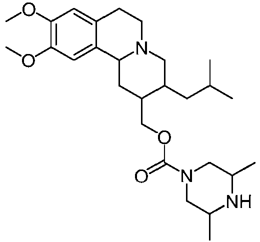 | 2-158 |
| 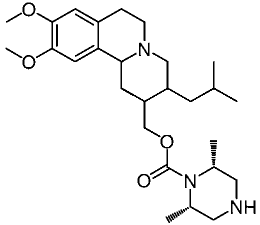 | 2-159 |
| 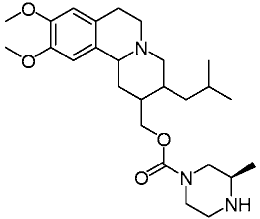 | 2-160 |
| 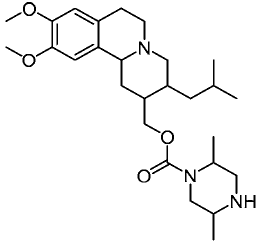 | 2-161 |
| 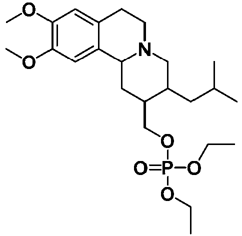 | 2-162 |
| 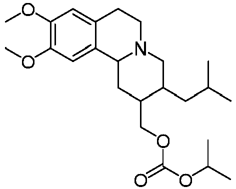 | 2-163 |
| 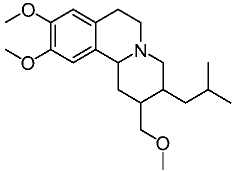 | 2-164 |
| 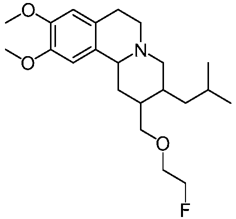 | 2-165 |
| 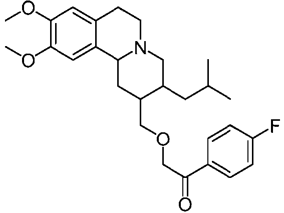 | 2-166 |
| 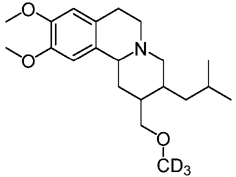 | 2-167 |
| 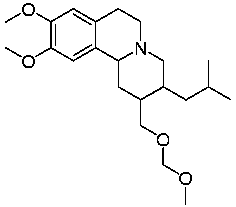 | 2-168 |
| 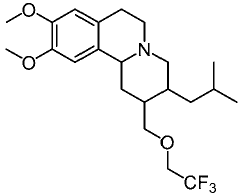 | 2-169 |
| 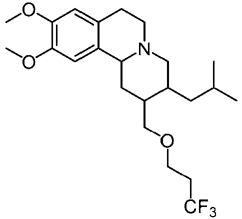 | 2-170 |
| 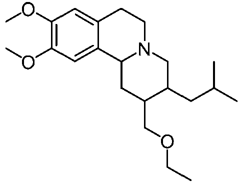 | 2-171 |
| 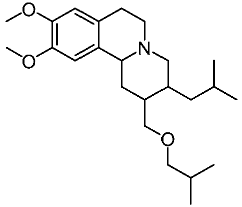 | 2-172 |
| 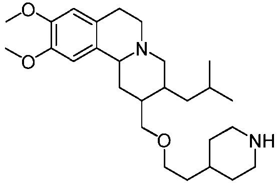 | 2-173 |
| 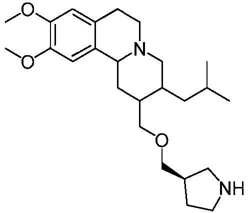 | 2-174 |
| 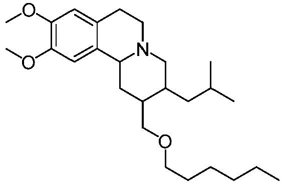 | 2-175 |
| 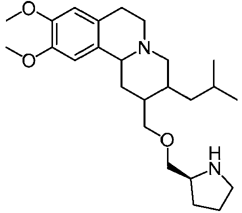 | 2-176 |
| 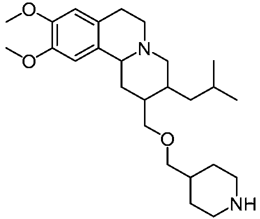 | 2-177 |
| 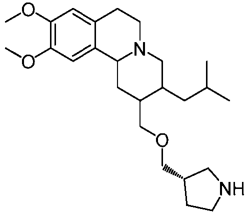 | 2-178 |
| 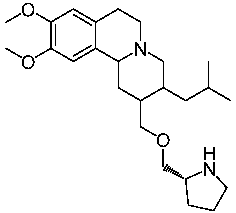 | 2-179 |

In some embodiments, the isotopic variant of Formula (II) is a compound of Formula (II), or a pharmaceutically acceptable salt, solvate, or hydrate thereof, wherein 1, 2, 3, 4, 5, or 6 hydrogens are replaced by deuterium.

In some embodiments, the isotopic variant of Formula (II) is a compound of Formula (IIa), or a pharmaceutically acceptable salt, solvate, or hydrate thereof: 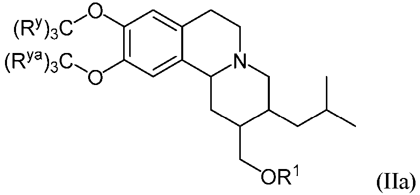 wherein:
each R^{y} is independently selected from hydrogen (¹H) and deuterium (²H);
each R^{ya} is independently selected from hydrogen (¹H) and deuterium (²H);
R¹ is
   a) hydrogen;
   b) -P(=O)(OR³)₂;
   c) -C(=O)alkyl, wherein alkyl is optionally substituted with R¹⁰ and/or R²⁰;
   d) -C(=O)heterocyclyl, wherein heterocyclyl is optionally substituted with R¹⁰ and/or R²⁰;
   e) -C(=O)carbocyclyl, wherein carbocyclyl is optionally substituted with R¹⁰ and/or R²⁰;
   f) -C(=O)N(R₃)alkyl, wherein alkyl is optionally substituted with R¹⁰ and/or R²⁰;
   g) -C(=O)N(R₃)carbocyclyl, wherein carbocyclyl is optionally substituted with R¹⁰ and/or R²⁰;
   h) -C(=O)Oalkyl, wherein alkyl is optionally substituted with R¹⁰ and/or R²⁰; or
   i) alkyl, wherein alkyl is optionally substituted with R¹⁰ and/or R²⁰;
and wherein,
each R³ is independently hydrogen or alkyl;
each R¹⁰ is independently halo, haloalkyl, cyano, nitro, trimethylsilanyl, -OR³⁰, -SR³⁰, -OC(=O)-R³⁰, -N(R³⁰)₂, -C(=O)R³⁰, -C(=O)OR³⁰, -C(=O)N(R³⁰)₂, -N(R³⁰)C(=O)OR³¹, -N(R³⁰)C(=O)R³¹, -N(R³⁰)C(=NR³¹)N(R³²)₂, -N(R³⁰)S(=O)ₜR³¹ (where t is 1 to 2), -S(=O)ₜOR³⁰ (where t is 1 to 2), -S(=O)ₚR³⁰ (where p is 0 to 2), -S(=O)ₜN(R³⁰)₂ (where t is 1 to 2), or -OP(=O)(OR³⁰)₂, or when a single atom bears two R¹⁰ groups such two R¹⁰ groups may be taken together to form oxo;
each R²⁰ is independently alkyl, alkenyl, aryl, aralkyl, cycloalkyl, cycloalkylalkyl, heterocyclyl, heterocyclylalkyl, heteroaryl, or heteroarylalkyl, or when a single atom bears two R²⁰ groups such two R²⁰ groups may be taken together to form cycloalkyl, wherein each of said alkyl, alkenyl, aryl, aralkyl, cycloalkyl, cycloalkylalkyl, heterocyclyl, heterocyclylalkyl, heteroaryl, and heteroarylalkyl groups is optionally substituted with R¹⁰ and/or R²²;
each R²² is independently alkyl, alkenyl, aryl, aralkyl, cycloalkyl, cycloalkylalkyl, heterocyclyl, heterocyclylalkyl, heteroaryl, or heteroarylalkyl, wherein each of said alkyl, alkenyl, aryl, aralkyl, cycloalkyl, cycloalkylalkyl, heterocyclyl, heterocyclylalkyl, heteroaryl, and heteroarylalkyl groups is optionally substituted with R¹⁰; and
each R³⁰, R³¹, and R³² is independently hydrogen or alkyl.

In some embodiments of the compound of Formula (IIa), or a pharmaceutically acceptable salt, solvate, or hydrate thereof, each R^{y} is deuterium.

In some embodiments of the compound of Formula (IIa), or a pharmaceutically acceptable salt, solvate, or hydrate thereof, each R^{ya} is deuterium.

In some embodiments of the compound of Formula (IIa), or a pharmaceutically acceptable salt, solvate, or hydrate thereof, each R^{y} is deuterium; and each R^{ya} is deuterium.

In some embodiments, the VMAT2 inhibitor is selected from compounds of Formula (III): 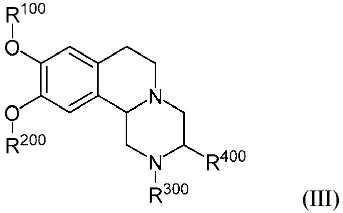 and pharmaceutically acceptable hydrates, solvates, and salts and/or isotopic variants thereof, wherein:
R¹⁰⁰ and R²⁰⁰ are independently lower alkyl, lower cycloalkyl, or lower cycloalkylalkyl, wherein each lower alkyl, lower cycloalkyl, and lower cycloalkylalkyl is independently unsubstituted or substituted with one or more halo, cyano, or lower alkoxy;
R³⁰⁰ is lower alkyl;
R⁴⁰⁰ is lower alkyl or lower cycloalkylalkyl.

In some embodiments, the VMAT2 inhibitor is selected from compounds of Formula (IV): 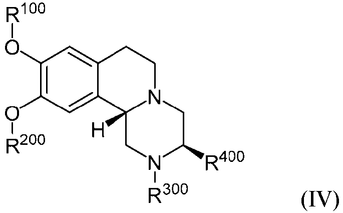 and pharmaceutically acceptable hydrates, solvates, and salts and/or isotopic variants thereof.

In some embodiments, the VMAT2 inhibitor of Formula (III) or Formula (IV) is a compound of any one of the structures shown in Table 3, or a pharmaceutically acceptable salt, solvate, or hydrate and/or isotopic variant thereof.

**Table 3**

| Structure | Cmpd. No. |
|---|---|
| 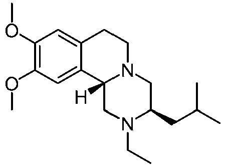 | 3-1 |
| 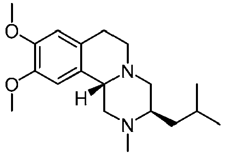 | 3-2 |
| 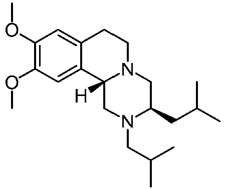 | 3-3 |
| 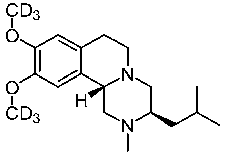 | 3-4 |
| 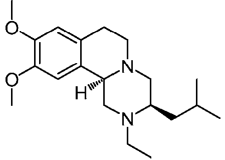 | 3-5 |
| 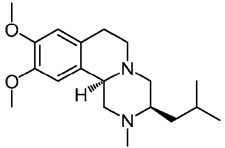 | 3-6 |
| 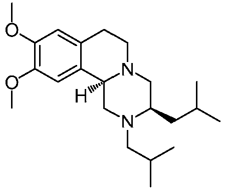 | 3-7 |
| 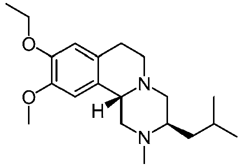 | 3-8 |
| 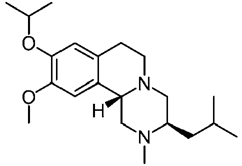 | 3-9 |
| 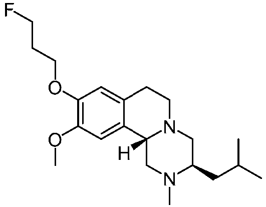 | 3-10 |
| 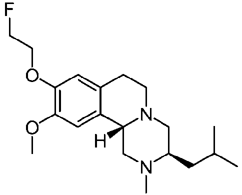 | 3-11 |
| 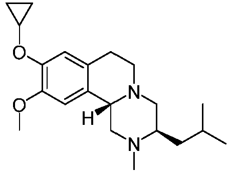 | 3-12 |
| 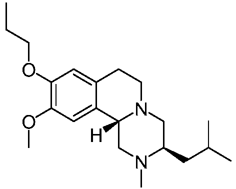 | 3-13 |
| 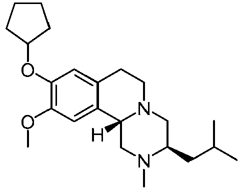 | 3-14 |
| 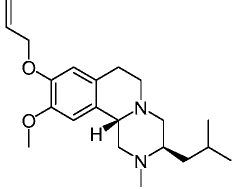 | 3-15 |
| 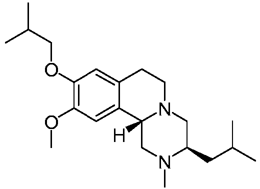 | 3-16 |
| 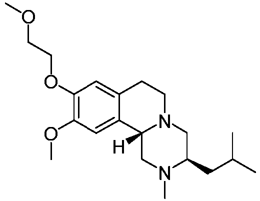 | 3-17 |
| 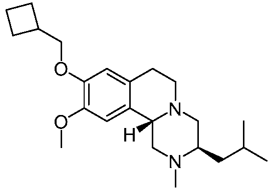 | 3-18 |
| 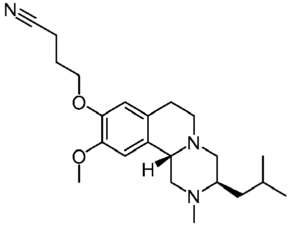 | 3-19 |
| 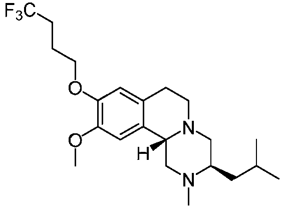 | 3-20 |
| 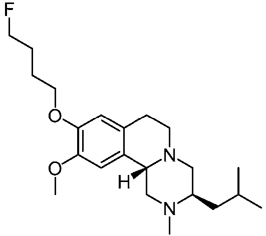 | 3-21 |
| 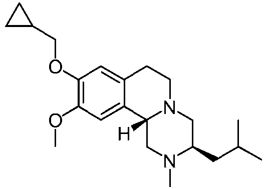 | 3-22 |
| 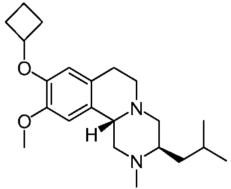 | 3-23 |
| 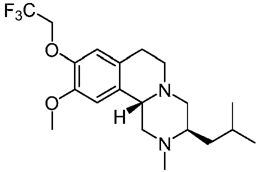 | 3-24 |
| 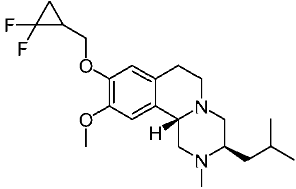 | 3-25 |
| 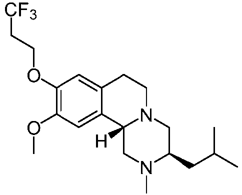 | 3-26 |
| 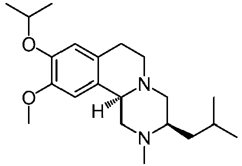 | 3-27 |
| 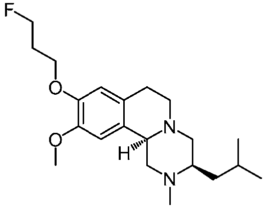 | 3-28 |
| 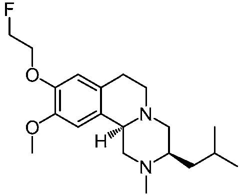 | 3-29 |
| 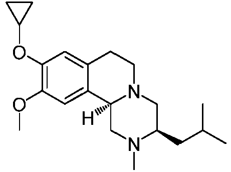 | 3-30 |
| 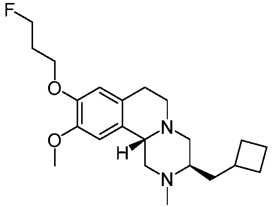 | 3-31 |
| 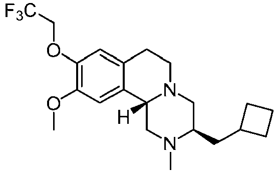 | 3-32 |
| 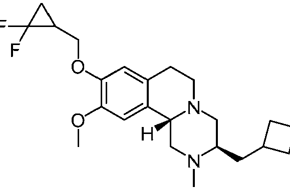 | 3-33 |
| 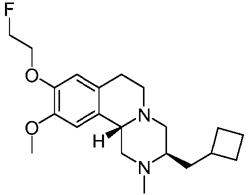 | 3-34 |
| 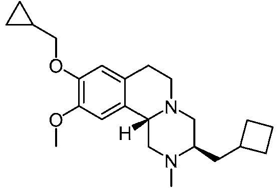 | 3-35 |
| 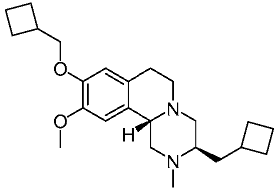 | 3-36 |
| 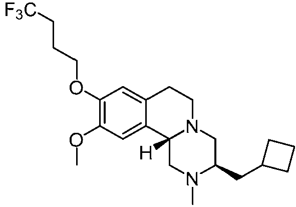 | 3-37 |
| 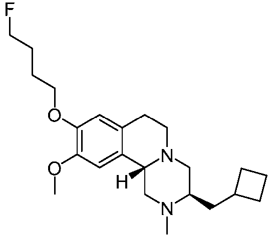 | 3-38 |
| 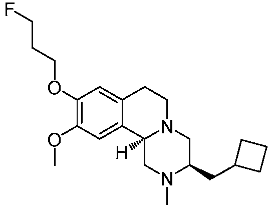 | 3-39 |
| 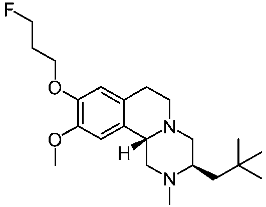 | 3-40 |
| 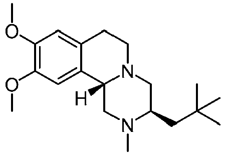 | 3-41 |
| 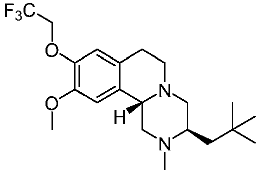 | 3-42 |
| 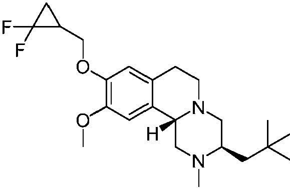 | 3-43 |
| 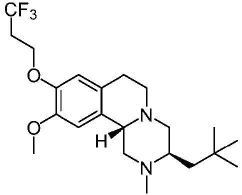 | 3-44 |
| 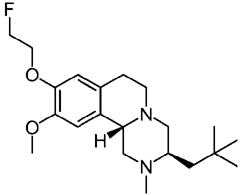 | 3-45 |
| 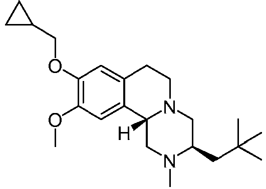 | 3-46 |
| 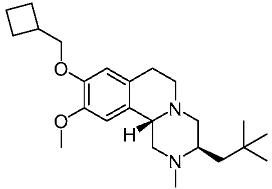 | 3-47 |
| 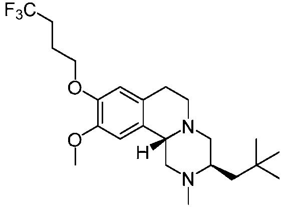 | 3-48 |
| 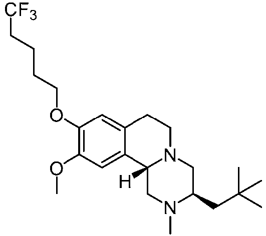 | 3-49 |
| 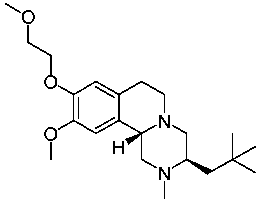 | 3-50 |
| 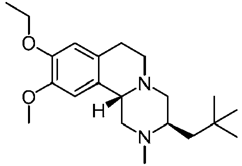 | 3-51 |
| 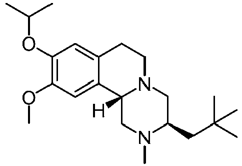 | 3-52 |
| 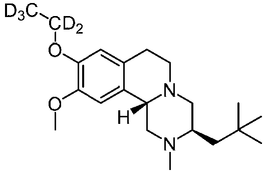 | 3-53 |
| 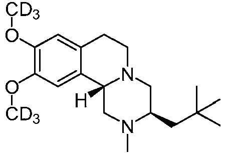 | 3-54 |
| 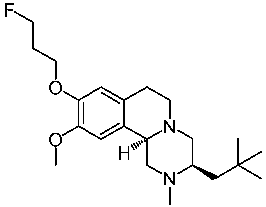 | 3-55 |
| 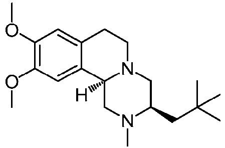 | 3-56 |

In some embodiments, the isotopic variant of Formula (III) or (IV) is a compound of Formula (III) or (IV), or a pharmaceutically acceptable salt, solvate, or hydrate thereof, wherein 1, 2, 3, 4, 5, or 6 hydrogens are replaced by deuterium.

In some embodiments, the VMAT2 inhibitor is:

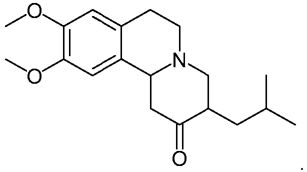

In some embodiments, the VMAT2 inhibitor is: 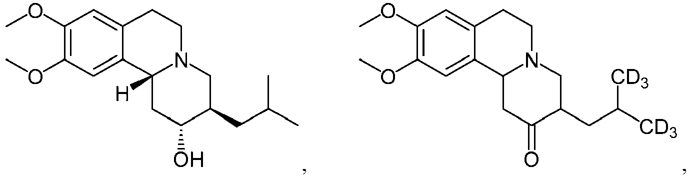 or

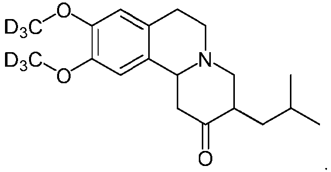

In some embodiments, the VMAT2 inhibitor is valbenazine, or a pharmaceutically acceptable salt thereof. Valbenazine is also referred to as (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester. Valbenazine can be prepared according to U.S. Patent Nos. 8,039,627 and 8,357,697, and U.S. Patent Application No. 15/388,960, the disclosure of each of which is incorporated herein by reference in its entirety. In certain embodiments, the valbenazine for use in the compositions and methods provided herein is in polymorphic Form I as disclosed in U.S. Serial No. 15/338,214, the disclosure of which is incorporated herein by reference in its entirety. Use of valbenazine for treating schizophrenia or schizoaffective disorder is described in WO 2018/102673, which is incorporated herein by reference in its entirety. Use of valbenazine for the treatment of hyperkinetic movement disorders is described in US 2017/071932, which is incorporated herein by reference in its entirety. Methods for formulating valbenazine are found in WO 2019/060322, which is incorporated herein by reference in its entirety. VMAT2 inhibitors for treating mania in bipolar disorder are described in WO 2016/210180, which is incorporated herein by reference in its entirety.

In some embodiments, the VMAT2 inhibitor is [9,10-Dimethoxy-3-(2-methylpropyl)-1H,2H,3H,4H,6H,7H,11bH-pyrido[2,1-a] isoquinolin-2-yl]methanol, or a pharmaceutically acceptable salt thereof, or a related compound, or a pharmaceutically acceptable salt thereof. [9,10-Dimethoxy-3-(2-methylpropyl)-1H,2H,3H,4H,6H,7H,11bH-pyrido[2,1-a] isoquinolin-2-yl]methanol and compounds, compositions and methods relating thereto are described in WO 2016/127133, which is incorporated herein by reference in its entirety.

In some embodiments, the VMAT2 inhibitor may be administered with an antipsychotic drug. Pharmaceutical compositions comprising an antipsychotic drug and a VMAT2 inhibitor and uses thereof are described in US 2016/0339011 (US Patent No. 8,782,398), which is incorporated herein by reference in its entirety.

In some embodiments, the VMAT2 inhibitor is tetrabenazine, or a pharmaceutically acceptable salt thereof. Tetrabenazine is also referred to as 3-isobutyl-9,10-dimethoxy-1,3,4,6,7,11b-hexahydro-2H-pyrido[2,l-a]isoquinolin-2-one. Tetrabenazine may be administered by a variety of methods including the formulations disclosed in PCT Publications WO 2010/018408, WO 2011/019956, and WO 2014/047167, the disclosure of each of which is incorporated herein by reference in its entirety.

In some embodiments, the VMAT2 inhibitor is deutetrabenazine, or a pharmaceutically acceptable salt thereof. Deutetrabenazine is also referred to as 3-isobutyl-9,10-d₆-dimethoxy-1,3,4,6,7,11b-hexahydro-2H-pyrido[2,1-a]isoquinolin-2-one. Deutetrabenazine is disclosed in US Patent No. 8,524,733, which is incorporated herein by reference in its entirety.

In some embodiments, the VMAT2 inhibitor is dihydrotetrabenazine (DHTBZ), or a pharmaceutically acceptable salt thereof. Dihydrotetrabenazine is also referred to as 3-isobutyl-9, 10-dimethoxy-1,3,4,6,7,11b-hexahydro-2H-pyrido[2,1-a]isoquinolin-2-ol. Dihydrotetrabenazine is described in PCT Publications WO2005077946, WO2018178233, and WO2018178243, the disclosure of each of which is incorporated herein by reference in its entirety.

In some embodiments, the VMAT2 inhibitor is lobeline, or derivatives thereof, or a pharmaceutically acceptable salt thereof. Lobeline derivatives include lobelane and GZ-793A.

In some embodiments, the compounds described herein and their salts may be hydrolyzed in the body of a mammal to compounds that may inhibit the human monoamine transporter isoform 2. As such, these compounds and their salts may have additional utility in altering the *in vivo* properties of the metabolite in a mammal such as the maximum concentration or duration of action.

In some embodiments, pharmaceutical compositions containing one or more VMAT2 inhibitors are disclosed. For the purposes of administration, the compounds described herein may be formulated as pharmaceutical compositions. Pharmaceutical compositions comprise a monoamine re-uptake inhibitor described herein and a pharmaceutically acceptable excipient, carrier and/or diluent. The VMAT2 inhibitor is present in the composition in an amount that is effective to treat a particular disorder--that is, in an amount sufficient to reduce the supply of monoamines in the central nervous system, and preferably with acceptable toxicity to the patient. Appropriate concentrations and dosages can be readily determined by one skilled in the art.

The present disclosure further provides for pharmaceutical compositions comprising any one of the compounds described herein and optionally, one or more pharmaceutically acceptable carrier and/or diluent.

Pharmaceutically acceptable carriers and/or diluents are familiar to those skilled in the art. For compositions formulated as liquid solutions, acceptable carriers and/or diluents include saline and sterile water, and may optionally include antioxidants, buffers, bacteriostats and other common additives. The compositions can also be formulated as pills, capsules, granules, or tablets which contain, in addition to a VMAT2 inhibitor, diluents, dispersing and surface active agents, binders, and lubricants. One skilled in this art may further formulate the VMAT2 inhibitor in an appropriate manner, and in accordance with accepted practices, such as those disclosed in Remington's Pharmaceutical Sciences, Gennaro, Ed., Mack Publishing Co., Easton, PA 1990.

The pharmaceutical compositions provided herein may be formulated as immediate or modified release dosage forms, including delayed-, sustained, pulsed-, controlled, targeted-, and programmed-release forms. The pharmaceutical compositions may also be formulated as a suspension, solid, semi-solid, or thixotropic liquid, for administration as an implanted depot.

In some embodiments, a method is provided herein for treating disorders of the central or peripheral nervous system. Such methods include administering a compound described herein to a warm-blooded animal *(e.g.,* a human) in an amount sufficient to treat the condition. In this context, "treat" includes prophylactic administration. Such methods include systemic administration of a VMAT2 inhibitor described herein, preferably in the form of a pharmaceutical composition as discussed above. As used herein, systemic administration includes oral and parenteral methods of administration.

The pharmaceutical compositions described herein that comprise at least one of the VMAT2 inhibitor compounds described herein may be administered to a subject in need by any one of several routes that effectively deliver an effective amount of the compound. The pharmaceutical compositions may be administered alone, or in combination with one or more other compounds provided herein, or one or more other active ingredients. Such administrative routes include, for example, oral, parenteral, enteral, rectal, intranasal, buccal, sublingual, intramuscular, and transdermal. Compositions administered by these routes of administration and others are described in greater detail herein.

The pharmaceutical compositions may also be formulated as a modified release dosage form, including delayed-, extended-, prolonged-, sustained-, pulsatile-, controlled-, accelerated- and fast-, targeted-, programmed-release, and gastric retention dosage forms. These dosage forms can be prepared according to conventional methods and techniques known to those skilled in the art *(see,* Remington: The Science and Practice of Pharmacy, supra; Modified-Release Drug Delivery Technology, Rathbone et al., Eds., Drugs and the Pharmaceutical Science, Marcel Dekker, Inc.: New York, NY, 2002; Vol. 126).

The pharmaceutical composition may further comprise at least one physiologically (or pharmaceutically) acceptable or suitable excipient. Any physiologically or pharmaceutically suitable excipient or carrier *(i.e.,* a non-toxic material that does not interfere with the activity of the active ingredient(s)) known to those of ordinary skill in the art for use in pharmaceutical compositions may be employed in the compositions described herein. Exemplary excipients include diluents and carriers that maintain stability and integrity of the compound.

Pharmaceutically acceptable excipients are well known in the pharmaceutical art and described, for example, in Rowe et al., Handbook of Pharmaceutical Excipients: A Comprehensive Guide to Uses, Properties, and Safety, 5th Ed., 2006, and in Remington: The Science and Practice of Pharmacy (Gennaro, 21st Ed. Mack Pub. Co., Easton, PA (2005)). Exemplary pharmaceutically acceptable excipients include sterile saline and phosphate buffered saline at physiological pH. Preservatives, stabilizers, dyes, buffers, and the like may be provided in the pharmaceutical composition. In addition, antioxidants and suspending agents may also be used.

The pharmaceutical compositions may be in the form of a solution. The solution may comprise saline or sterile water, and may optionally include antioxidants, buffers, bacteriostats, and other common additives. Alternatively, they may be in the form of a solid, such as powder, tablets, pills, or the like. A composition comprising any one of the compounds described herein may be formulated for depot injection, sustained or slow release (also called timed release). Such compositions may generally be prepared using well known technology and administered by, for example, oral, rectal or subcutaneous implantation, intramuscular, or by implantation at the desired target site. Sustained-release formulations may contain the compound dispersed in a carrier matrix and/or contained within a reservoir surrounded by a rate controlling membrane. Excipients for use within such formulations are biocompatible, and may also be biodegradable; preferably the formulation provides a relatively constant level of VMAT2 inhibitor compound release. The amount of compound contained within a sustained release formulation depends upon the site of implantation, the rate and expected duration of release, and the nature of the condition to be treated or prevented.

### Oral Administration

The pharmaceutical compositions provided herein may be provided in solid, semisolid, or liquid dosage forms for oral administration. As used herein, oral administration also include buccal, lingual, and sublingual administration. Suitable oral dosage forms include, but are not limited to, tablets, capsules, pills, troches, lozenges, pastilles, cachets, pellets, medicated chewing gum, granules, bulk powders, effervescent or non-effervescent powders or granules, solutions, emulsions, suspensions, solutions, wafers, sprinkles, elixirs, and syrups. In addition to the active ingredient(s), the pharmaceutical compositions may contain one or more pharmaceutically acceptable carriers or excipients, including, but not limited to, binders, fillers, diluents, disintegrants, wetting agents, lubricants, glidants, coloring agents, dye-migration inhibitors, sweetening agents, and flavoring agents.

Binders or granulators impart cohesiveness to a tablet to ensure the tablet remaining intact after compression. Suitable binders or granulators include, but are not limited to, starches, such as corn starch, potato starch, and pre-gelatinized starch (e.g., STARCH 1500); gelatin; sugars, such as sucrose, glucose, dextrose, molasses, and lactose; natural and synthetic gums, such as acacia, alginic acid, alginates, extract of Irish moss, Panwar gum, ghatti gum, mucilage of isabgol husks, carboxymethylcellulose, methylcellulose, polyvinylpyrrolidone (PVP), Veegum, larch arabogalactan, powdered tragacanth, and guar gum; celluloses, such as ethyl cellulose, cellulose acetate, carboxymethyl cellulose calcium, sodium carboxymethyl cellulose, methyl cellulose, hydroxyethylcellulose (HEC), hydroxypropylcellulose (HPC), hydroxypropyl methyl cellulose (HPMC); microcrystalline celluloses, such as AVICEL-PH-101, AVICEL-PH-103, AVICEL RC-581, AVICEL-PH-105 (FMC Corp., Marcus Hook, PA); and mixtures thereof. Suitable fillers include, but are not limited to, talc, calcium carbonate, microcrystalline cellulose, powdered cellulose, dextrates, kaolin, mannitol, silicic acid, sorbitol, starch, pregelatinized starch, and mixtures thereof. The binder or filler may be present from about 50 to about 99% by weight in the pharmaceutical compositions provided herein.

Suitable diluents include, but are not limited to, dicalcium phosphate, calcium sulfate, lactose, sorbitol, sucrose, inositol, cellulose, kaolin, mannitol, sodium chloride, dry starch, and powdered sugar. Certain diluents, such as mannitol, lactose, sorbitol, sucrose, and inositol, when present in sufficient quantity, can impart properties to some compressed tablets that permit disintegration in the mouth by chewing. Such compressed tablets can be used as chewable tablets.

Suitable disintegrants include, but are not limited to, agar; bentonite; celluloses, such as methylcellulose and carboxymethylcellulose; wood products; natural sponge; cation-exchange resins; alginic acid; gums, such as guar gum and Vee gum HV; citrus pulp; cross-linked celluloses, such as croscarmellose; cross-linked polymers, such as crospovidone; cross-linked starches; calcium carbonate; microcrystalline cellulose, such as sodium starch glycolate; polacrilin potassium; starches, such as com starch, potato starch, tapioca starch, and pre-gelatinized starch; clays; aligns; and mixtures thereof. The amount of disintegrant in the pharmaceutical compositions provided herein varies upon the type of formulation, and is readily discernible to those of ordinary skill in the art. The pharmaceutical compositions provided herein may contain from about 0.5 to about 15% or from about 1 to about 5% by weight of a disintegrant.

Suitable lubricants include, but are not limited to, calcium stearate; magnesium stearate; mineral oil; light mineral oil; glycerin; sorbitol; mannitol; glycols, such as glycerol behenate and polyethylene glycol (PEG); stearic acid; sodium lauryl sulfate; talc; hydrogenated vegetable oil, including peanut oil, cottonseed oil, sunflower oil, sesame oil, olive oil, com oil, and soybean oil; zinc stearate; ethyl oleate; ethyl laureate; agar; starch; lycopodium; silica or silica gels, such as AEROSIL^{®} 200 (W.R. Grace Co., Baltimore, MD) and CAB-0-SIL^{®} (Cabot Co. of Boston, MA); and mixtures thereof. The pharmaceutical compositions provided herein may contain about 0.1 to about 5% by weight of a lubricant.

Suitable glidants include colloidal silicon dioxide, CAB-0-SIL^{®} (Cabot Co. of Boston, MA), and asbestos-free talc. Coloring agents include any of the approved, certified, water soluble FD&C dyes, and water insoluble FD&C dyes suspended on alumina hydrate, and color lakes and mixtures thereof. A color lake is the combination by adsorption of a water-soluble dye to a hydrous oxide of a heavy metal, resulting in an insoluble form of the dye. Flavoring agents include natural flavors extracted from plants, such as fruits, and synthetic blends of compounds which produce a pleasant taste sensation, such as peppermint and methyl salicylate. Sweetening agents include sucrose, lactose, mannitol, syrups, glycerin, and artificial sweeteners, such as saccharin and aspartame. Suitable emulsifying agents include gelatin, acacia, tragacanth, bentonite, and surfactants, such as polyoxyethylene sorbitan monooleate (TWEEN^{®} 20), polyoxyethylene sorbitan monooleate 80 (TWEEN^{®} 80), and triethanolamine oleate. Suspending and dispersing agents include sodium carboxymethylcellulose, pectin, tragacanth, Veegum, acacia, sodium carbomethylcellulose, hydroxypropyl methylcellulose, and polyvinylpyrolidone. Preservatives include glycerin, methyl and propylparaben, benzoic add, sodium benzoate and alcohol. Wetting agents include propylene glycol monostearate, sorbitan monooleate, diethylene glycol monolaurate, and polyoxyethylene lauryl ether. Solvents include glycerin, sorbitol, ethyl alcohol, and syrup. Examples of non-aqueous liquids utilized in emulsions include mineral oil and cottonseed oil. Organic acids include citric and tartaric acid. Sources of carbon dioxide include sodium bicarbonate and sodium carbonate.

It should be understood that many carriers and excipients may serve several functions, even within the same formulation. The pharmaceutical compositions provided herein may be provided as compressed tablets, tablet triturates, chewable lozenges, rapidly dissolving tablets, multiple compressed tablets, or enteric-coating tablets, sugar-coated, or film-coated tablets. Enteric coated tablets are compressed tablets coated with substances that resist the action of stomach acid but dissolve or disintegrate in the intestine, thus protecting the active ingredients from the acidic environment of the stomach. Enteric-coatings include, but are not limited to, fatty acids, fats, phenylsalicylate, waxes, shellac, ammoniated shellac, and cellulose acetate phthalates. Sugar-coated tablets are compressed tablets surrounded by a sugar coating, which may be beneficial in covering up objectionable tastes or odors and in protecting the tablets from oxidation. Film-coated tablets are compressed tablets that are covered with a thin layer or film of a water-soluble material. Film coatings include, but are not limited to, hydroxyethylcellulose, sodium carboxymethylcellulose, polyethylene glycol 4000, and cellulose acetate phthalate. Film coating imparts the same general characteristics as sugar coating. Multiple compressed tablets are compressed tablets made by more than one compression cycle, including layered tablets, and press-coated or dry-coated tablets.

The tablet dosage forms may be prepared from the active ingredient in powdered, crystalline, or granular forms, alone or in combination with one or more carriers or excipients described herein, including binders, disintegrants, controlled-release polymers, lubricants, diluents, and/or colorants. Flavoring and sweetening agents are especially useful in the formation of chewable tablets and lozenges.

The pharmaceutical compositions provided herein may be provided as soft or hard capsules, which can be made from gelatin, methylcellulose, starch, or calcium alginate. The hard gelatin capsule, also known as the dry-filled capsule (DFC), consists of two sections, one slipping over the other, thus completely enclosing the active ingredient. The soft elastic capsule (SEC) is a soft, globular shell, such as a gelatin shell, which is plasticized by the addition of glycerin, sorbitol, or a similar polyol. The soft gelatin shells may contain a preservative to prevent the growth of microorganisms. Suitable preservatives are those as described herein, including methyl- and propyl-parabens, and sorbic acid. The liquid, semisolid, and solid dosage forms provided herein may be encapsulated in a capsule. Suitable liquid and semisolid dosage forms include solutions and suspensions in propylene carbonate, vegetable oils, or triglycerides. Capsules containing such solutions can be prepared as described in U.S. Pat. Nos. 4,328,245; 4,409,239; and 4,410,545. The capsules may also be coated as known by those of skill in the art in order to modify or sustain dissolution of the active ingredient.

The pharmaceutical compositions provided herein may be provided in liquid and semisolid dosage forms, including emulsions, solutions, suspensions, elixirs, and syrups. An emulsion is a two-phase system, in which one liquid is dispersed in the form of small globules throughout another liquid, which can be oil-in-water or water-in-oil. Emulsions may include a pharmaceutically acceptable non-aqueous liquids or solvent, emulsifying agent, and preservative. Suspensions may include a pharmaceutically acceptable suspending agent and preservative. Aqueous alcoholic solutions may include a pharmaceutically acceptable acetal, such as a di(lower alkyl) acetal of a lower alkyl aldehyde (the term "lower" means an alkyl having between 1 and 6 carbon atoms), *e.g.,* acetaldehyde diethyl acetal; and a water-miscible solvent having one or more hydroxyl groups, such as propylene glycol and ethanol. Elixirs are clear, sweetened, and hydroalcoholic solutions. Syrups are concentrated aqueous solutions of a sugar, for example, sucrose, and may also contain a preservative. For a liquid dosage form, for example, a solution in a polyethylene glycol may be diluted with a sufficient quantity of a pharmaceutically acceptable liquid carrier, e.g., water, to be measured conveniently for administration.

Other useful liquid and semisolid dosage forms include, but are not limited to, those containing the active ingredient(s) provided herein, and a dialkylated mono- or polyalkylene glycol, including, 1,2-dimethoxymethane, diglyme, triglyme, tetraglyme, polyethylene glycol-350-dimethyl ether, polyethylene glycol-550-dimethyl ether, polyethylene glycol-750-dimethyl ether, wherein 350, 550, and 750 refer to the approximate average molecular weight of the polyethylene glycol. These formulations may further comprise one or more antioxidants, such as butylated hydroxytoluene (BHT), butylated hydroxyanisole (BHA), propyl gallate, vitamin E, hydroquinone, hydroxycoumarins, ethanolamine, lecithin, cephalin, ascorbic acid, malic acid, sorbitol, phosphoric acid, bisulfite, sodium metabisulfite, thiodipropionic acid and its esters, and dithiocarbamates.

The pharmaceutical compositions provided herein for oral administration may be also provided in the forms of liposomes, micelles, microspheres, or nanosystems. Micellar dosage forms can be prepared as described in U.S. Pat. No. 6,350,458.

The pharmaceutical compositions provided herein may be provided as noneffervescent or effervescent, granules and powders, to be reconstituted into a liquid dosage form. Pharmaceutically acceptable carriers and excipients used in the non-effervescent granules or powders may include diluents, sweeteners, and wetting agents. Pharmaceutically acceptable carriers and excipients used in the effervescent granules or powders may include organic acids and a source of carbon dioxide.

Coloring and flavoring agents can be used in all of the above dosage forms. The pharmaceutical compositions provided herein may be formulated as immediate or modified release dosage forms, including delayed-, sustained, pulsed-, controlled, targeted-, and programmed-release forms.

The pharmaceutical compositions provided herein may be co-formulated with other active ingredients which do not impair the desired therapeutic action, or with substances that supplement the desired action, such as antacids, proton pump inhibitors, and H₂-receptor antagonists.

The pharmaceutical compositions provided herein may be administered parenterally by injection, infusion, or implantation, for local or systemic administration. Parenteral administration, as used herein, include intravenous, intraarterial, intraperitoneal, intrathecal, intraventricular, intraurethral, intrasternal, intracranial, intramuscular, intrasynovial, and subcutaneous administration.

### Parenteral Administration

The pharmaceutical compositions provided herein may be formulated in any dosage forms that are suitable for parenteral administration, including solutions, suspensions, emulsions, micelles, liposomes, microspheres, nanosystems, and solid forms suitable for solutions or suspensions in liquid prior to injection. Such dosage forms can be prepared according to conventional methods known to those skilled in the art of pharmaceutical science *(see,* Remington: *The Science and Practice of Pharmacy,* supra).

The pharmaceutical compositions intended for parenteral administration may include one or more pharmaceutically acceptable carriers and excipients, including, but not limited to, aqueous vehicles, water-miscible vehicles, non-aqueous vehicles, antimicrobial agents or preservatives against the growth of microorganisms, stabilizers, solubility enhancers, isotonic agents, buffering agents, antioxidants, local anesthetics, suspending and dispersing agents, wetting or emulsifying agents, complexing agents, sequestering or chelating agents, cryoprotectants, lyoprotectants, thickening agents, pH adjusting agents, and inert gases.

Suitable aqueous vehicles include, but are not limited to, water, saline, physiological saline or phosphate buffered saline (PBS), sodium chloride injection, Ringers injection, isotonic dextrose injection, sterile water injection, dextrose and lactated Ringers injection. Non-aqueous vehicles include, but are not limited to, fixed oils of vegetable origin, castor oil, com oil, cottonseed oil, olive oil, peanut oil, peppermint oil, safflower oil, sesame oil, soybean oil, hydrogenated vegetable oils, hydrogenated soybean oil, and medium-chain triglycerides of coconut oil, and palm seed oil. Water-miscible vehicles include, but are not limited to, ethanol, 1 ,3-butanediol, liquid polyethylene glycol (e.g., polyethylene glycol 300 and polyethylene glycol 400), propylene glycol, glycerin, N-methyl-2-pyrrolidone, dimethylacetamide, and dimethylsulfoxide.

Suitable antimicrobial agents or preservatives include, but are not limited to, phenols, cresols, mercurials, benzyl alcohol, chlorobutanol, methyl and propyl phydroxybenzates, thimerosal, benzalkonium chloride, benzethonium chloride, methyl- and propyl-parabens, and sorbic acid. Suitable isotonic agents include, but are not limited to, sodium chloride, glycerin, and dextrose. Suitable buffering agents include, but are not limited to, phosphate and citrate. Suitable antioxidants are those as described herein, including bisulfite and sodium metabisulfite. Suitable local anesthetics include, but are not limited to, procaine hydrochloride. Suitable suspending and dispersing agents are those as described herein, including sodium carboxymethylcelluose, hydroxypropyl methylcellulose, and polyvinylpyrrolidone. Suitable emulsifying agents include those described herein, including polyoxyethylene sorbitan monolaurate, polyoxyethylene sorbitan monooleate 80, and triethanolamine oleate. Suitable sequestering or chelating agents include, but are not limited to EDTA. Suitable pH adjusting agents include, but are not limited to, sodium hydroxide, hydrochloric acid, citric acid, and lactic acid. Suitable complexing agents include, but are not limited to, cyclodextrins, including alpha-cyclodextrin, beta-cyclodextrin, hydroxypropyl-beta-cyclodextrin, sulfobutylether-beta-cyclodextrin, and sulfobutylether 7-beta-cyclodextrin (CAPTISOL^{®}, CyDex, Lenexa, KS).

The pharmaceutical compositions provided herein may be formulated for single or multiple dosage administration. The single dosage formulations are packaged in an ampule, a vial, or a syringe. The multiple dosage parenteral formulations may contain an antimicrobial agent at bacteriostatic or fungistatic concentrations.

In some embodiments, the pharmaceutical compositions are provided as ready-to-use sterile solutions. In some embodiments, the pharmaceutical compositions are provided as sterile dry soluble products, including lyophilized powders and hypodermic tablets, to be reconstituted with a vehicle prior to use. In some embodiments, the pharmaceutical compositions are provided as ready-to-use sterile suspensions. In some embodiments, the pharmaceutical compositions are provided as sterile dry insoluble products to be reconstituted with a vehicle prior to use. In some embodiments, the pharmaceutical compositions are provided as ready-to-use sterile emulsions.

The pharmaceutical compositions provided herein may be formulated as immediate or modified release dosage forms, including delayed-, sustained, pulsed-, controlled, targeted-, and programmed-release forms.

The pharmaceutical compositions may be formulated as a suspension, solid, semi-solid, or thixotropic liquid, for administration as an implanted depot. In some embodiments, the pharmaceutical compositions provided herein are dispersed in a solid inner matrix, which is surrounded by an outer polymeric membrane that is insoluble in body fluids but allows the active ingredient in the pharmaceutical compositions diffuse through.

Suitable inner matrixes include polymethylmethacrylate, polybutylmethacrylate, plasticized or unplasticized polyvinylchloride, plasticized nylon, plasticized polyethyleneterephthalate, natural rubber, polyisoprene, polyisobutylene, polybutadiene, polyethylene, ethylene-vinylacetate copolymers, silicone rubbers, polydimethylsiloxanes, silicone carbonate copolymers, hydrophilic polymers, such as hydrogels of esters of acrylic and methacrylic acid, collagen, cross-linked polyvinylalcohol, and cross-linked partially hydrolyzed polyvinyl acetate.

Suitable outer polymeric membranes include polyethylene, polypropylene, ethylene/propylene copolymers, ethylene/ethyl acrylate copolymers, ethylene/vinylacetate copolymers, silicone rubbers, polydimethyl siloxanes, neoprene rubber, chlorinated polyethylene, polyvinylchloride, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer.

### Topical Administration

The pharmaceutical compositions provided herein may be administered topically to the skin, orifices, or mucosa. The topical administration, as used herein, include (intra)dermal, conjuctival, intracorneal, intraocular, ophthalmic, auricular, transdermal, nasal, vaginal, uretheral, respiratory, and rectal administration.

The pharmaceutical compositions provided herein may be formulated in any dosage forms that are suitable for topical administration for local or systemic effect, including emulsions, solutions, suspensions, creams, gels, hydrogels, ointments, dusting powders, dressings, elixirs, lotions, suspensions, tinctures, pastes, foams, films, aerosols, irrigations, sprays, suppositories, bandages, dermal patches. The topical formulation of the pharmaceutical compositions provided herein may also comprise liposomes, micelles, microspheres, nanosystems, and mixtures thereof.

Pharmaceutically acceptable carriers and excipients suitable for use in the topical formulations provided herein include, but are not limited to, aqueous vehicles, water miscible vehicles, non-aqueous vehicles, antimicrobial agents or preservatives against the growth of microorganisms, stabilizers, solubility enhancers, isotonic agents, buffering agents, antioxidants, local anesthetics, suspending and dispersing agents, wetting or emulsifying agents, complexing agents, sequestering or chelating agents, penetration enhancers, cryopretectants, lyoprotectants, thickening agents, and inert gases.

The pharmaceutical compositions may also be administered topically by electroporation, iontophoresis, phonophoresis, sonophoresis and microneedle or needle-free injection, such as POWDERJECT^{™} (Chiron Corp., Emeryville, CA), and BIOJECT^{™} (Bioject Medical Technologies Inc., Tualatin, OR).

The pharmaceutical compositions provided herein may be provided in the forms of ointments, creams, and gels. Suitable ointment vehicles include oleaginous or hydrocarbon bases, including such as lard, benzoinated lard, olive oil, cottonseed oil, and other oils, white petrolatum; emulsifiable or absorption bases, such as hydrophilic petrolatum, hydroxystearin sulfate, and anhydrous lanolin; water-removable bases, such as hydrophilic ointment; water-soluble ointment bases, including polyethylene glycols of varying molecular weight; emulsion bases, either water-in-oil (W/O) emulsions or oil-in-water (O/W) emulsions, including cetyl alcohol, glyceryl monostearate, lanolin, and stearic acid *(see,* Remington: *The Science and Practice of Pharmacy,* supra). These vehicles are emollient but generally require addition of antioxidants and preservatives.

Suitable cream base can be oil-in-water or water-in-oil. Cream vehicles may be water-washable, and contain an oil phase, an emulsifier, and an aqueous phase. The oil phase is also called the "internal" phase, which is generally comprised of petrolatum and a fatty alcohol such as cetyl or stearyl alcohol. The aqueous phase usually, although not necessarily, exceeds the oil phase in volume, and generally contains a humectant. The emulsifier in a cream formulation may be a nonionic, anionic, cationic, or amphoteric surfactant.

Gels are semisolid, suspension-type systems. Single-phase gels contain organic macromolecules distributed substantially uniformly throughout the liquid carrier. Suitable gelling agents include crosslinked acrylic acid polymers, such as carbomers, carboxypolyalkylenes, Carbopol^{®}; hydrophilic polymers, such as polyethylene oxides, polyoxyethylene-polyoxypropylene copolymers, and polyvinylalcohol; cellulosic polymers, such as hydroxypropyl cellulose, hydroxyethyl cellulose, hydroxypropyl methylcellulose, hydroxypropyl methylcellulose phthalate, and methylcellulose; gums, such as tragacanth and xanthan gum; sodium alginate; and gelatin. In order to prepare a uniform gel, dispersing agents such as alcohol or glycerin can be added, or the gelling agent can be dispersed by trituration, mechanical mixing, and/or stirring.

The pharmaceutical compositions provided herein may be administered rectally, urethrally, vaginally, or perivaginally in the forms of suppositories, pessaries, bougies, poultices or cataplasm, pastes, powders, dressings, creams, plasters, contraceptives, ointments, solutions, emulsions, suspensions, tampons, gels, foams, sprays, or enemas. These dosage forms can be manufactured using conventional processes as described in Remington: *The Science and Practice of Pharmacy,* supra.

Rectal, urethral, and vaginal suppositories are solid bodies for insertion into body orifices, which are solid at ordinary temperatures but melt or soften at body temperature to release the active ingredient(s) inside the orifices. Pharmaceutically acceptable carriers utilized in rectal and vaginal suppositories include vehicles, such as stiffening agents, which produce a melting point in the proximity of body temperature, when formulated with the pharmaceutical compositions provided herein; and antioxidants as described herein, including bisulfite and sodium metabisulfite. Suitable vehicles include, but are not limited to, cocoa butter (theobroma oil), glycerin-gelatin, carbowax (polyoxyethylene glycol), spermaceti, paraffin, white and yellow wax, and appropriate mixtures of mono-, di- and triglycerides of fatty acids, hydrogels, such as polyvinyl alcohol, hydroxyethyl methacrylate, polyacrylic acid; glycerinated gelatin. Combinations of the various vehicles may be used. Rectal and vaginal suppositories may be prepared by the compressed method or molding. The typical weight of a rectal and vaginal suppository is about 2 to 3 g.

The pharmaceutical compositions provided herein may be administered ophthalmically in the forms of solutions, suspensions, ointments, emulsions, gel-forming solutions, powders for solutions, gels, ocular inserts, and implants.

The pharmaceutical compositions provided herein may be administered intranasally or by inhalation to the respiratory tract. The pharmaceutical compositions may be provided in the form of an aerosol or solution for delivery using a pressurized container, pump, spray, atomizer, such as an atomizer using electrohydrodynamics to produce a fine mist, or nebulizer, alone or in combination with a suitable propellant, such as 1,1,1,2-tetrafluoroethane or 1,1,1,2,3,3,3-heptafluoropropane. The pharmaceutical compositions may also be provided as a dry powder for insufflation, alone or in combination with an inert carrier such as lactose or phospholipids; and nasal drops. For intranasal use, the powder may comprise a bioadhesive agent, including chitosan or cyclodextrin.

Solutions or suspensions for use in a pressurized container, pump, spray, atomizer, or nebulizer may be formulated to contain ethanol, aqueous ethanol, or a suitable alternative agent for dispersing, solubilizing, or extending release of the active ingredient provided herein, a propellant as solvent; and/or a surfactant, such as sorbitan trioleate, oleic acid, or an oligolactic acid.

The pharmaceutical compositions provided herein may be micronized to a size suitable for delivery by inhalation, such as 50 micrometers or less, or 10 micrometers or less. Particles of such sizes may be prepared using a comminuting method known to those skilled in the art, such as spiral jet milling, fluid bed jet milling, supercritical fluid processing to form nanoparticles, high pressure homogenization, or spray drying.

Capsules, blisters and cartridges for use in an inhaler or insufflator may be formulated to contain a powder mix of the pharmaceutical compositions provided herein; a suitable powder base, such as lactose or starch; and a performance modifier, such as *l*-leucine, mannitol, or magnesium stearate. The lactose may be anhydrous or in the form of the monohydrate. Other suitable excipients include dextran, glucose, maltose, sorbitol, xylitol, fructose, sucrose, and trehalose. The pharmaceutical compositions provided herein for inhaled/intranasal administration may further comprise a suitable flavor, such as menthol and levomenthol, or sweeteners, such as saccharin or saccharin sodium.

The pharmaceutical compositions provided herein for topical administration may be formulated to be immediate release or modified release, including delayed-, sustained-, pulsed-, controlled-, targeted, and programmed release.

### Modified Release

The pharmaceutical compositions provided herein may be formulated as a modified release dosage form. As used herein, the term "modified release" refers to a dosage form in which the rate or place of release of the active ingredient(s) is different from that of an immediate dosage form when administered by the same route. Modified release dosage forms include delayed-, extended-, prolonged-, sustained-, pulsatile- or pulsed-, controlled-, accelerated- and fast-, targeted-, programmed-release, and gastric retention dosage forms. The pharmaceutical compositions in modified release dosage forms can be prepared using a variety of modified release devices and methods known to those skilled in the art, including, but not limited to, matrix controlled release devices, osmotic controlled release devices, multiparticulate controlled release devices, ionexchange resins, enteric coatings, multilayered coatings, microspheres, liposomes, and combinations thereof. The release rate of the active ingredient(s) can also be modified by varying the particle sizes and polymorphorism of the active ingredient(s).

Examples of modified release include, but are not limited to, those described in U.S. Pat. Nos.: 3,845,770; 3,916,899; 3,536,809; 3,598,123; 4,008,719; 5,674,533; 5,059,595; 5,591,767; 5,120,548; 5,073,543; 5,639,476; 5,354,556; 5,639,480; 5,733,566; 5,739,108; 5,891,474; 5,922,356; 5,972,891; 5,980,945; 5,993,855; 6,045,830; 6,087,324; 6,113,943; 6,197,350; 6,248,363; 6,264,970; 6,267,981; 6,376,461; 6,419,961; 6,589,548; 6,613,358; and 6,699,500.

### Matrix Controlled Release Devices

The pharmaceutical compositions provided herein in a modified release dosage form may be fabricated using a matrix controlled release device known to those skilled in the art *(see,* Takada et al. in "Encyclopedia of Controlled Drug Delivery," Vol. 2, Mathiowitz ed., Wiley, 1999).

In some embodiments, the pharmaceutical compositions provided herein in a modified release dosage form is formulated using an erodible matrix device, which is water swellable, erodible, or soluble polymers, including synthetic polymers, and naturally occurring polymers and derivatives, such as polysaccharides and proteins.

Materials useful in forming an erodible matrix include, but are not limited to, chitin, chitosan, dextran, and pullulan; gum agar, gum arabic, gum karaya, locust bean gum, gum tragacanth, carrageenans, gum ghatti, guar gum, xanthan gum, and scleroglucan; starches, such as dextrin and maltodextrin; hydrophilic colloids, such as pectin; phosphatides, such as lecithin; alginates; propylene glycol alginate; gelatin; collagen; and cellulosics, such as ethyl cellulose (EC), methylethyl cellulose (MEC), carboxymethyl cellulose (CMC), CMEC, hydroxyethyl cellulose (HEC), hydroxypropyl cellulose (HPC), cellulose acetate (CA), cellulose propionate (CP), cellulose butyrate (CB), cellulose acetate butyrate (CAB), CAP, CAT, hydroxypropyl methyl cellulose (HPMC), HPMCP, HPMCAS, hydroxypropyl methyl cellulose acetate trimellitate (HPMCAT), and ethylhydroxy ethylcellulose (EHEC); polyvinyl pyrrolidone; polyvinyl alcohol; polyvinyl acetate; glycerol fatty acid esters; polyacrylamide; polyacrylic acid; copolymers of ethacrylic acid or methacrylic acid (EUDRAGIT^{®}, Rohm America, Inc., Piscataway, NJ); poly(2-hydroxyethylmethacrylate); polylactides; copolymers of L-glutamic acid and ethyl-L-glutamate; degradable lactic acidglycolic acid copolymers; poly-D-(-)-3-hydroxybutyric acid; and other acrylic acid derivatives, such as homopolymers and copolymers of butyl methacrylate, methylmethacrylate, ethylmethacrylate, ethylacrylate, (2-dimethylaminoethyl)methacrylate, and (trimethylaminoethyl)methacrylate chloride.

In some embodiments, the pharmaceutical compositions are formulated with a non-erodible matrix device. The active ingredient(s) is dissolved or dispersed in an inert matrix and is released primarily by diffusion through the inert matrix once administered. Materials suitable for use as a non-erodible matrix device included, but are not limited to, insoluble plastics, such as polyethylene, polypropylene, polyisoprene, polyisobutylene, polybutadiene, polymethylmethacrylate, polybutylmethacrylate, chlorinated polyethylene, polyvinylchloride, methyl acrylate-methyl methacrylate copolymers, ethylene-vinylacetate copolymers, ethylene/propylene copolymers, ethylene/ethyl acrylate copolymers, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer, polyvinyl chloride, plasticized nylon, plasticized polyethyleneterephthalate, natural rubber, silicone rubbers, polydimethylsiloxanes, silicone carbonate copolymers, and; hydrophilic polymers, such as ethyl cellulose, cellulose acetate, crospovidone, and cross-linked partially hydrolyzed polyvinyl acetate,; and fatty compounds, such as camauba wax, microcrystalline wax, and triglycerides.

In a matrix controlled release system, the desired release kinetics can be controlled, for example, via the polymer type employed, the polymer viscosity, the particle sizes of the polymer and/or the active ingredient(s), the ratio of the active ingredient(s) versus the polymer, and other excipients in the compositions.

The pharmaceutical compositions provided herein in a modified release dosage form may be prepared by methods known to those skilled in the art, including direct compression, dry or wet granulation followed by compression, melt-granulation followed by compression.

### Osmotic Controlled Release Devices

The pharmaceutical compositions provided herein in a modified release dosage form may be fabricated using an osmotic controlled release device, including one-chamber system, two-chamber system, asymmetric membrane technology (AMT), and extruding core system (ECS). In general, such devices have at least two components: (a) the core which contains the active ingredient(s); and (b) a semipermeable membrane with at least one delivery port, which encapsulates the core. The semipermeable membrane controls the influx of water to the core from an aqueous environment of use so as to cause drug release by extrusion through the delivery port(s).

In addition to the active ingredient(s), the core of the osmotic device optionally includes an osmotic agent, which creates a driving force for transport of water from the environment of use into the core of the device. One class of osmotic agents waterswellable hydrophilic polymers, which are also referred to as "osmopolymers" and "hydrogels," including, but not limited to, hydrophilic vinyl and acrylic polymers, polysaccharides such as calcium alginate, polyethylene oxide (PEO), polyethylene glycol (PEG), polypropylene glycol (PPG), poly(2-hydroxyethyl methacrylate), poly(acrylic) acid, poly(methacrylic) acid, polyvinylpyrrolidone (PVP), crosslinked PVP, polyvinyl alcohol (PVA), PVA/PVP copolymers, PVA/PVP copolymers with hydrophobic monomers such as methyl methacrylate and vinyl acetate, hydrophilic polyurethanes containing large PEO blocks, sodium croscarmellose, carrageenan, hydroxyethyl cellulose (HEC), hydroxypropyl cellulose (HPC), hydroxypropyl methyl cellulose (HPMC), carboxymethyl cellulose (CMC) and carboxyethyl, cellulose (CEC), sodium alginate, polycarbophil, gelatin, xanthan gum, and sodium starch glycolate.

The other class of osmotic agents is osmogens, which are capable of imbibing water to affect an osmotic pressure gradient across the barrier of the surrounding coating. Suitable osmogens include, but are not limited to, inorganic salts, such as magnesium sulfate, magnesium chloride, calcium chloride, sodium chloride, lithium chloride, potassium sulfate, potassium phosphates, sodium carbonate, sodium sulfite, lithium sulfate, potassium chloride, and sodium sulfate; sugars, such as dextrose, fructose, glucose, inositol, lactose, maltose, mannitol, raffinose, sorbitol, sucrose, trehalose, and xylitol,; organic acids, such as ascorbic acid, benzoic acid, fumaric acid, citric acid, maleic acid, sebacic acid, sorbic acid, adipic acid, edetic acid, glutamic acid, p-tolunesulfonic acid, succinic acid, and tartaric acid; urea; and mixtures thereof.

Osmotic agents of different dissolution rates may be employed to influence how rapidly the active ingredient(s) is initially delivered from the dosage form. For example, amorphous sugars, such as Mannogeme EZ (SPI Pharma, Lewes, DE) can be used to provide faster delivery during the first couple of hours to promptly produce the desired therapeutic effect, and gradually and continually release of the remaining amount to maintain the desired level of therapeutic or prophylactic effect over an extended period of time. In this case, the active ingredient(s) is released at such a rate to replace the amount of the active ingredient metabolized and excreted.

The core may also include a wide variety of other excipients and carriers as described herein to enhance the performance of the dosage form or to promote stability or processing.

Materials useful in forming the semipermeable membrane include various grades of acrylics, vinyls, ethers, polyamides, polyesters, and cellulosic derivatives that are water-permeable and water-insoluble at physiologically relevant pHs, or are susceptible to being rendered water-insoluble by chemical alteration, such as crosslinking. Examples of suitable polymers useful in forming the coating, include plasticized, unplasticized, and reinforced cellulose acetate (CA), cellulose diacetate, cellulose triacetate, CA propionate, cellulose nitrate, cellulose acetate butyrate (CAB), CA ethyl carbamate, CAP, CA methyl carbamate, CA succinate, cellulose acetate trimellitate (CAT), CA dimethylaminoacetate, CAethyl carbonate, CA chloroacetate, CA ethyl oxalate, CA methyl sulfonate, CA butyl sulfonate, CA p-toluene sulfonate, agar acetate, amylose triacetate, beta glucan acetate, beta glucan triacetate, acetaldehyde dimethyl acetate, triacetate of locust bean gum, hydroxlated ethylene-vinylacetate, EC, PEG, PPG, PEG/PPG copolymers, PVP, HEC, HPC, CMC, CMEC, HPMC, HPMCP, HPMCAS, HPMCAT, poly(acrylic) acids and esters and poly(methacrylic) acids and esters and copolymers thereof, starch, dextran, dextrin, chitosan, collagen, gelatin, polyalkenes, polyethers, polysulfones, polyethersulfones, polystyrenes, polyvinyl halides, polyvinyl esters and ethers, natural waxes, and synthetic waxes.

Semipermeable membrane may also be a hydrophobic microporous membrane, wherein the pores are substantially filled with a gas and are not wetted by the aqueous medium but are permeable to water, as disclosed in U.S. Pat. No. 5,798,119. Such hydrophobic but water-permeable membrane are typically composed of hydrophobic polymers such as polyalkenes, polyethylene, polypropylene, polytetrafluoroethylene, polyacrylic acid derivatives, polyethers, polysulfones, polyethersulfones, polystyrenes, polyvinyl halides, polyvinylidene fluoride, polyvinyl esters and ethers, natural waxes, and synthetic waxes.

The delivery port(s) on the semipermeable membrane may be formed postcoating by mechanical or laser drilling. Delivery port(s) may also be formed in situ by erosion of a plug of water-soluble material or by rupture of a thinner portion of the membrane over an indentation in the core. In addition, delivery ports may be formed during coating process, as in the case of asymmetric membrane coatings of the type disclosed in U.S. Pat. Nos. 5,612,059 and 5,698,220.

The total amount of the active ingredient(s) released and the release rate can substantially by modulated via the thickness and porosity of the semipermeable membrane, the composition of the core, and the number, size, and position of the delivery ports.

The pharmaceutical compositions in an osmotic controlled-release dosage form may further comprise additional conventional excipients as described herein to promote performance or processing of the formulation.

The osmotic controlled-release dosage forms can be prepared according to conventional methods and techniques known to those skilled in the art *(see,* Remington: *The Science and Practice of Pharmacy,* supra; Santus and Baker, J. Controlled Release 1995, 35, 1-21; Verma et al., Drug Development and Industrial Pharmacy 2000,26, 695-708; Verma et al., J. Controlled Release 2002, 79, 7-27).

In certain embodiments, the pharmaceutical compositions provided herein are formulated as AMT controlled-release dosage form, which comprises an asymmetric osmotic membrane that coats a core comprising the active ingredient(s) and other pharmaceutically acceptable excipients. *See,* U.S. Pat. No. 5,612,059 and WO 2002/17918. The AMT controlled-release dosage forms can be prepared according to conventional methods and techniques known to those skilled in the art, including direct compression, dry granulation, wet granulation, and a dip-coating method.

In some embodiments, the pharmaceutical compositions provided herein are formulated as ESC controlled-release dosage form, which comprises an osmotic membrane that coats a core comprising the active ingredient(s), hydroxylethyl cellulose, and other pharmaceutically acceptable excipients.

### Multiparticulate Controlled Release Devices

The pharmaceutical compositions provided herein in a modified release dosage form may be fabricated a multiparticulate controlled release device, which comprises a multiplicity of particles, granules, or pellets, ranging from about 10 µm to about 3 mm, about 50 µm to about 2.5 mm, or from about 100 µm to 1 mm in diameter. Such multiparticulates may be made by the processes know to those skilled in the art, including wet-and dry-granulation, extrusion/spheronization, roller-compaction, melt-congealing, and by spray-coating seed cores. *See,* for example, Multiparticulate Oral Drug Delivery; Marcel Dekker: 1994; and Pharmaceutical Pelletization Technology; Marcel Dekker: 1989.

Other excipients as described herein may be blended with the pharmaceutical compositions to aid in processing and forming the multiparticulates. The resulting particles may themselves constitute the multiparticulate device or may be coated by various filmforming materials, such as enteric polymers, water-swellable, and water-soluble polymers. The multiparticulates can be further processed as a capsule or a tablet.

### Targeted Delivery

The pharmaceutical compositions provided herein may also be formulated to be targeted to a particular tissue, receptor, or other area of the body of the subject to be treated, including liposome-, resealed erythrocyte-, and antibody-based delivery systems. Examples include, but are not limited to, U.S. Pat. Nos. 6,316,652; 6,274,552; 6,271,359; 6,253,872; 6,139,865; 6,131,570; 6,120,751; 6,071,495; 6,060,082; 6,048,736; 6,039,975; 6,004,534; 5,985,307; 5,972,366; 5,900,252; 5,840,674; 5,759,542; and 5,709,874.

The pharmaceutical compositions provided herein may be formulated for single or multiple dosage administration. The single dosage formulations are packaged in an ampule, a vial, or a syringe.

Also provided are kits that comprise one or more unit doses of the VMAT2 inhibitor. A non-limiting example of such a kit includes a blister pack. In certain embodiments, a kit can include one or more delivery systems, e.g., for delivering or administering a VMAT2 inhibitor as described herein, and directions for use of the kit (e.g., instructions for treating a subject). In some embodiments, the kit can include a VMAT2 inhibitor as described herein and a label indicating that the contents are to be administered to a subject having COMT haploinsufficiency, e.g., 22q11.2 deletion syndrome. The actual dose of the compound of the VMAT2 inhibitor depends on the specific formulation and on the condition to be treated; the selection of the appropriate dose is well within the knowledge of the skilled artisan.

Also provided is a VMAT2 inhibitor that is selected from compounds described herein and pharmaceutically acceptable salts, solvates, and hydrates thereof, for use in a method of treatment of a psychiatric disorder in a subject having COMT haploinsufficiency, the treatment comprising administering a therapeutically effective amount of a VMAT2 inhibitor to the subject.

Also provided is a VMAT2 inhibitor that is selected from compounds described herein and pharmaceutically acceptable salts, solvates, and hydrates thereof, for use in a method of treatment of a psychiatric disorder in a subject having COMT haploinsufficiency, the treatment comprising administering a therapeutically effective amount of a VMAT2 inhibitor to the subject, wherein the VMAT2 inhibitor is not tetrabenazine or reserpine.

Also provided is the use of a VMAT2 inhibitor selected from compounds described herein and pharmaceutically acceptable salts, solvates, and hydrates thereof: in the manufacture of a medicament for the treatment of a psychiatric disorder in a subject having COMT haploinsufficiency, the treatment comprising administering a therapeutically effective amount of a VMAT2 inhibitor to the subject.

Also provided is the use of a VMAT2 inhibitor selected from compounds described herein and pharmaceutically acceptable salts, solvates, and hydrates thereof: in the manufacture of a medicament for the treatment of a psychiatric disorder in a subject having COMT haploinsufficiency, the treatment comprising administering a therapeutically effective amount of a VMAT2 inhibitor to the subject, wherein the VMAT2 inhibitor is not tetrabenazine or reserpine.

Also provided is a VMAT2 inhibitor that is selected from compounds described herein and pharmaceutically acceptable salts, solvates, and hydrates thereof, for use in a method of treatment of a psychiatric disorder in a subject having 22q11.2 deletion syndrome, the treatment comprising administering a therapeutically effective amount of a VMAT2 inhibitor to the subject.

Also provided is a VMAT2 inhibitor that is selected from compounds described herein and pharmaceutically acceptable salts, solvates, and hydrates thereof, for use in a method of treatment of a psychiatric disorder in a subject having 22q11.2 deletion syndrome, the treatment comprising administering a therapeutically effective amount of a VMAT2 inhibitor to the subject, wherein the VMAT2 inhibitor is not tetrabenazine or reserpine.

Also provided is the use of a VMAT2 inhibitor selected from compounds described herein and pharmaceutically acceptable salts, solvates, and hydrates thereof: in the manufacture of a medicament for the treatment of a psychiatric disorder in a subject having 22q11.2 deletion syndrome, the treatment comprising administering a therapeutically effective amount of a VMAT2 inhibitor to the subject.

Also provided is the use of a VMAT2 inhibitor selected from compounds described herein and pharmaceutically acceptable salts, solvates, and hydrates thereof: in the manufacture of a medicament for the treatment of a psychiatric disorder in a subject having 22q11.2 deletion syndrome, the treatment comprising administering a therapeutically effective amount of a VMAT2 inhibitor to the subject, wherein the VMAT2 inhibitor is not tetrabenazine or reserpine.

Also provided is a VMAT2 inhibitor that is selected from compounds described herein and pharmaceutically acceptable salts, solvates, and hydrates thereof, for use in a method of treatment of a psychiatric disorder in a subject, the treatment comprising administering a therapeutically effective amount of a VMAT2 inhibitor to the subject.

Also provided is a VMAT2 inhibitor that is selected from compounds described herein and pharmaceutically acceptable salts, solvates, and hydrates thereof, for use in a method of treatment of a psychiatric disorder in a subject, the treatment comprising administering a therapeutically effective amount of a VMAT2 inhibitor to the subject, wherein the VMAT2 inhibitor is not tetrabenazine or reserpine.

Also provided is the use of a VMAT2 inhibitor selected from compounds described herein and pharmaceutically acceptable salts, solvates, and hydrates thereof: in the manufacture of a medicament for the treatment of a psychiatric disorder in a subject, the treatment comprising administering a therapeutically effective amount of a VMAT2 inhibitor to the subject.

Also provided is the use of a VMAT2 inhibitor selected from compounds described herein and pharmaceutically acceptable salts, solvates, and hydrates thereof: in the manufacture of a medicament for the treatment of a psychiatric disorder in a subject, the treatment comprising administering a therapeutically effective amount of a VMAT2 inhibitor to the subject, wherein the VMAT2 inhibitor is not tetrabenazine or reserpine.

The following examples are provided for purposes of illustration, not limitation.

### EXAMPLE 1

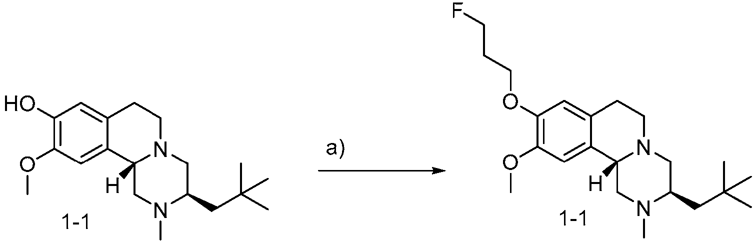

### Synthesis of 1-1:

Product 1-1 (8.0 mg, 0.025 mmol) was dissolved in acetone (0.5 mL) and Cs₂CO₃ (21.0 mg, 0.063 mmol, 2.5 equiv.) was added followed by 1-bromo-3-fluoropropane (5.4 mg, 0.038 mmol, 1.5 equiv). The reaction mixture was heated to 50 °C for one hour. The crude mixture was filtered, diluted to 1 mL with MeOH, and submitted directly for preparative chromatography (3R,11bS)-3-(2,2-dimethylpropyl)-9-(3-fluoropropoxy)-10-methoxy-2-methyl-1H,2H,3H,4H,6H,7H,11bH-piperazino[2,1-a]isoquinoline 1-1.

Table 4 below provides the observed (Obs) ion m/z ratio for 1-1 and other representative compounds that were made according to the procedure as described in this example.

**Table 4**

| 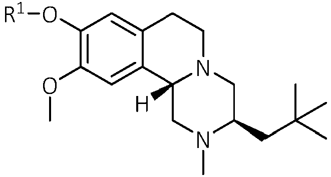 | | | |
|---|---|---|---|
| **Cpd. No.** | **-R¹** | **Compound Name** | **Obs Ion (m/z)** |
| 1-1 | -CH₂CH₂CH₂F | (3R,11bS)-3-(2,2-dimethylpropyl)-9-(3-fluoropropoxy)-10-methoxy-2-methyl-1H,2H,3H,4H,6H,7H,11bH-piperazino[2,1-a]isoquinoline | 379.1 |

| **Cpd. No.** | **-R¹** | **Compound Name** | **Obs Ion (m/z)** |
|---|---|---|---|
| 1-2 | -CH₃ | (3R,11bS)-3-(2,2-dimethylpropyl)-9,10-dimethoxy-2-methyl-1H,2H,3H,4H,6H,7H,11bH-piperazino[2,1-a]isoquinoline | 333.1 |
| 1-3 | -CH₂CF₃ | (3R,11bS)-3-(2,2-dimethylpropyl)-10-methoxy-2-methyl-9-(2,2,2-trifluoroethoxy)-1H,2H,3H,4H,6H,7H,11bH-piperazino[2,1-a]isoquinoline | 401.4 |
| 1-4 | 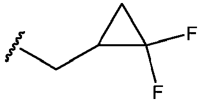 | (3R,11bS)-9-[(2,2-difluorocyclopropyl)methoxy]-3-(2,2-dimethylpropyl)-10-methoxy-2-methyl-1H,2H,3H,4H,6H,7H,11bH-piperazino[2,1-a]isoquinoline | 409.1 |
| 1-5 | -CH₂CH₂CF₃ | (3R,11bS)-3-(2,2-dimethylpropyl)-10-methoxy-2-methyl-9-(3,3,3-trifluoropropoxy)-1H,2H,3H,4H,6H,7H,11bH-piperazino[2,1-a]isoquinoline | 415.1 |
| 1-6 | -CH₂CH₂F | (3R,11bS)-3-(2,2-dimethylpropyl)-9-(2-fluoroethoxy)-10-methoxy-2-methyl-1H,2H,3H,4H,6H,7H,11bH-piperazino[2,1-a]isoquinoline | 365.1 |
| 1-7 | 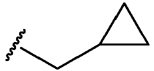 | (3R,11bS)-9-(cyclopropylmethoxy)-3-(2,2-dimethylpropyl)-10-methoxy-2-methyl-1H,2H,3H,4H,6H,7H,11bH-piperazino[2,1-a]isoquinoline | 373.1 |
| 1-8 | 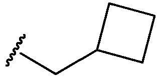 | (3R,11bS)-9-(cyclobutylmethoxy)-3-(2,2-dimethylpropyl)-10-methoxy-2-methyl-1H,2H,3H,4H,6H,7H,11bH-piperazino[2,1-a]isoquinoline | 387.1 |
| 1-9 | -CH₂CH₂CH₂CF₃ | (3R.1 1bS)-3-(2,2-dimethylpropyl)-10-methoxy-2-methyl-9-(4,4,4-trifluorobutoxy)-1H,2H,3H,4H,6H,7H,11bH-piperazino[2,1-a]isoquinoline | 429.1 |
| 1-10 | -CH₂CH₂CH₂CH₂F | (3F,11bS)-3-(2,2-dimethylpropyl)-9-(4-fluorobutoxy)-10-methoxy-2-methyl-1H,2H,3H,4H,6H,7H,11bH-piperazino[2,1-a]isoquinoline | 393.1 |
| 1-11 | -CH₂CH₂OCH₃ | (3R,11bS)-3-(2,2-dimethylpropyl)-10-methoxy-9-(2-methoxyethoxy)-2-methyl-1H,2H,3H,4H,6H,7H,11bH-piperazino[2,1-a]isoquinoline | 377.1 |
| 1-12 | -CH₂CH₃ | (3R,11bS)-3-(2,2-dimethylpropyl)-9-ethoxy-10-methoxy-2-methyl-1H,2H,3H,4H,6H,7H,11bH-piperazino[2,1-a]isoquinoline | 347.1 |
| 1-13 | -CH(CH₃)₂ | (3R,11bS)-3-(2,2-dimethylpropyl)-10-methoxy-2-methyl-9-(propan-2-yloxy)-1H,2H,3H,4H,6H,7H,11bH-piperazino[2,1-a]isoquinoline | 361.1 |
| 1-14 | -CD₂CD₃ | (3R,11bS)-3-(2,2-dimethylpropyl)-9-(ethoxy-d₅)-10-methoxy-2-methyl-1H,2H,3H,4H,6H,7H,11bH-piperazino[2,1-a]isoquinoline | 352.1 |
| 1-15 | -CD₃ | (3R,11bS)-3-(2,2-dimethylpropyl)-9,10-bis(methoxy-d₃)-2-methyl-1H,2H,3H,4H,6H,7H,11bH-piperazino[2,1-a]isoquinoline | |

Additionally, Compounds **1-2** and **1-3** were prepared according to the general schemes below.

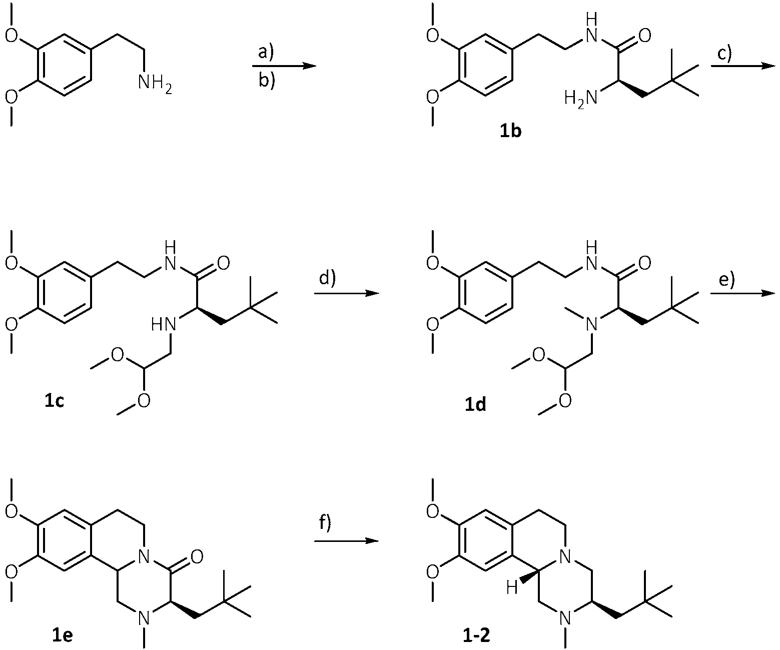

### Synthesis of 1b:

2-(3,4-Dimethoxyphenyl)ethan-1-amine ([40 g, 221 mmol]) was dissolved in DCM ([400 mL]). The solution was cooled and (*R*)-2-((tert-butoxycarbonyl)amino)-4,4-dimethylpentanoic acid ([59.6 g, 243 mmol, 1.1 eq.]) was added. DMAP (7.3 g, 60 mmol, 0.27 eq.) was added, followed by EDAC (74.1 g, 386 mmol, 1.75 eq.) in portions, producing a heterogeneous mixture. The mixture was warmed to room temperature and stirred until completion. Upon completion the reaction was quenched with citric acid. The phases were separated and the aqueous phase was back-extracted with DCM. The pooled organics were washed with brine and distilled to provide tert-butyl (R)-(1-((3,4-dimethoxyphenethyl)amino)-4,4-dimethyl-1-oxopentan-2-yl)carbamate 1a. 1a (90 g, 221 mmol) was dissolved in DCM (315 mL). The solution was cooled prior to the addition of trifluoroacetic acid (135 mL). The reaction was warmed to room temperature and stirred until completion. Upon completion, the reaction was cooled, diluted with water and DCM before being quenched with sodium hydroxide. The phases were separated and the aqueous phase was back-extracted with DCM. The pooled organics were washed with brine and distilled into THF to provide (*R*)-2-amino-*N*-(3,4-dimethoxyphenethyl)-4,4-dimethylpentanamide **1b.**

### Synthesis of 1c:

**1b** (66 g, 214 mmol) was dissolved in THF (528 mL). Acetic acid (64.3 g, 1070 mmol, 5 eq.) and 2,2-Dimethoxyacetaldehyde, 60% in water, (39 g, 225 mmol, 1.05 eq.) were added and stirred at room temperature for 1 hour. The reaction was cooled prior to the portionwise addition of sodium cyanoborohydride (26.9 g, 428 mmol, 2 eq.). Upon completion, the reaction was diluted with water and quenched with sodium hydroxide. The THF was stripped and replaced with EtOAc. The phases were separated and the aqueous phase was back-extracted with EtOAc. The pooled organics are washed with brine and distilled into MeOH to provide (*R*)-2-((2,2-dimethoxyethyl)amino)-N-(3,4-dimethoxyphenethyl)-4,4-dimethylpentanamide 1c.

### Synthesis of 1d:

**1c** (84.9 g, 214 mmol) was dissolved in MeOH (1.25L). Paraformaldehyde (38.6 g, 1285 mmol, 6 eq.) and acetic acid (84.9 g, 1413 mmol, 6.6 eq.) were added. Sodium cyanoborohydride (33.6 g, 535 mmol, 2.5 eq.) was added in portions at room temperature. The reaction was slowly heated to control an exotherm. Upon completion, the reaction was diluted with water and quenched with sodium hydroxide. The MeOH was stripped and replaced with EtOAc. The phases were separated and the aqueous phase was back-extracted with EtOAc. The pooled organics were washed with brine and solvent exchanged into DCM to provide (R)-2-((2,2-dimethoxyethyl)(methyl)amino)-N-(3,4-dimethoxyphenethyl)-4,4-dimethylpentanamide 1**d.**

### Synthesis of 1e diastereomer mix:

Concentrated sulfuric acid (11.9 g, 122 mmol, 5 eq.) was dissolved/suspended in DCM (50 mL) and cooled. **1d** (10 g, 24.4 mmol) was dissolved in DCM (25 mL) and added rapidly to the acid solution with vigorous stirring. Upon completion, the reaction was diluted with water and quenched with ammonium hydroxide. The phases were separated and the aqueous phase was back-extracted with DCM. The pooled organics are washed with brine and solvent exchanged into THF to provide (3*R*)-9,10-dimethoxy-2-methyl-3-neopentyl-1,2,3,6,7,11b-hexahydro-4*H*-pyrazino[2,1-*a*]isoquinolin-4-one **1e.**

### Synthesis of 1-2 diastereomer mix:

**1e** (8.45 g, 24.4 mmol) was dissolved in anhydrous THF (101 mL) and cooled. Lithium aluminum hydride, 2M, (36.6 mL, 73.2 mmol, 3 eq.) was added and the reaction was heated. Upon completion, the LAH was quenched in the manner of Feiser. The precipitated aluminum salts were filtered and washed. The filtrate was stripped of THF and replaced with methyl-t-butyl ether. The phases were separated and the aqueous phase was back-extracted with MTBE. The pooled organics were washed with brine and solvent exchanged into MeOH to provide (3R)-3-(2,2-dimethylpropyl)-9,10-dimethoxy-2-methyl-1H,2H,3H,4H,6H,7H,11bH-piperazino[2,1-a]isoquinoline **1-2**

### diastereomer mix.

### Synthesis of 1-2 diphosphate:

**1-2 diastereomer mix** (20 g, 60.2 mmol) was dissolved in MeOH (160 mL). The solution was filtered and heated to 50 °C. Phosphoric acid (7.25 g, 1.05 eq.) was added. Solution was heated to 60 °C and a second equivalent of phosphoric acid was added over 1 hour (7.25 g, 1.05 eq.) and stirred at 60 °C for 10 minutes before cooling to 20 °C over 4 hours. The suspension was filtered and the solids were washed with MeOH. Solids were dried in a vacuum oven for 3 days at 50 °C to provide **1-2,** (3R,11bS)-9,10-dimethoxy-2-methyl-3-neopentyl-1,3,4,6,7,11b-hexahydro-2H-pyrazino[2,1-a]isoquinoline, also known as (3R,11bS)-3-(2,2-dimethylpropyl)-9,10-dimethoxy-2-methyl-1H,2H,3H,4H,6H,7H,11bH-piperazino[2,1-a]isoquinoline, as the diphosphate salt.

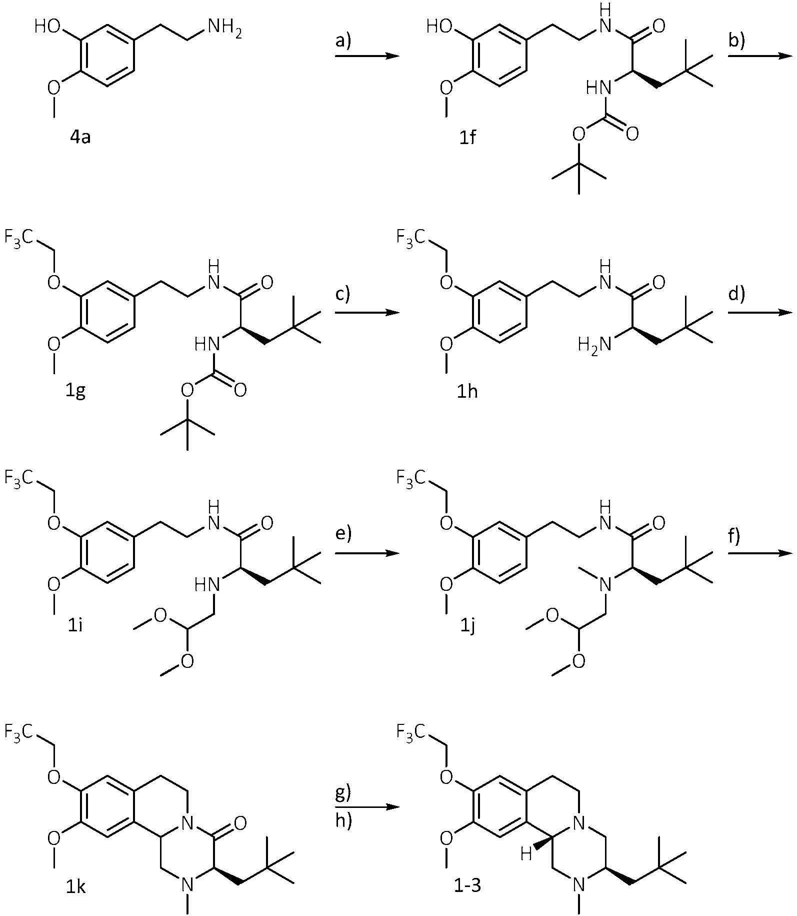

### Synthesis of 1f:

(*R*)-2-((tert-Butoxycarbonyl)amino)-4,4-dimethylpentanoic acid (62 g, 252 mmol, 1.05 eq.) and 5-(2-aminoethyl)-2-methoxyphenol hydrochloride (40.2 g, 240 mmol) were dissolved in DMF (400 mL) and the mixture was cooled to 0 °C. HATU (96 g, 252 mmol, 1.05 eq.) was added followed by addition of DIEA (93.2 g, 721 mmol, 3.0 eq.) over 10 minutes. The mixture was stirred at 0 °C for 60 minutes and then added to water (1000 mL) and EtOAc (1000 mL). The mixture was transferred to a separation funnel, separated and the aqueous extracted with EtOAc. The pooled organics were washed with brine and water and then dried over MgSO₄ and concentrated *in vacuo* to provide tert-butyl (*R*)-(1-((3-hydroxy-4-methoxyphenethyl)amino)-4,4-dimethyl-1-oxopentan-2-yl)carbamate **1f** as a viscous oil.

### Synthesis of 1g:

Compound **1f** (90 g, 228 mmol) and K₂CO₃ (94.5 g, 684 mmol, 3.0 eq.) were dissolved in acetone (630 mL) and 2,2,2-trifluoroethyl triflate (79.4 g, 342 mmol, 1.5 eq.) was slowly added. The reaction was warmed to 50 °C and stirred for 5 hours. After cooling to room temperature, the mixture was diluted with water (450 mL). The mixture was concentrated to remove the acetone and EtOAc (450 mL) was added. The phases were separated and the aqueous layer was extracted with EtOAc. The pooled organics were washed with brine and water and then dried over MgSO₄ and concentrated *in vacuo* to provide tert-butyl (*R*)-(1-((4-methoxy-3-(2,2,2-trifluoroethoxy)phenethyl)amino)-4,4-dimethyl-1-oxopentan-2-yl)carbamate **1g** as a viscous oil.

### Synthesis of 1h:

Compound **1g** (96.5 g, 203 mmol) was dissolved in DCM (290 mL) and cooled to 0 °C followed by addition of TFA (193 mL). The mixture was stirred for 10 minutes and then warmed to room temperature and stirred for 6 hours. The reaction was then cooled to 0 °C and diluted with water and DCM. A 50% w/w NaOH solution was added under vigorous stirring until a pH of 13 was reached. The layers were separated and the organic was washed with brine and concentrated in *vacuo* to provide (*R*)-2-amino-*N*-(4-methoxy-3-(2,2,2-trifluoroethoxy)phenethyl)-4,4-dimethylpentanamide **1h** as a viscous oil.

### Synthesis of 1i:

Compound **1h** (5.0 g, 13.3 mmol) was dissolved in MTBE (35 mL) and the reaction mixture was purged with nitrogen and cooled to 0 °C. Acetic acid (4.0g, 66.4 mmol, 5.0 eq.) was added at and maintained at 0 °C. 2,2-Dimethoxyacetaldehyde, 60% in water, (4.61g, 26.6 mmol, 2.0 eq.) was added and the reaction mixture was purged with nitrogen. The reaction was stirred for 90 minutes at 0 °C. NaBH₄ (1.0g, 26.6 mmol, 2.0 eq.) was added portion-wise over 10 minutes and then the reaction mixture stirred at 0 °C for 60 minutes. Water (25 mL) was added. A 4M solution of K₂CO₃ was added until a pH of 11 was reached. The layers were separated. The organic was washed with brine and concentrated *in vacuo* to provide (*R*)-2-((2,2-dimethoxyethyl)amino)-N-(4-methoxy-3-(2,2,2-trifluoroethoxy)phenethyl)-4,4-dimethylpentanamide **1i**.

### Synthesis of 1j:

Compound **1i** (6.2 g, 13.3 mmol) was dissolved in MTBE (18mL) and and EtOH (9 mL), purged with nitrogen, and cooled to 0 °C. Acetic acid (4.0g, 66.4 mmol, 5.0 eq.) and formalin (5.4g, 66.4 mmol, 5.0 eq.) were added and stirred for 90 minutes at 0 °C. Na(AcO)₃BH (8.5g, 40.0 mmol, 3.0 eq.) was added portion-wise over 5 minutes and stirred at 0 °C for 30 minutes. At 0 °C, water (25 mL) was added, followed by a 4M solution of K₂CO₃ until a pH of 11. The layers were separated. The organic was washed with brine and concentrated *in vacuo* to provide (*R*)-2-((2,2-dimethoxyethyl)(methyl)amino)-N-(4-methoxy-3-(2,2,2-trifluoroethoxy)phenethyl)-4,4-dimethylpentanamide **1j.**

### Synthesis of 1k:

Compound **1j** (22.9 g, 49.3 mmol) was dissolved in DCM (57 mL) and added over2 minutes to -30 °C solution of H₂SO₄ (24.2 g, 247 mmol, 5.0 eq.) in DCM (115 mL). The reaction mixture warmed to 0 °C during the addition and then stirred for 30 minutes. At 0 °C, water (69 mL) was added then NH₄OH was added under vigorous stirring until a pH of 10 was reached. The phases were separated and the organic layer was washed with brine and then dried over MgSO₄ and concentrated *in vacuo* to provide (3*R*)-10-methoxy-2-methyl-3-neopentyl-9-(2,2,2-trifluoroethoxy)-1,2,3,6,7,11b-hexahydro-4*H*-pyrazino[2,1-a]isoquinolin-4-one **1k.**

### Synthesis of 1-3 diastereomer mix:

Compound **1k** (12.5 g, 30.2 mmol) was dissolved in 2-methyltetrahydrofuran (87.5 mL) and 9-BBN (14.7 g, 2 eq. of the dimer solid) was added portion-wise. The reaction mixture was heated to 50 °C for 2 hours. The reaction mixture was then cooled to room temperature and heptane (87.5 mL) and HCl (1N, 62.5 mL) were added to reach a pH of about 2. The reaction mixture was stirred for 60 minutes and the phases were separated. The organic phase was washed with 37.5 mL of 1N HCl. The combined aqueous phases were charged with MTBE (62.5 mL) and 2M K₂CO₃ (62.5 mL) to reach a pH of 9-10. The mixture was stirred for 30 minutes and the phases were separated. The aqueous layer was washed with MTBE (62.5 mL). The pooled organics were washed with brine and concentrated *in vacuo* to provide **1-3** and its diastereomer as a mixture.

### Synthesis of 1-3 L-DBTA salt:

The diastereomer mixture containing **1-3** (5.12g, 12.8 mmol) was dissolved in acetonitrile (30 mL) and dibenzoyl-L-tartaric acid (L-DBTA, 4.59 g, 1.0 eq.) was added. The reaction mixture was heated to 50 °C. Water (12.5 mL) was added maintaining the temperature at 40-50 °C. The mixture was cooled to room temperature over 3 hours. The mixture was filtered and then washed with 6:2.5 acetonitrile: water to provide an L-DBTA salt of (3*R*,11b*S*)-10-methoxy-2-methyl-3-neopentyl-9-(2,2,2-trifluoroethoxy)-1,3,4,6,7,11b-hexahydro-2*H*-pyrazino[2,1-*a*]isoquinoline **1-3 L-DBTA salt.**

### Synthesis of 1-3 and 1-3 di-HCl salt:

L-DBTA salt of (3*R*,11b*S*)-10-methoxy-2-methyl-3-neopentyl-9-(2,2,2-trifluoroethoxy)-1,3,4,6,7,11b-hexahydro-2*H*-pyrazino[2,1-*a*]isoquinoline **1-3 L-DBTA salt** (30.0 g, 39.5 mmol) was suspended in water (300 mL) and isopropyl acetate (300 mL). NaOH was added (50% aqueous solution) until the pH was 13. Phases were separated and the organic washed with brine (90 mL). The organic layer was concentrated resulting in 15.9 g of **1-3** as the free base which was dissolved in ethyl acetate (80 mL). The solution was filtered and water was added (6.4 mL). The solution was heated to 35 °C and 37% HCl (3.4 mL, 1.05 eq.), was added over 5 minutes. A second charge of HCl (3.4 mL, 1.05 eq.) was added over 5 min. The mixture was cooled to 30 °C and seeded. The slurry was stirred for 90 minutes before being slowly cooled to room temperature. Solids were filtered and washed with ethyl acetate (16 mL). Wet cake was dried in vacuum oven at 50 °C. to provide (3*R*,11b*S*)-10-methoxy-2-methyl-3-neopentyl-9-(2,2,2-trifluoroethoxy)-1,3,4,6,7,11b-hexahydro-2*H*-pyrazino[2,1-*a*]isoquinoline as the dihydrochloride salt **1-3 di-HCl salt.**

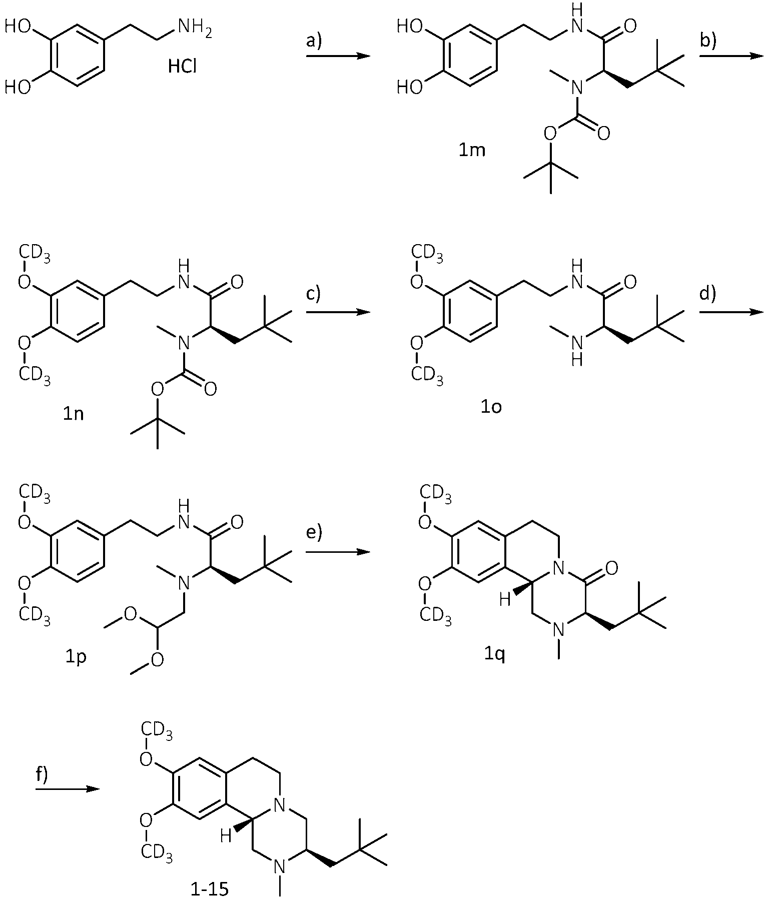

### Synthesis of 1m:

Dopamine hydrochloride (15 g, 79.1 mmol) and 2-[[(1,1-dimethylethoxy)carbonyl]methylamino]-4,4-dimethyl-(2R)-pentanoic acid (21.5 g, 83.1 mmol, 1.05 eq.) were dissolved in DMF (150 mL) and cooled to 0 °C. HATU (31.6 g, 83.1 mmol, 1.05 eq.) was added followed by addition of DIEA (30.7 g, 237 mmol, 3.0 eq.). The mixture was stirred at 0 °C for 2 hours and then added to water (300 mL) and EtOAc (300 mL). The mixture was transferred to a separation funnel, separated and the aqueous extracted with EtOAc. The pooled organics were washed with brine and water and concentrated *in vacuo* to provide tert-butyl (R)-(1-((3,4-dihydroxyphenethyl)amino)-4,4-dimethyl-1-oxopentan-2-yl)(methyl)carbamate 1m as a viscous oil.

### Synthesis of 1n:

Compound **1m** (31.2 g, 79.1 mmol) was dissolved in acetone (218 mL). K₂CO₃ (32.8 g, 237 mmol, 3.0 eq.) was added followed by iodomethane- d₃ (25.2 g, 174 mmol, 2.2 eq.). The reaction was warmed to 50 °C and stirred for 26 hours. After cooling to room temperature, the mixture was diluted with water (220 mL). The mixture was concentrated to remove acetone. EtOAc (156 mL) was added. The phases were separated and the aqueous layer was extracted with EtOAc. The pooled organics were washed with brine, dried over MgSO₄ and concentrated *in vacuo* to provide tert-butyl (R)-(1-((3,4-bis(methoxy-d₃)phenethyl)amino)-4,4-dimethyl-1-oxopentan-2-yl)(methyl)carbamate 1n as a viscous oil.

### Synthesis of 1o:

Compound **1n** (17.0 g, 39.7 mmol) was dissolved in DCM (60 mL) and cooled to 0 °C followed by addition of TFA (34 mL). The mixture was stirred for 10 minutes and then warmed to room temperature and stirred for 21 hours. The reaction was then cooled to 0 °C and diluted with water and DCM. A 50% w/w NaOH solution was added under vigorous stirring until a pH of 13 was reached. The layers were separated and the aqueous layer was extracted with DCM. The pooled organics were washed with brine and concentrated *in vacuo* to provide (R)-N-(3,4-bis(methoxy-d₃)phenethyl)-4,4-dimethyl-2-(methylamino)pentanamide 1o as a viscous oil.

### Synthesis of 1p:

**1o** (13.0 g, 39.6 mmol) was dissolved in THF (104 mL). Acetic acid (11.4 mL, 200 mmol, 5 eq.) and 2,2-Dimethoxyacetaldehyde, 60% in water, (10.3 g, 59.4 mmol, 1.5 eq.) were added and stirred at room temperature for 2 hours. The reaction was cooled prior to the portionwise addition of sodium cyanoborohydride (5.2 g, 83.4 mmol, 2 eq.). Two more concurrent additions of 2,2-Dimethoxyacetaldehyde and sodium cyanoborohydride (0.4 eq. and 0.3 eq.) was required for completion of the reaction. Upon completion, the reaction was cooled to 0 °C, diluted with water and quenched with 10% sodium hydroxide till a pH > 10. The THF was stripped and replaced with EtOAc. The phases were separated and the aqueous phase was back-extracted with EtOAc. The pooled organics were washed with brine and concentrated *in vacuo* to provide (R)-N-(3,4-bis(methoxy-d₃)phenethyl)-2-((2,2-dimethoxyethyl)(methyl)amino)-4,4-dimethylpentanamide 1p.

### Synthesis of 1q:

Concentrated sulfuric acid (10.6 mL, 199 mmol, 5 eq.) was dissolved/suspended in DCM (83 mL) and cooled to -20 °C. **1p** (16.5 g, 39.6 mmol) was dissolved in DCM (41 mL) and added rapidly to the acid solution with vigorous stirring keeping the reaction less than 0 °C. Upon completion, the reaction was diluted with water (50 mL) and quenched with ammonium hydroxide to pH 9. The phases were separated and the aqueous phase was back-extracted with DCM. The pooled organics are washed with brine and concentrated *in vacuo* to provide a mix of diastereomers. Separation of the diastereomers was accomplished with normal phase silica column chromatography with hexane and ethyl acetate elution to provide (3R,11bS)-9,10-bis(methoxy-d₃)-2-methyl-3-neopentyl-1,2,3,6,7,11b-hexahydro-4H-pyrazino[2,1-a]isoquinolin-4-one **1q.**

### Synthesis of 1-15:

**1q** (6.9 g, 19.6 mmol) was dissolved in anhydrous THF (103 mL) and cooled to 0 °C. Lithium aluminum hydride, 2.4M, (32.5 mL, 78 mmol, 4 eq.) was slowly added and the reaction was heated to 40 °C over 2 hours. Upon completion, the LAH was quenched in the manner of Feiser. The precipitated aluminum salts were filtered and washed with THF. The filtrate was stripped of THF and replaced with methyl-t-butyl ether. The phases were separated and the aqueous phase was back-extracted with MTBE. The pooled organics were washed with brine and concentrated *in vacuo* to provide (3R,11bS)-3-(2,2-dimethylpropyl)-9,10-bis(methoxy-d₃)-2-methyl-1H,2H,3H,4H,6H,7H,11bH-piperazino[2,1-a]isoquinoline also known as (3R,11bS)-9,10-bis(methoxy-d₃)-2-methyl-3-neopentyl-1,3,4,6,7,11b-hexahydro-2H-pyrazino[2,1-a]isoquinoline **1-15.**

### EXAMPLE 2:

### METHODS FOR DETERMINING VMAT2 INHIBITORY ACTIVITY OF A COMPOUND

Examples of techniques for determining the capability of a compound to inhibit VMAT2 are provided below. The procedure is adapted from that described previously *(see, e.g.,* Near, (1986), Mol. Pharmacol. 30: 252-57; Teng, et al., J. Neurochem. 71, 258-65, 1998). Homogenates from human platelets or Sprague-Dawley rat forebrain are prepared by homogenization and then washed by centrifugation as described previously *(see, e.g.,* Hoare et al., (2003) Peptides 24:1881-97). In a total volume of 0.2 mL in low-binding 96-well plates (Corning #3605), twelve concentrations of test compound are competed against 6 nM ³H-dihydrotetrabenazine (American Radiolabeled Chemicals, Kd 2.6 nM) on rat forebrain homogenate (100 µg membrane protein per well) or human platelet homogenate (50 µg membrane protein per well) in VMAT2 binding buffer (Dulbecco's phosphate buffered saline, 1 mM EDTA, pH 7.4). Following incubation at 25 °C for two hours, bound radioligand was collected by rapid filtration onto GF/B glass fiber filters using a Unifilter-96 Harvester (PerkinElmer). Filter plates are pre-treated for 10 minutes with 0.1% polyethylenimine, and following harvesting the filter plates are washed with 800 µl VMAT2 binding buffer. Bound radioligand is quantified by scintillation counting using a Topcount NXT (PerkinElmer).

Human *K*ᵢs can be determined using a slightly modified procedure described below. In a total volume of 0.15 mL in low-binding 96-well plates (Corning #3605), twelve concentrations of test compound are competed against 10 nM ³H-dihydrotetrabenazine (American Radiolabeled Chemicals, Kd 2.6 nM) on rat forebrain homogenate (100 µg membrane protein per well) or human platelet homogenate (15 µg membrane protein per well) in VMAT2 binding buffer (Dulbecco's phosphate buffered saline, 1 mM EDTA, pH 7.4). Following incubation at 25°C for 90 minutes, bound radioligand is collected by rapid filtration onto GF/B glass fiber filters using a Unifilter-96 Harvester (PerkinElmer). Filter plates are pre-treated with 0.1% polyethylenimine and allowed to dry overnight, and following harvesting the filter plates are washed with 800 µl VMAT2 binding buffer. Bound radioligand is quantified by scintillation counting using a Topcount NXT (PerkinElmer).

### EXAMPLE 3:

### PREPARATION OF CAPSULES CONTAINING 80 MG VALBENAZINE

### I. Method 1

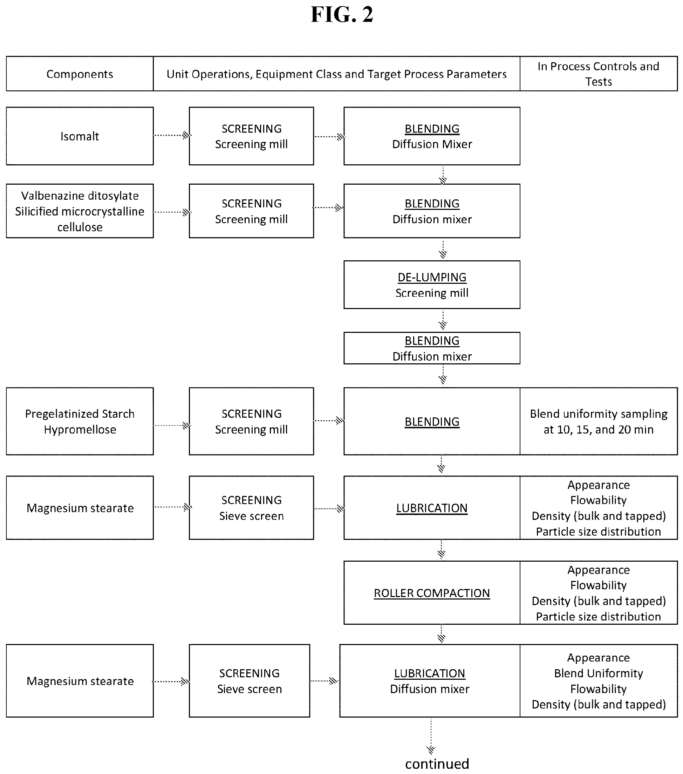

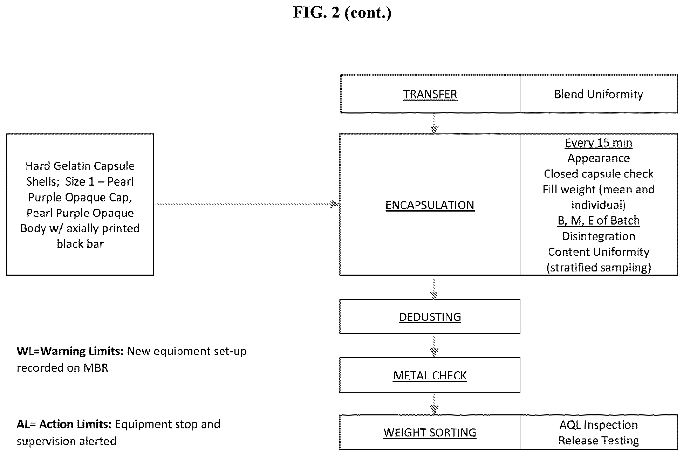

Capsules containing 80 mg valbenazine (measured as the free base) may be prepared according to the procedure set forth below, and the makeup of exemplary tablets are listed in Table 5. A flow diagram of the manufacturing process for Valbenazine Capsules, 80 mg, which comprises unit operations of low shear (tumble) blending, screening, roller compaction and encapsulation, is presented in Figure 2.

**Table 5**

| **Component** | **Quantity 80mg capsule** | | **Function** |
|---|---|---|---|
| | **(mg/ capsule)** | **% (w/w)** | |
| Valbenazine ditosylate | 145.80 | 40.0 | Active |
| Silicified Microcrystalline Cellulose | 91.25 | 25.0 | Diluent |
| Isomalt | 73.00 | 20.0 | Diluent |
| Partially pregelatinized maize starch | 27.38 | 7.5 | Disintegrant |
| Hydroxypropyl Methylcellulose | 18.25 | 5.0 | Binder |
| Magnesium stearate | 9.12 | 2.5 | Lubricant |
| **Total Capsule Fill Weight** | 364.80 | 100.00 | - |
| Hard gelatin capsule - Size #1 | 1 | - | Shell |

Valbenazine ditosylate, silicified microcrystalline cellulose (USP), isomalt (USNF), partially pregelatinized maize starch (USNF), hydroxypropyl methylcellulose (USNF) and magnesium stearate (USNF) are weighed according to the amounts in Table 5.

### Wallpapering:

Isomalt is transferred through a screening mill equipped with an 813 µm or equivalent round-hole screen to a tote bin for blending. The screened isomalt component is then blended.

### Pre-Blending and Screening:

The following components are transferred into the tote bin through a screening mill equipped with a ~813 µm or equivalent round-hole screen:
a. Valbenazine ditosylate
b. Silicified microcrystalline cellulose ("SMCC")

The components are then blended.

### Delumping:

The blend is vacuum transferred through a buffer tank equipped with a ~813µm or equivalent round-hole screen.

### Pre-Blending #2:

The screened components are again blended.

### Intragranular Blending:

The following components are then transferred, into the tote bin through a screening mill equipped with a ~813µm or equivalent round-hole screen:
a. Partially pregelatinized maize starch
b. Hydroxypropyl methylcellulose

The components are then blended. Inadequate de-agglomeration and subsequent dispersion of valbenazine ditosylate in isomalt and SMCC diluent can potentially impact content and uniformity of dosage units.

### Lubricant Blending:

Magnesium stearate is manually screened (~1 mm sieve) (intragranular quantity adjusted as needed based on pre-lubricated blend yield- limit 98%) into the opened tote bin for blending. The components are then blended. The desired output for this step is improved flowability with increased bulk and tapped density and improved particle size distribution.

### Roller Compaction:

The blend is then gravity fed through a roller compactor with a mill screen of 0.8-1.0 mm. The blend characterisics are important factors to consider for how well the blend will handle during encapsulation. Improper processing parameters can result in poor granule flow and compressibility which impacts encapsulation. The high solubility of the API and excipients should not impact dissolution. All roller compaction blends show improvement over the initial intragranular blend properties which supports better capsule weight uniformity.

### Final Lubricant Blending:

Magnesium stearate is manually screened (~1 mm sieve) (quantity to be adjusted as needed based on pre-lubricated blend yield- limit 98%) into the opened tote bin for blending. The components are then blended. The desired output for this step is a uniform and free flowing lubricated final blend for encapsulation. Inadequate blending can impact content and uniformity of dosage units. Over blending with hydrophobic magnesium stearate can impact dissolution. Blending is performed in a controlled environment minimizing moisture exposure.

### Encapsulation:

The lubricated blend is transferred to an automatic encapsulation machine and encapsulated into a size 1 capsule. Improper encapsulation equipment setup can impact filled capsule shell appearance. Capsule fill weight can impact content and dose uniformity. Capsule fill plug compression could impact dissolution and fill weight/content uniformity.

Encapsulation is performed in a controlled environment minimizing moisture exposure.

Dedusting and metal detection of the encapsulated product is performed, and the product is weight-checked.

### II. Method 2

An 80 mg dose formulation strategy can proceed according to the known 40 mg capsule direct encapsulation formulation. A Size 0 capsule can be filled with twice the amount of the 40 mg powder blend to yield an 80 mg strength capsule, as shown in Table 6.

**Table 6**

| **Component** | **Quantity 80mg capsule** | | **Function** |
|---|---|---|---|
| | **(mg/ capsule)** | **% (w/w)** | |
| Valbenazine ditosylate | 146.0 | 28.21 | Active |
| Mannitol | 320.0 | 61.82 | Diluent |
| Partially pregelatinized maize starch | 40.0 | 7.73 | Disintegra |
| Fumed silica | 6.4 | 1.24 | nt |
| Magnesium stearate | 2.4 | 1.00 | Glidant Lubricant |
| **Total Capsule Fill Weight** | 517.6 | 100.00 | - |
| Hard gelatin capsule - Size #0 | 1 | - | Shell |

Valbenazine ditosylate, mannitol (USP), partially pregelatinized maize starch (USNF), fumed silica (USNF) and magnesium stearate (USNF) are weighed according to the amounts in Table 6. A portion of the mannitol (1/4) is transferred through a screening mill equipped with a ~0.8 mm or equivalent round-hole screen to a tote bin for blending. The screened mannitol component is then blended.

### Pre-Blending and Screening:

The following components are transferred into the tote bin through a screening mill equipped with a ~0.8 mm or equivalent round-hole screen:
a. Valbenazine ditosylate
b. Fumed silica
c. Partially pregelatinized maize starch
d. Remaining mannitol (3/4) - (the adjustment of mannitol weight to compensate DS assay is performed on this fraction)

The components are blended and then transferred into polyethylene (PE) bags. The preblend is transferred through a screening mill equipped with an ~0.8 mm or equivalent round-hole screen to a tote bin for blending.

### Final Lubricant Blending:

Magnesium stearate (quantity to be adjusted as needed based on pre-lubricated blend yield-limit 98%) is manually screened (~1 mm sieve) into the opened tote bin for blending. The components are then blended.

### Encapsulation:

Efforts to fill a Size 0 capsule can be unsuccessful as it may not be possible to compress enough powder into a compact that would fit into a Size 0 capsule shell.

The various embodiments described above can be combined to provide further embodiments. This application also claims the benefit of U.S. Provisional Patent Application No. 62/684,935, filed June 14, 2018 and is incorporated herein by reference in its entirety. All of the U.S. patents, U.S. patent application publications, U.S. patent applications, foreign patents, foreign patent applications and non-patent publications referred to in this specification are incorporated herein by reference, in their entirety. Aspects of the embodiments can be modified, if necessary to employ concepts of the various patents, applications and publications to provide yet further embodiments.

These and other changes can be made to the embodiments in light of the above-detailed description. In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.

The following numbered paragraphs set out preferred features and preferred combinations of features of the present invention:
1. A method for selecting a subject for treatment with a VMAT2 inhibitor, comprising:
   determining whether the subject has COMT haploinsufficiency; and
   administering a therapeutically effective amount of a VMAT2 inhibitor to the subject if the subject has been determined to have COMT haploinsufficiency.
2. The method of para. 1, wherein the subject has a COMT108met variant on the nondeleted allele.
3. A method for selecting a subject for treatment with a VMAT2 inhibitor, comprising:
   determining whether the subject has 22q11.2 deletion syndrome; and
   administering a therapeutically effective amount of a VMAT2 inhibitor to the subject if the subject has been determined to have 22q11.2 deletion syndrome.
4. A method of treating a psychiatric disorder in a subject in need thereof, comprising:
   administering a therapeutically effective amount of a VMAT2 inhibitor to the subject,
   wherein the subject is predisposed to developing said psychiatric disorder due to the subject having COMT haploinsufficiency.
5. The method of para. 4, wherein the subject has a COMT108met variant on the nondeleted allele.
6. A method of treating a psychiatric disorder in a subject in need thereof, comprising:
   administering a therapeutically effective amount of a VMAT2 inhibitor to the subject,
   wherein the subject is predisposed to developing said psychiatric disorder due to the subject having 22q11.2 deletion syndrome.
7. A method of treating a psychiatric disorder in a subject in need thereof, comprising:
   determining whether the subject has COMT haploinsufficiency; and
   administering a therapeutically effective amount of a VMAT2 inhibitor to the subject if the subject has been determined to have COMT haploinsufficiency
8. The method of para. 7, wherein the subject has a COMT108met variant on the nondeleted allele.
9. A method of treating a psychiatric disorder in a subject in need thereof, comprising:
   determining whether the subject has 22q11.2 deletion syndrome; and
   administering a therapeutically effective amount of a VMAT2 inhibitor to the subject if the subject has been determined to have 22q11.2 deletion syndrome.
10. The method of any one of paras. 4 to 9, wherein the psychiatric disorder is schizophrenia spectrum disorder, attention deficit hyperactivity disorder, separation anxiety disorder, obsessive-compulsive disorder, autism spectrum disorder, mood disorder, anorexia nervosa, narcolepsy, heroin addiction, or early onset alcoholism.
11. The method of para. 10, wherein the psychiatric disorder is schizophrenia spectrum disorder.
12. The method of para. 10, wherein the psychiatric disorder is mood disorder.
13. The method of para. 10, wherein the psychiatric disorder is autism spectrum disorder.
14. The method of any one of paras. 1 to 13, wherein the VMAT2 inhibitor is selected from compounds of Formula (I) and pharmaceutically acceptable salts, solvates, and hydrates thereof: 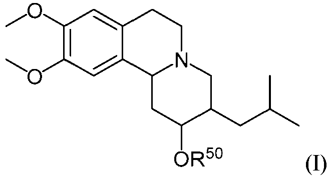 wherein:
   R⁵⁰ is -C(=O)-O-(CR^{a}R^{b})ₘ or -C(=O)-(CR^{a}R^{b})ₙNH₂,
   R^{a} and R^{b}, at each occurrence, are independently H, C₁₋₆alkyl, -NH-C(=NH)NH₂, -C(=O)OH, -C(=O)O-Me, -SH, -C(=O)NH₂, -NH₂, -SCH₃, phenyl, -OH, 4-hydroxy-phenyl, imidazolyl, or indolyl;
   m is 1 to 6; and
   n is 1 to 6.
15. Use of a VMAT2 inhibitor selected from compounds of Formula (I) and pharmaceutically acceptable salts, solvates, and hydrates thereof: 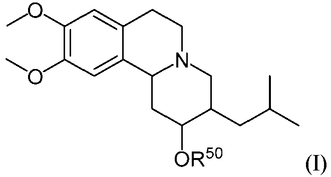 wherein:
   R⁵⁰ is -C(=O)-O-(CR^{a}R^{b})ₘ or -C(=O)-(CR^{a}R^{b})ₙNH₂,
   R^{a} and R^{b}, at each occurrence, are independently H, C₁₋₆alkyl, -NH-C(=NH)NH₂, -C(=O)OH, -C(=O)O-Me, -SH, -C(=O)NH₂, -NH₂, -SCH₃, phenyl, -OH, 4-hydroxy-phenyl, imidazolyl, or indolyl;
   m is 1 to 6; and
   n is 1 to 6,
   in the manufacture of a medicament for the treatment of a psychiatric disorder in a subject, the treatment comprising administering a therapeutically effective amount of a VMAT2 inhibitor to the subject,
   wherein the subject is predisposed to developing said psychiatric disorder due to the subject having COMT haploinsufficiency.
16. Use of a VMAT2 inhibitor selected from compounds of Formula (I) and pharmaceutically acceptable salts, solvates, and hydrates thereof: 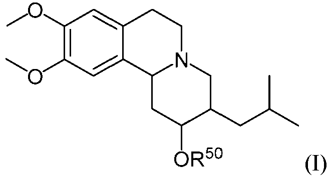 wherein:
   R⁵⁰ is -C(=O)-O-(CR^{a}R^{b})ₘ or -C(=O)-(CR^{a}R^{b})ₙNH₂,
   R^{a} and R^{b}, at each occurrence, are independently H, C₁₋₆alkyl, -NH-C(=NH)NH₂, -C(=O)OH, -C(=O)O-Me, -SH, -C(=O)NH₂, -NH₂, -SCH₃, phenyl, -OH, 4-hydroxy-phenyl, imidazolyl, or indolyl;
   m is 1 to 6; and
   n is 1 to 6,
   in the manufacture of a medicament for the treatment of a psychiatric disorder in a subject, the treatment comprising administering a therapeutically effective amount of a VMAT2 inhibitor to the subject,
   wherein the subject is predisposed to developing said psychiatric disorder due to the subject having 22q11.2 deletion syndrome.
17. A VMAT2 inhibitor that is selected from compounds of Formula (I) and pharmaceutically acceptable salts, solvates, and hydrates thereof: 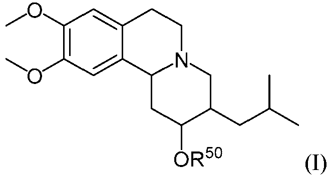 wherein:
   R⁵⁰ is -C(=O)-O-(CR^{a}R^{b})ₘ, or -C(=O)-(CR^{a}R^{b})ₙNH₂,
   R^{a} and R^{b}, at each occurrence, are independently H, C₁₋₆alkyl, -NH-C(=NH)NH₂, -C(=O)OH, -C(=O)O-Me, -SH, -C(=O)NH₂, -NH₂, -SCH₃, phenyl, -OH, 4-hydroxy-phenyl, imidazolyl, or indolyl;
   m is 1 to 6; and
   n is 1 to 6,
   for use in a method of treatment of a psychiatric disorder in a subject, the treatment comprising administering a therapeutically effective amount of a VMAT2 inhibitor to the subject,
   wherein the subject is predisposed to developing said psychiatric disorder due to the subject having COMT haploinsufficiency.
18. A VMAT2 inhibitor that is selected from compounds of Formula (I) and pharmaceutically acceptable salts, solvates, and hydrates thereof: 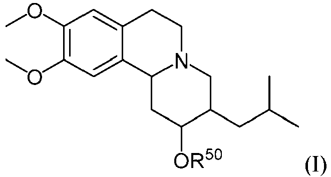 wherein:
   R⁵⁰ is -C(=O)-O-(CR^{a}R^{b})ₘ or -C(=O)-(CR^{a}R^{b})ₙNH₂,
   R^{a} and R^{b}, at each occurrence, are independently H, C₁₋₆alkyl, -NH-C(=NH)NH₂, -C(=O)OH, -C(=O)O-Me, -SH, -C(=O)NH₂, -NH₂, -SCH₃, phenyl, -OH, 4-hydroxy-phenyl, imidazolyl, or indolyl;
   m is 1 to 6; and
   n is 1 to 6,
   in the manufacture of a medicament for the treatment of a psychiatric disorder in a subject, the treatment comprising administering a therapeutically effective amount of a VMAT2 inhibitor to the subject,
   wherein the subject is predisposed to developing said psychiatric disorder due to the subject having 22q11.2 deletion syndrome.
19. The method of any one of paras. 1 to 13, wherein the VMAT2 inhibitor is selected from compounds of Formula (II) and pharmaceutically acceptable salts, solvates, and hydrates thereof: 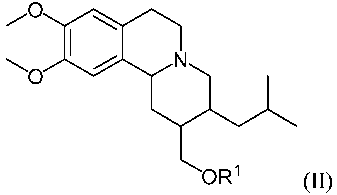
   wherein:
      R¹ is
      a) hydrogen;
      b) -P(=O)(OR³)₂;
      c) -C(=O)alkyl, wherein alkyl is optionally substituted with R¹⁰ and/or R²⁰;
      d) -C(=O)heterocyclyl, wherein heterocyclyl is optionally substituted with R¹⁰ and/or R²⁰;
      e) -C(=O)carbocyclyl, wherein carbocyclyl is optionally substituted with R¹⁰ and/or R²⁰;
      f) -C(=O)N(R³)alkyl, wherein alkyl is optionally substituted with R¹⁰ and/or R²⁰;
      g) -C(=O)N(R³)carbocyclyl, wherein carbocyclyl is optionally substituted with R¹⁰ and/or R²⁰;
      h) -C(=O)Oalkyl, wherein alkyl is optionally substituted with R¹⁰ and/or R²⁰; or
      i) alkyl, wherein alkyl is optionally substituted with R¹⁰ and/or R²⁰;
   and wherein,
      each R³ is independently hydrogen or alkyl;
      each R¹⁰ is independently halo, haloalkyl, cyano, nitro, trimethylsilanyl, -OR³⁰, -SR³⁰, -OC(=O)-R³⁰, -N(R³⁰)₂, -C(=O)R³⁰, -C(=O)OR³⁰, -C(=O)N(R³⁰)₂, -N(R³⁰)C(=O)OR³¹, -N(R³⁰)C(=O)R³¹, -N(R³⁰)C(=NR³¹)N(R³²)₂, -N(R³⁰)S(=O)ₜR³¹ (where t is 1 to 2), -S(=O)ₜOR³⁰ (where t is 1 to 2), -S(=O)ₚR³⁰ (where p is 0 to 2), -S(=O)ₜN(R³⁰)₂ (where t is 1 to 2), or -OP(=O)(OR³⁰)₂, or when a single atom bears two R¹⁰ groups such two R¹⁰ groups may be taken together to form oxo;
      each R²⁰ is independently alkyl, alkenyl, aryl, aralkyl, cycloalkyl, cycloalkylalkyl, heterocyclyl, heterocyclylalkyl, heteroaryl, or heteroarylalkyl, or when a single atom bears two R²⁰ groups such two R²⁰ groups may be taken together to form cycloalkyl, wherein each of said alkyl, alkenyl, aryl, aralkyl, cycloalkyl, cycloalkylalkyl, heterocyclyl, heterocyclylalkyl, heteroaryl, and heteroarylalkyl groups is optionally substituted with R¹⁰ and/or R²²;
      each R²² is independently alkyl, alkenyl, aryl, aralkyl, cycloalkyl, cycloalkylalkyl, heterocyclyl, heterocyclylalkyl, heteroaryl, or heteroarylalkyl, wherein each of said alkyl, alkenyl, aryl, aralkyl, cycloalkyl, cycloalkylalkyl, heterocyclyl, heterocyclylalkyl, heteroaryl, and heteroarylalkyl groups is optionally substituted with R¹⁰; and each R³⁰, R³¹, and R³² is independently hydrogen or alkyl.
20. Use of a VMAT2 inhibitor selected from compounds of Formula (II) and pharmaceutically acceptable salts, solvates, and hydrates thereof: 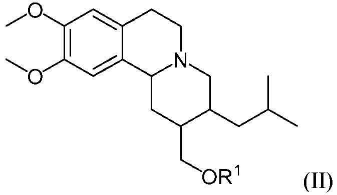
   wherein:
      R¹ is
      a) hydrogen;
      b) -P(=O)(OR³)₂;
      c) -C(=O)alkyl, wherein alkyl is optionally substituted with R¹⁰ and/or R²⁰;
      d) -C(=O)heterocyclyl, wherein heterocyclyl is optionally substituted with R¹⁰ and/or R²⁰;
      e) -C(=O)carbocyclyl, wherein carbocyclyl is optionally substituted with R¹⁰ and/or R²⁰;
      f) -C(=O)N(R³)alkyl, wherein alkyl is optionally substituted with R¹⁰ and/or R²⁰;
      g) -C(=O)N(R³)carbocyclyl, wherein carbocyclyl is optionally substituted with R¹⁰ and/or R²⁰;
      h) -C(=O)Oalkyl, wherein alkyl is optionally substituted with R¹⁰ and/or R²⁰; or
      i) alkyl, wherein alkyl is optionally substituted with R¹⁰ and/or R²⁰;
   and wherein,
      each R³ is independently hydrogen or alkyl;
      each R¹⁰ is independently halo, haloalkyl, cyano, nitro, trimethylsilanyl, -OR³⁰, -SR³⁰, -OC(=O)-R³⁰, -N(R³⁰)₂, -C(=O)R³⁰, -C(=O)OR³⁰, -C(=O)N(R³⁰)₂, -N(R³⁰)C(=O)OR³¹, -N(R³⁰)C(=O)R³¹, -N(R³⁰)C(=NR³¹)N(R³²)₂, -N(R³⁰)S(=O)ₜR³¹ (where t is 1 to 2), -S(=O)ₜOR³⁰ (where t is 1 to 2), -S(=O)ₚR³⁰ (where p is 0 to 2), -S(=O)ₜN(R³⁰)₂ (where t is 1 to 2), or -OP(=O)(OR³⁰)₂, or when a single atom bears two R¹⁰ groups such two R¹⁰ groups may be taken together to form oxo;
      each R²⁰ is independently alkyl, alkenyl, aryl, aralkyl, cycloalkyl, cycloalkylalkyl, heterocyclyl, heterocyclylalkyl, heteroaryl, or heteroarylalkyl, or when a single atom bears two R²⁰ groups such two R²⁰ groups may be taken together to form cycloalkyl, wherein each of said alkyl, alkenyl, aryl, aralkyl, cycloalkyl, cycloalkylalkyl, heterocyclyl, heterocyclylalkyl, heteroaryl, and heteroarylalkyl groups is optionally substituted with R¹⁰ and/or R²²;
      each R²² is independently alkyl, alkenyl, aryl, aralkyl, cycloalkyl, cycloalkylalkyl, heterocyclyl, heterocyclylalkyl, heteroaryl, or heteroarylalkyl, wherein each of said alkyl, alkenyl, aryl, aralkyl, cycloalkyl, cycloalkylalkyl, heterocyclyl, heterocyclylalkyl, heteroaryl, and heteroarylalkyl groups is optionally substituted with R¹⁰; and
      each R³⁰, R³¹, and R³² is independently hydrogen or alkyl,
   in the manufacture of a medicament for the treatment of a psychiatric disorder in a subject, the treatment comprising administering a therapeutically effective amount of a VMAT2 inhibitor to the subject,
   wherein the subject is predisposed to developing said psychiatric disorder due to the subject having COMT haploinsufficiency.
21. Use of a VMAT2 inhibitor selected from compounds of Formula (II) and pharmaceutically acceptable salts, solvates, and hydrates thereof: 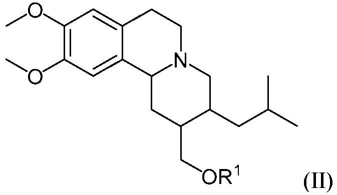
   wherein:
      R¹ is
      a) hydrogen;
      b) -P(=O)(OR³)₂;
      c) -C(=O)alkyl, wherein alkyl is optionally substituted with R¹⁰ and/or R²⁰;
      d) -C(=O)heterocyclyl, wherein heterocyclyl is optionally substituted with R¹⁰ and/or R²⁰;
      e) -C(=O)carbocyclyl, wherein carbocyclyl is optionally substituted with R¹⁰ and/or R²⁰;
      f) -C(=O)N(R³)alkyl, wherein alkyl is optionally substituted with R¹⁰ and/or R²⁰;
      g) -C(=O)N(R³)carbocyclyl, wherein carbocyclyl is optionally substituted with R¹⁰ and/or R²⁰;
      h) -C(=O)Oalkyl, wherein alkyl is optionally substituted with R¹⁰ and/or R²⁰; or
      i) alkyl, wherein alkyl is optionally substituted with R¹⁰ and/or R²⁰;
   and wherein,
      each R³ is independently hydrogen or alkyl;
      each R¹⁰ is independently halo, haloalkyl, cyano, nitro, trimethylsilanyl, -OR³⁰, -SR³⁰, -OC(=O)-R³⁰, -N(R³⁰)₂, -C(=O)R³⁰, -C(=O)OR³⁰, -C(=O)N(R³⁰)₂, -N(R³⁰)C(=O)OR³¹, -N(R³⁰)C(=O)R³¹, -N(R³⁰)C(=NR³¹)N(R³²)₂, -N(R³⁰)S(=O)ₜR³¹ (where t is 1 to 2), -S(=O)ₜOR³⁰ (where t is 1 to 2), -S(=O)ₚR³⁰ (where p is 0 to 2), -S(=O)ₜN(R³⁰)₂ (where t is 1 to 2), or -OP(=O)(OR³⁰)₂, or when a single atom bears two R¹⁰ groups such two R¹⁰ groups may be taken together to form oxo;
      each R²⁰ is independently alkyl, alkenyl, aryl, aralkyl, cycloalkyl, cycloalkylalkyl, heterocyclyl, heterocyclylalkyl, heteroaryl, or heteroarylalkyl, or when a single atom bears two R²⁰ groups such two R²⁰ groups may be taken together to form cycloalkyl, wherein each of said alkyl, alkenyl, aryl, aralkyl, cycloalkyl, cycloalkylalkyl, heterocyclyl, heterocyclylalkyl, heteroaryl, and heteroarylalkyl groups is optionally substituted with R¹⁰ and/or R²²;
      each R²² is independently alkyl, alkenyl, aryl, aralkyl, cycloalkyl, cycloalkylalkyl, heterocyclyl, heterocyclylalkyl, heteroaryl, or heteroarylalkyl, wherein each of said alkyl, alkenyl, aryl, aralkyl, cycloalkyl, cycloalkylalkyl, heterocyclyl, heterocyclylalkyl, heteroaryl, and heteroarylalkyl groups is optionally substituted with R¹⁰; and
      each R³⁰, R³¹, and R³² is independently hydrogen or alkyl,
   in the manufacture of a medicament for the treatment of a psychiatric disorder in a subject, the treatment comprising administering a therapeutically effective amount of a VMAT2 inhibitor to the subject,
   wherein the subject is predisposed to developing said psychiatric disorder due to the subject having 22q11.2 deletion syndrome.
22. A VMAT2 inhibitor that is selected from compounds of Formula (II) and pharmaceutically acceptable salts, solvates, and hydrates thereof: 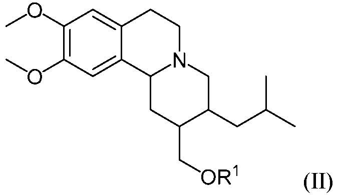
   wherein:
      R¹ is
      a) hydrogen;
      b) -P(=O)(OR³)₂;
      c) -C(=O)alkyl, wherein alkyl is optionally substituted with R¹⁰ and/or R²⁰;
      d) -C(=O)heterocyclyl, wherein heterocyclyl is optionally substituted with R¹⁰ and/or R²⁰;
      e) -C(=O)carbocyclyl, wherein carbocyclyl is optionally substituted with R¹⁰ and/or R²⁰;
      f) -C(=O)N(R³)alkyl, wherein alkyl is optionally substituted with R¹⁰ and/or R²⁰;
      g) -C(=O)N(R³)carbocyclyl, wherein carbocyclyl is optionally substituted with R¹⁰ and/or R²⁰;
      h) -C(=O)Oalkyl, wherein alkyl is optionally substituted with R¹⁰ and/or R²⁰; or
      i) alkyl, wherein alkyl is optionally substituted with R¹⁰ and/or R²⁰;
   and wherein,
      each R³ is independently hydrogen or alkyl;
      each R¹⁰ is independently halo, haloalkyl, cyano, nitro, trimethylsilanyl, -OR³⁰, -SR³⁰, -OC(=O)-R³⁰, -N(R³⁰)₂, -C(=O)R³⁰, -C(=O)OR³⁰, -C(=O)N(R³⁰)₂, -N(R³⁰)C(=O)OR³¹, -N(R³⁰)C(=O)R³¹, -N(R³⁰)C(=NR³¹)N(R³²)₂, -N(R³⁰)S(=O)ₜR³¹ (where t is 1 to 2), -S(=O)ₜOR³⁰ (where t is 1 to 2), -S(=O)ₚR³⁰ (where p is 0 to 2), -S(=O)ₜN(R³⁰)₂ (where t is 1 to 2), or -OP(=O)(OR³⁰)₂, or when a single atom bears two R¹⁰ groups such two R¹⁰ groups may be taken together to form oxo;
      each R²⁰ is independently alkyl, alkenyl, aryl, aralkyl, cycloalkyl, cycloalkylalkyl, heterocyclyl, heterocyclylalkyl, heteroaryl, or heteroarylalkyl, or when a single atom bears two R²⁰ groups such two R²⁰ groups may be taken together to form cycloalkyl, wherein each of said alkyl, alkenyl, aryl, aralkyl, cycloalkyl, cycloalkylalkyl, heterocyclyl, heterocyclylalkyl, heteroaryl, and heteroarylalkyl groups is optionally substituted with R¹⁰ and/or R²²;
      each R²² is independently alkyl, alkenyl, aryl, aralkyl, cycloalkyl, cycloalkylalkyl, heterocyclyl, heterocyclylalkyl, heteroaryl, or heteroarylalkyl, wherein each of said alkyl, alkenyl, aryl, aralkyl, cycloalkyl, cycloalkylalkyl, heterocyclyl, heterocyclylalkyl, heteroaryl, and heteroarylalkyl groups is optionally substituted with R¹⁰; and
      each R³⁰, R³¹, and R³² is independently hydrogen or alkyl,
   for use in a method of treatment of a psychiatric disorder in a subject, the treatment comprising administering a therapeutically effective amount of a VMAT2 inhibitor to the subject,
   wherein the subject is predisposed to developing said psychiatric disorder due to the subject having COMT haploinsufficiency.
23. A VMAT2 inhibitor that is selected from compounds of Formula (II) and pharmaceutically acceptable salts, solvates, and hydrates thereof: 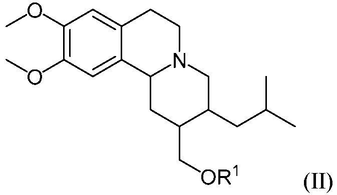
   wherein:
      R¹ is
      a) hydrogen;
      b) -P(=O)(OR³)₂;
      c) -C(=O)alkyl, wherein alkyl is optionally substituted with R¹⁰ and/or R²⁰;
      d) -C(=O)heterocyclyl, wherein heterocyclyl is optionally substituted with R¹⁰ and/or R²⁰;
      e) -C(=O)carbocyclyl, wherein carbocyclyl is optionally substituted with R¹⁰ and/or R²⁰;
      f) -C(=O)N(R³)alkyl, wherein alkyl is optionally substituted with R¹⁰ and/or R²⁰;
      g) -C(=O)N(R³)carbocyclyl, wherein carbocyclyl is optionally substituted with R¹⁰ and/or R²⁰;
      h) -C(=O)Oalkyl, wherein alkyl is optionally substituted with R¹⁰ and/or R²⁰; or
      i) alkyl, wherein alkyl is optionally substituted with R¹⁰ and/or R²⁰;
   and wherein,
      each R³ is independently hydrogen or alkyl;
      each R¹⁰ is independently halo, haloalkyl, cyano, nitro, trimethylsilanyl, -OR³⁰, -SR³⁰, -OC(=O)-R³⁰, -N(R³⁰)₂, -C(=O)R³⁰, -C(=O)OR³⁰, -C(=O)N(R³⁰)₂, -N(R³⁰)C(=O)OR³¹, -N(R³⁰)C(=O)R³¹, -N(R³⁰)C(=NR³¹)N(R³²)₂, -N(R³⁰)S(=O)ₜR³¹ (where t is 1 to 2), -S(=O)ₜOR³⁰ (where t is 1 to 2), -S(=O)ₚR³⁰ (where p is 0 to 2), -S(=O)ₜN(R³⁰)₂ (where t is 1 to 2), or -OP(=O)(OR³⁰)₂, or when a single atom bears two R¹⁰ groups such two R¹⁰ groups may be taken together to form oxo;
      each R²⁰ is independently alkyl, alkenyl, aryl, aralkyl, cycloalkyl, cycloalkylalkyl, heterocyclyl, heterocyclylalkyl, heteroaryl, or heteroarylalkyl, or when a single atom bears two R²⁰ groups such two R²⁰ groups may be taken together to form cycloalkyl, wherein each of said alkyl, alkenyl, aryl, aralkyl, cycloalkyl, cycloalkylalkyl, heterocyclyl, heterocyclylalkyl, heteroaryl, and heteroarylalkyl groups is optionally substituted with R¹⁰ and/or R²²;
      each R²² is independently alkyl, alkenyl, aryl, aralkyl, cycloalkyl, cycloalkylalkyl, heterocyclyl, heterocyclylalkyl, heteroaryl, or heteroarylalkyl, wherein each of said alkyl, alkenyl, aryl, aralkyl, cycloalkyl, cycloalkylalkyl, heterocyclyl, heterocyclylalkyl, heteroaryl, and heteroarylalkyl groups is optionally substituted with R¹⁰; and
      each R³⁰, R³¹, and R³² is independently hydrogen or alkyl,
   in the manufacture of a medicament for the treatment of a psychiatric disorder in a subject, the treatment comprising administering a therapeutically effective amount of a VMAT2 inhibitor to the subject,
   wherein the subject is predisposed to developing said psychiatric disorder due to the subject having 22q11.2 deletion syndrome.
24. The method of any one of paras. 1 to 13, wherein the VMAT2 inhibitor is selected from compounds of Formula (III) and pharmaceutically acceptable salts, solvates, and hydrates thereof: 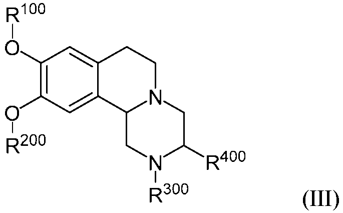 wherein:
   R¹⁰⁰ and R²⁰⁰ are independently lower alkyl, lower cycloalkyl, or lower cycloalkylalkyl, wherein each lower alkyl, lower cycloalkyl, and lower cycloalkylalkyl is independently unsubstituted or substituted with one or more halo, cyano, or lower alkoxy;
   R³⁰⁰ is lower alkyl; and
   R⁴⁰⁰ is lower alkyl or lower cycloalkylalkyl.
25. Use of a VMAT2 inhibitor selected from compounds of Formula (III) and pharmaceutically acceptable salts, solvates, and hydrates thereof: 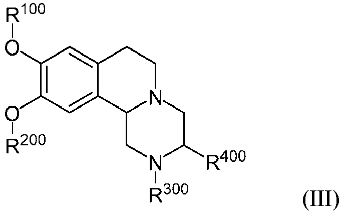 wherein:
   R¹⁰⁰ and R²⁰⁰ are independently lower alkyl, lower cycloalkyl, or lower cycloalkylalkyl, wherein each lower alkyl, lower cycloalkyl, and lower cycloalkylalkyl is independently unsubstituted or substituted with one or more halo, cyano, or lower alkoxy;
   R³⁰⁰ is lower alkyl; and
   R⁴⁰⁰ is lower alkyl or lower cycloalkylalkyl
   in the manufacture of a medicament for the treatment of a psychiatric disorder in a subject, the treatment comprising administering a therapeutically effective amount of a VMAT2 inhibitor to the subject,
   wherein the subject is predisposed to developing said psychiatric disorder due to the subject having COMT haploinsufficiency
26. Use of a VMAT2 inhibitor selected from compounds of Formula (III) and pharmaceutically acceptable salts, solvates, and hydrates thereof: 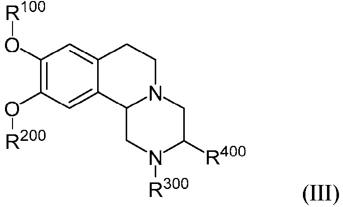 wherein:
   R¹⁰⁰ and R²⁰⁰ are independently lower alkyl, lower cycloalkyl, or lower cycloalkylalkyl, wherein each lower alkyl, lower cycloalkyl, and lower cycloalkylalkyl is independently unsubstituted or substituted with one or more halo, cyano, or lower alkoxy;
   R³⁰⁰ is lower alkyl; and
   R⁴⁰⁰ is lower alkyl or lower cycloalkylalkyl
   in the manufacture of a medicament for the treatment of a psychiatric disorder in a subject, the treatment comprising administering a therapeutically effective amount of a VMAT2 inhibitor to the subject,
   wherein the subject is predisposed to developing said psychiatric disorder due to the subject having 22q11.2 deletion syndrome.
27. A VMAT2 inhibitor that is selected from compounds of Formula (III) and pharmaceutically acceptable salts, solvates, and hydrates thereof: 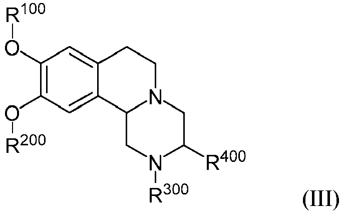 wherein:
   R¹⁰⁰ and R²⁰⁰ are independently lower alkyl, lower cycloalkyl, or lower cycloalkylalkyl, wherein each lower alkyl, lower cycloalkyl, and lower cycloalkylalkyl is independently unsubstituted or substituted with one or more halo, cyano, or lower alkoxy;
   R³⁰⁰ is lower alkyl; and
   R⁴⁰⁰ is lower alkyl or lower cycloalkylalkyl,
   for use in a method of treatment of a psychiatric disorder in a subject, the treatment comprising administering a therapeutically effective amount of a VMAT2 inhibitor to the subject,
   wherein the subject is predisposed to developing said psychiatric disorder due to the subject having COMT haploinsufficiency.
28. A VMAT2 inhibitor that is selected from compounds of Formula (III) and pharmaceutically acceptable salts, solvates, and hydrates thereof: 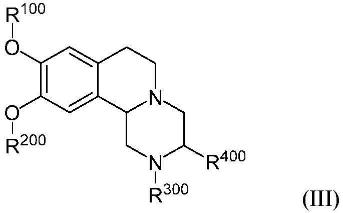 wherein:
   R¹⁰⁰ and R²⁰⁰ are independently lower alkyl, lower cycloalkyl, or lower cycloalkylalkyl, wherein each lower alkyl, lower cycloalkyl, and lower cycloalkylalkyl is independently unsubstituted or substituted with one or more halo, cyano, or lower alkoxy;
   R³⁰⁰ is lower alkyl; and
   R⁴⁰⁰ is lower alkyl or lower cycloalkylalkyl,
   in the manufacture of a medicament for the treatment of a psychiatric disorder in a subject, the treatment comprising administering a therapeutically effective amount of a VMAT2 inhibitor to the subject,
   wherein the subject is predisposed to developing said psychiatric disorder due to the subject having 22q11.2 deletion syndrome.
29. The method of para. 24, the use of para. 25 or 26, or the VMAT2 inhibitor of para. 27 or 28, wherein the VMAT2 inhibitor is selected from compounds of Formula (IV) and pharmaceutically acceptable salts, solvates, and hydrates thereof: 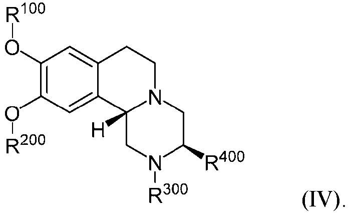
30. The method of any one of paras. 1 to 13, wherein the VMAT2 inhibitor is selected from the compounds of Table 1 and pharmaceutically acceptable salts, solvates, and hydrates thereof.
31. Use of a VMAT2 inhibitor selected from compounds of Table 1 and pharmaceutically acceptable salts, solvates, and hydrates thereof,
   in the manufacture of a medicament for the treatment of a psychiatric disorder in a subject, the treatment comprising administering a therapeutically effective amount of a VMAT2 inhibitor to the subject,
   wherein the subject is predisposed to developing said psychiatric disorder due to the subject having COMT haploinsufficiency.
32. Use of a VMAT2 inhibitor selected from compounds of Table 1 and pharmaceutically acceptable salts, solvates, and hydrates thereof,
   in the manufacture of a medicament for the treatment of a psychiatric disorder in a subject, the treatment comprising administering a therapeutically effective amount of a VMAT2 inhibitor to the subject,
   wherein the subject is predisposed to developing said psychiatric disorder due to the subject having 22q11.2 deletion syndrome.
33. A VMAT2 inhibitor that is selected from compounds of Table 1 and pharmaceutically acceptable salts, solvates, and hydrates thereof,
   for use in a method of treatment of a psychiatric disorder in a subject, the treatment comprising administering a therapeutically effective amount of a VMAT2 inhibitor to the subject,
   wherein the subject is predisposed to developing said psychiatric disorder due to the subject having COMT haploinsufficiency.
34. A VMAT2 inhibitor that is selected from compounds of Table 1 and pharmaceutically acceptable salts, solvates, and hydrates thereof,
   in the manufacture of a medicament for the treatment of a psychiatric disorder in a subject, the treatment comprising administering a therapeutically effective amount of a VMAT2 inhibitor to the subject,
   wherein the subject is predisposed to developing said psychiatric disorder due to the subject having 22q11.2 deletion syndrome.
35. The method of any one of paras. 1 to 13, wherein the VMAT2 inhibitor is selected from the compounds of Table 2 and pharmaceutically acceptable salts, solvates, and hydrates thereof.
36. Use of a VMAT2 inhibitor selected from compounds of Table 2 and pharmaceutically acceptable salts, solvates, and hydrates thereof,
   in the manufacture of a medicament for the treatment of a psychiatric disorder in a subject, the treatment comprising administering a therapeutically effective amount of a VMAT2 inhibitor to the subject,
   wherein the subject is predisposed to developing said psychiatric disorder due to the subject having COMT haploinsufficiency.
37. Use of a VMAT2 inhibitor selected from compounds of Table 2 and pharmaceutically acceptable salts, solvates, and hydrates thereof,
   in the manufacture of a medicament for the treatment of a psychiatric disorder in a subject, the treatment comprising administering a therapeutically effective amount of a VMAT2 inhibitor to the subject,
   wherein the subject is predisposed to developing said psychiatric disorder due to the subject having 22q11.2 deletion syndrome.
38. A VMAT2 inhibitor that is selected from compounds of Table 2 and pharmaceutically acceptable salts, solvates, and hydrates thereof,
   for use in a method of treatment of a psychiatric disorder in a subject, the treatment comprising administering a therapeutically effective amount of a VMAT2 inhibitor to the subject,
   wherein the subject is predisposed to developing said psychiatric disorder due to the subject having COMT haploinsufficiency.
39. A VMAT2 inhibitor that is selected from compounds of Table 2 and pharmaceutically acceptable salts, solvates, and hydrates thereof,
   in the manufacture of a medicament for the treatment of a psychiatric disorder in a subject, the treatment comprising administering a therapeutically effective amount of a VMAT2 inhibitor to the subject,
   wherein the subject is predisposed to developing said psychiatric disorder due to the subject having 22q11.2 deletion syndrome.
40. The method of any one of paras. 1 to 13, wherein the VMAT2 inhibitor is selected from the compounds of Table 3 and pharmaceutically acceptable salts, solvates, and hydrates thereof.
41. Use of a VMAT2 inhibitor selected from compounds of Table 3 and pharmaceutically acceptable salts, solvates, and hydrates thereof,
   in the manufacture of a medicament for the treatment of a psychiatric disorder in a subject, the treatment comprising administering a therapeutically effective amount of a VMAT2 inhibitor to the subject,
   wherein the subject is predisposed to developing said psychiatric disorder due to the subject having COMT haploinsufficiency.
42. Use of a VMAT2 inhibitor selected from compounds of Table 3 and pharmaceutically acceptable salts, solvates, and hydrates thereof,
   in the manufacture of a medicament for the treatment of a psychiatric disorder in a subject, the treatment comprising administering a therapeutically effective amount of a VMAT2 inhibitor to the subject,
   wherein the subject is predisposed to developing said psychiatric disorder due to the subject having 22q11.2 deletion syndrome.
43. A VMAT2 inhibitor that is selected from compounds of Table 3 and pharmaceutically acceptable salts, solvates, and hydrates thereof,
   for use in a method of treatment of a psychiatric disorder in a subject, the treatment comprising administering a therapeutically effective amount of a VMAT2 inhibitor to the subject,
   wherein the subject is predisposed to developing said psychiatric disorder due to the subject having COMT haploinsufficiency.
44. A VMAT2 inhibitor that is selected from compounds of Table 3 and pharmaceutically acceptable salts, solvates, and hydrates thereof,
   in the manufacture of a medicament for the treatment of a psychiatric disorder in a subject, the treatment comprising administering a therapeutically effective amount of a VMAT2 inhibitor to the subject,
   wherein the subject is predisposed to developing said psychiatric disorder due to the subject having 22q11.2 deletion syndrome.
45. The method of any one of paras. 1 to 13, wherein the VMAT2 inhibitor is selected from the following compounds and pharmaceutically acceptable salts, solvates, and hydrates thereof: 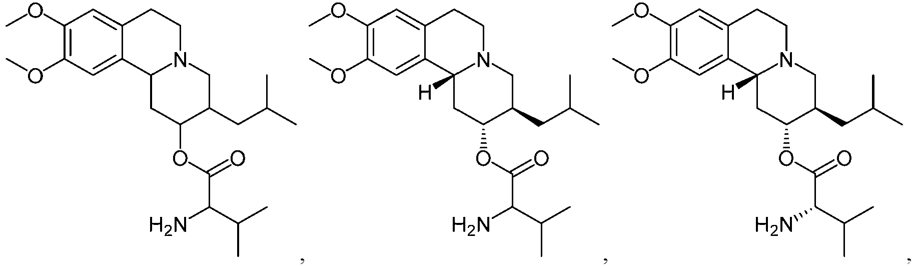 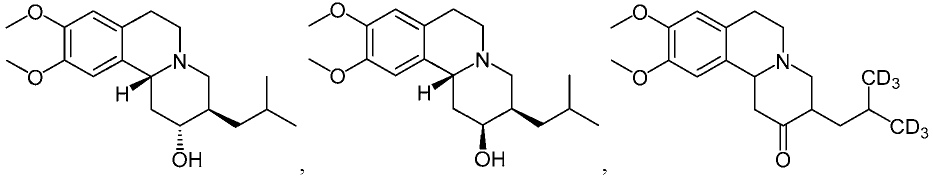 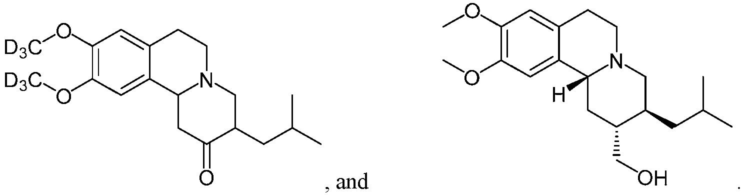
46. Use of a VMAT2 inhibitor selected from the following compounds and pharmaceutically acceptable salts, solvates, and hydrates thereof: 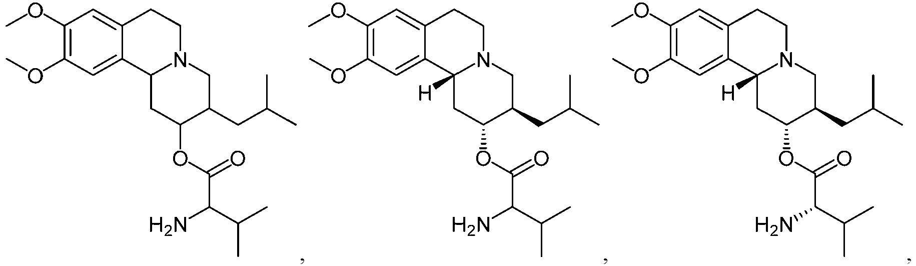 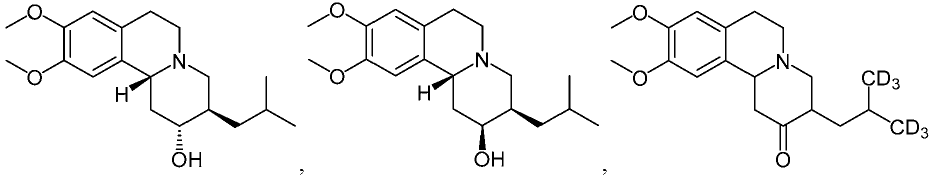 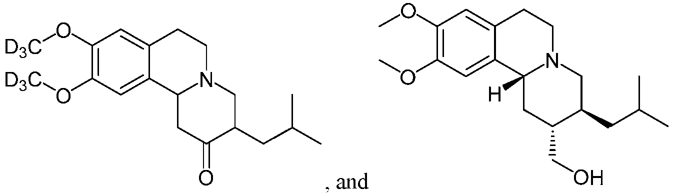
   in the manufacture of a medicament for the treatment of a psychiatric disorder in a subject, the treatment comprising administering a therapeutically effective amount of a VMAT2 inhibitor to the subject,
   wherein the subject is predisposed to developing said psychiatric disorder due to the subject having COMT haploinsufficiency.
47. Use of a VMAT2 inhibitor selected from the following compounds and pharmaceutically acceptable salts, solvates, and hydrates thereof: 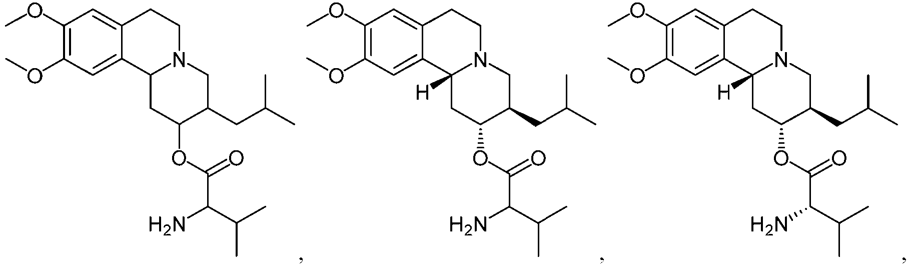 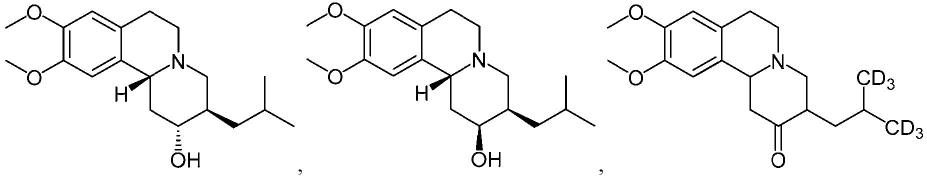 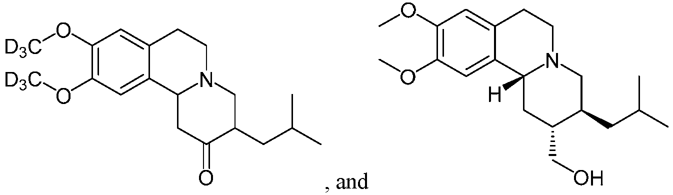
   in the manufacture of a medicament for the treatment of a psychiatric disorder in a subject, the treatment comprising administering a therapeutically effective amount of a VMAT2 inhibitor to the subject,
   wherein the subject is predisposed to developing said psychiatric disorder due to the subject having 22q11.2 deletion syndrome.
48. A VMAT2 inhibitor that is selected from the following compounds and pharmaceutically acceptable salts, solvates, and hydrates thereof: 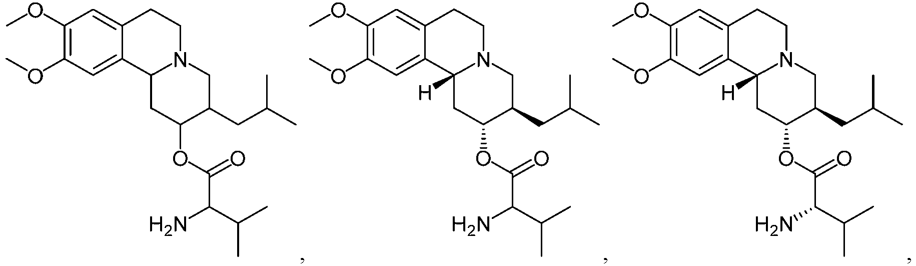 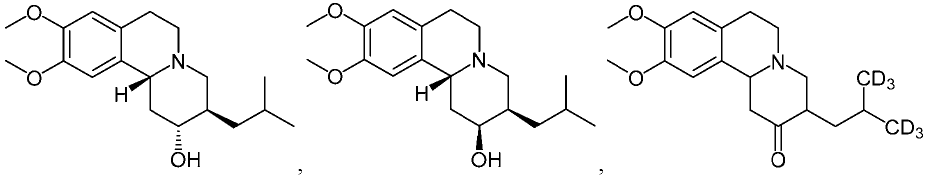 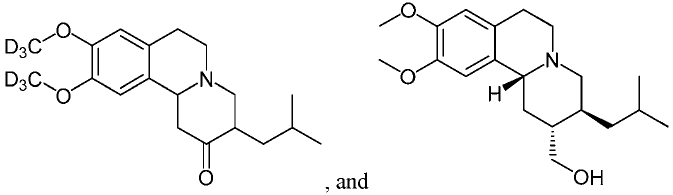
   for use in a method of treatment of a psychiatric disorder in a subject, the treatment comprising administering a therapeutically effective amount of a VMAT2 inhibitor to the subject,
   wherein the subject is predisposed to developing said psychiatric disorder due to the subject having COMT haploinsufficiency.
49. A VMAT2 inhibitor that is selected from the following compounds and pharmaceutically acceptable salts, solvates, and hydrates thereof: 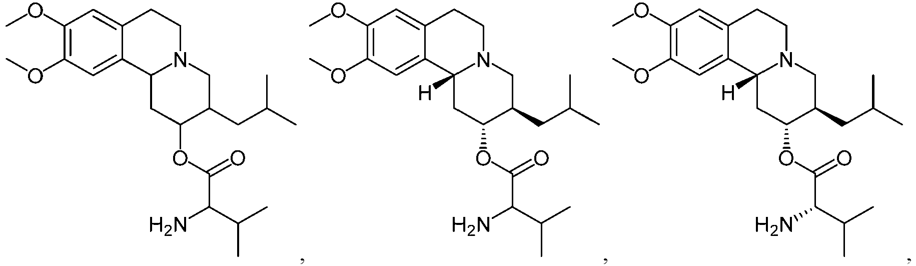 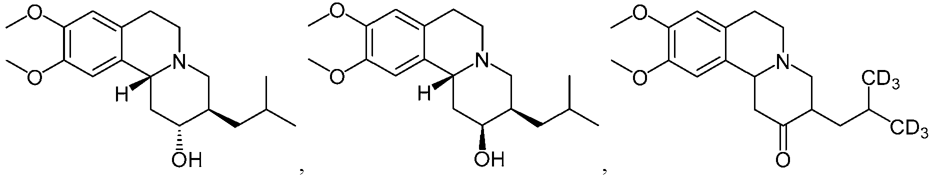 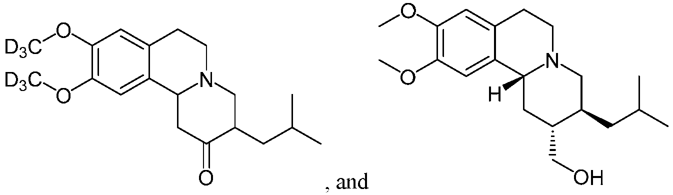
   in the manufacture of a medicament for the treatment of a psychiatric disorder in a subject, the treatment comprising administering a therapeutically effective amount of a VMAT2 inhibitor to the subject,
   wherein the subject is predisposed to developing said psychiatric disorder due to the subject having 22q11.2 deletion syndrome.
50. The method of any one of paras. 1 to 14, 19, 24, 29, 30, 35, 40, or 45, the use of any one of paras. 15, 16, 20, 21, 25, 26, 29, 31, 32, 36, 37, 41, 42, 46, or 47, or the VMAT2 inhibitor of any one of paras. 17, 18, 22, 23, 27, 28, 29, 33, 34, 38, 39, 43, 44, 48, or 49, wherein the pharmaceutically acceptable salt of the VMAT2 inhibitor is a ditosylate salt.
51. The method of any one of paras. 1 to 14, 19, 24, 29, 30, 35, 40, 45, or 50, the use of any one of paras. 15, 16, 20, 21, 25, 26, 29, 31, 32, 36, 37, 41, 42, 46, 47, or 50, or the VMAT2 inhibitor of any one of paras. 17, 18, 22, 23, 27, 28, 29, 33, 34, 38, 39, 43, 44, 48, 49, or 50, wherein the VMAT2 inhibitor is administered at a daily dose of about 5 mg to about 160 mg.
52. The method of para. 51, the use of para. 51, or the VMAT2 inhibitor of para. 51, wherein the VMAT2 inhibitor is administered at a daily dose of about 5 mg.
53. The method of para. 51, the use of para. 51, or the VMAT2 inhibitor of para. 51, wherein the VMAT2 inhibitor is administered at a daily dose of about 20 mg.
54. The method of para. 51, the use of para. 51, or the VMAT2 inhibitor of para. 51, wherein the VMAT2 inhibitor is administered at a daily dose of about 40 mg.
55. The method of para. 51, the use of para. 51, or the VMAT2 inhibitor of para. 51, wherein the VMAT2 inhibitor is administered at a daily dose of about 60 mg.
56. The method of para. 51, the use of para. 51, or the VMAT2 inhibitor of para. 51, wherein the VMAT2 inhibitor is administered at a daily dose of about 80 mg.
57. The method of para. 51, the use of para. 51, or the VMAT2 inhibitor of para. 51, wherein the VMAT2 inhibitor is administered at a daily dose of about 100 mg.
58. The method of para. 51, the use of para. 51, or the VMAT2 inhibitor of para. 51, wherein the VMAT2 inhibitor is administered at a daily dose of about 120 mg.
59. The method of para. 51, the use of para. 51, or the VMAT2 inhibitor of para. 51, wherein the VMAT2 inhibitor is administered at a daily dose of about 140 mg.
60. The method of para. 51, the use of para. 51, or the VMAT2 inhibitor of para. 51, wherein the VMAT2 inhibitor is administered at a daily dose of about 160 mg.
61. The method, use, or VMAT2 inhibitor of any one of the above paras., wherein the VMAT2 inhibitor is formulated for oral administration.
62. The method, use, or VMAT2 inhibitor of para. 61, wherein the VMAT2 inhibitor is administered as a solution, tablet, or capsule.
63. The method of any one of paras. 1 to 14, 19, 24, 29, 30, 35, 40, 45, or 50, the use of any one of paras. 15, 16, 20, 21, 25, 26, 29, 31, 32, 36, 37, 41, 42, 46, 47, or 50, or the VMAT2 inhibitor of any one of paras. 17, 18, 22, 23, 27, 28, 29, 33, 34, 38, 39, 43, 44, 48, 49, or 50, wherein the VMAT2 inhibitor has a binding affinity (*K*ᵢ) for VMAT2 of less than about 1000 nM.
64. The method of any one of paras. 1 to 14, 19, 24, 29, 30, 35, 40, 45, or 50, the use of any one of paras. 15, 16, 20, 21, 25, 26, 29, 31, 32, 36, 37, 41, 42, 46, 47, or 50, or the VMAT2 inhibitor of any one of paras. 17, 18, 22, 23, 27, 28, 29, 33, 34, 38, 39, 43, 44, 48, 49, or 50, wherein the VMAT2 inhibitor has a binding affinity (*K*ᵢ) for VMAT2 of less than about 100 nM.

## Claims

1. A compound which is (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester, or a pharmaceutically acceptable salt thereof,
for use in a method of treating a psychiatric disorder in a subject in need thereof,
the method comprising:
administering a therapeutically effective amount of the compound or salt to the subject,
wherein the subject is predisposed to developing said psychiatric disorder due to the subject having COMT haploinsufficiency.

2. The compound or salt for use according to claim 1, wherein the subject has a COMT108met variant on the nondeleted allele.

3. A compound which is (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester, or a pharmaceutically acceptable salt thereof,
for use in a method of treating a psychiatric disorder in a subject in need thereof,
the method comprising:
administering a therapeutically effective amount of the compound or salt to the subject,
wherein the subject is predisposed to developing said psychiatric disorder due to the subject having 22q11.2 deletion syndrome.

4. A compound which is (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester, or a pharmaceutically acceptable salt thereof,
for use in a method of treating a psychiatric disorder in a subject in need thereof,
the method comprising:
determining whether the subject has COMT haploinsufficiency; and
administering a therapeutically effective amount of the compound or salt to the subject if the subject has been determined to have COMT haploinsufficiency.

5. The compound or salt for use according to claim 4, wherein the subject has a COMT108met variant on the nondeleted allele.

6. A compound which is (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester, or a pharmaceutically acceptable salt thereof,
for use in a method of treating a psychiatric disorder in a subject in need thereof,
the method comprising:
determining whether the subject has 22q11.2 deletion syndrome; and
administering a therapeutically effective amount of the compound or salt to the subject if the subject has been determined to have 22q11.2 deletion syndrome.

7. The compound for use according to any one of claims 1-6, wherein the psychiatric disorder is schizophrenia spectrum disorder, attention deficit hyperactivity disorder, separation anxiety disorder, obsessive-compulsive disorder, autism spectrum disorder, mood disorder, anorexia nervosa, narcolepsy, heroin addiction, or early onset alcoholism.

8. The compound or salt for use according to claim 7, wherein the psychiatric disorder is schizophrenia spectrum disorder.

9. The compound or salt for use according to claim 7, wherein the psychiatric disorder is mood disorder.

10. The compound or salt for use according to claim 7, wherein the psychiatric disorder is autism spectrum disorder.

11. The compound or salt for use according to any one of claims 1-10, which is:
a pharmaceutically acceptable salt of (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester.

12. The compound or salt for use according to claim 11, which is:
a ditosylate salt of (S)-2-amino-3-methyl-butyric acid (2R,3R,11bR)-3-isobutyl-9,10-dimethoxy-1 ,3,4,6,7,11 b-hexahydro-2H-pyrido[2, 1-a]isoquinolin-2-yl ester.

13. The compound or salt for use according to any one of claims 1-12, wherein the compound or salt is administered at a daily dose of:
about 5 mg to about 160 mg;
about 40 mg;
about 60 mg; or
about 80 mg.

14. The compound or salt for use according to any one of claims 1-13, wherein the compound or salt is formulated for oral administration.

15. The compound or salt for use according to claim 14, wherein the compound or salt is administered as a solution, tablet, or capsule.
